(19) Europäisches Patentamt
European Patent Office
Office européen des brevets

(11) Publication number: 0 361 341 A2

(12) # EUROPEAN PATENT APPLICATION

(21) Application number: 89117616.6

(22) Date of filing: 23.09.89

(51) Int. Cl.5: C07K 5/02 , A61K 37/64 , C07D 401/12 , C07D 263/06 , C07F 9/32 , A61K 33/42 , A61K 31/395

A request for correction for pages 67 and 188 has been filed pursuant to Rule 88 EPC. A decision on the request will be taken during the proceedings before the Examining Division (Guidelines for Examination in the EPO, A-V, 2.2).

(30) Priority: 28.09.88 US 250472
01.08.89 US 386194

(43) Date of publication of application: 04.04.90 Bulletin 90/14

(84) Designated Contracting States: AT BE CH DE ES FR GB GR IT LI LU NL SE

(71) Applicant: Molecular Therapeutics, Inc.
400 Morgan Lane
West Haven, Connecticut 06516(US)

(72) Inventor: Hanko, Rudolf H., Dr.
101 Edgemont Road
Milford, CT 06440(US)
Inventor: Scangos, George A., Dr.
Waldseestrasse 42
D-4030 Ratingen(DE)
Inventor: Yoo-Warren, Heeja, Dr.
514 Treat Lane
Orange, CT 06477(US)
Inventor: Ramabhadran, Triprayar V., Dr.
112, Murray Lane
Guilford, CT 06437(US)
Inventor: Paessens, Arnold, Dr.
Stresemannstrasse 56
D-5657 Haan 1(DE)
Inventor: Henning, Rolf, Dr.
Boecklinstrasse 16
D-5600 Wuppertal 1(DE)
Inventor: Tamburini, Paul Perry, Dr.
36 Misty Mountain Road
Kensington, CT 06037(US)
Inventor: Hoppe, Dieter, Prof. Dr.
Schneiderkamp 17e
D-2300 Kiel 1(DE)
Inventor: Hansen, Jutta, Dr.
Claudiusweg 9
D-5600 Wuppertal 1(DE)
Inventor: Rabe, Klaus
Winterbekerweg 20
D-2300 Kiel 1(DE)

(74) Representative: Dänner, Klaus, Dr. et al
c/o Bayer AG Konzernverwaltung RP Patente Konzern
D-5090 Leverkusen 1 Bayerwerk(DE)

(54) **Therapeutics for aids based on inhibitors of HIV protease.**

(57) A therapeutic active compound, especially for treating AIDS, comprising
(A′)$_k$-(Z)$_n$-(Y)$_m$-A″
wherein
A′ is

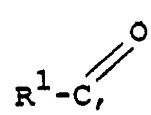

EP 0 361 341 A2

or hydrogen,
wherein
$R^1$ is $OR^2$, $NR^3R^2$ or $CR^2R^3R^4$,
$R^2$,$R^3$ and $R^4$ are, independent of each other, hydrogen, an aliphatic or an aromatic, wherein k is one or zero, wherein when Z is hydrogen, k is zero,
Z is hydrogen, Ser or Thr or

O
‖
$R^1$ - C , wherein n is one or zero, with the proviso that when Y is hydrogen, n is zero,
Y is hydrogen or

O
‖
$R^1$ - C , or

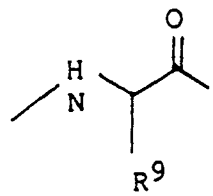

wherein $R^9$ is hydrogen or an aliphatic or aromatic,
wherein m is one or zero, wherein when $E^4$ is hydrogen, m is zero, A″ is

(a) 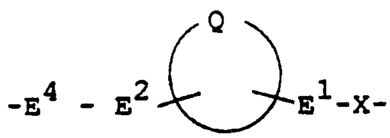 Q = S, $NY^2$, O or $CR^2R^3$,

(b) 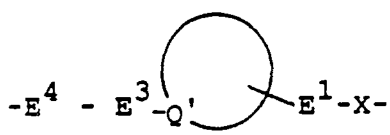 Q′ = N or $CR^2$, and

(c) 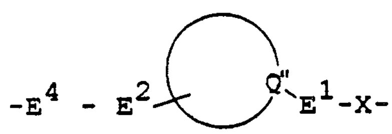 Q" = N or $CR^2$,

wherein $E^4$ is hydrogen or Asn or

O
‖
$R^1$- C ,
wherein

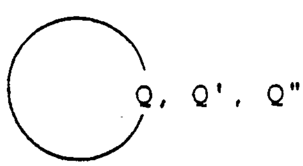

is a 4 to 7 membered ring system,
wherein only Q, Q′, Q″ may be different from carbon,
wherein the substituent is an aliphatic or aromatic,
wherein $E^1$ is C ;
‖
O
wherein $E^3$ is

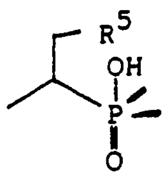 or 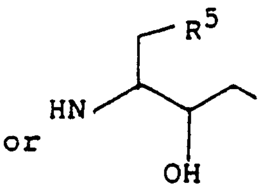 or 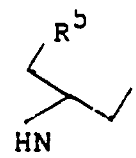

wherein $E^2$ is

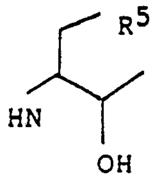 or 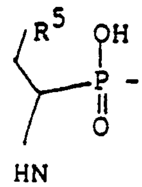 or 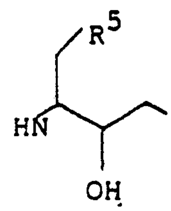 or 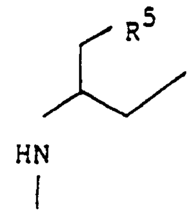

wherein $E^4$ is hydrogen or Asn, or

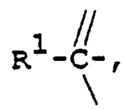

but if $E^2$ is $R^5$

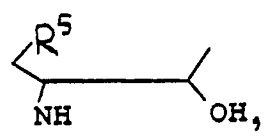

$E^4$ is not

O<br>
||<br>
$R^2$-O- C - ,

wherein $R^5$ is an aromatic or aliphatic,
wherein X is hydrogen or $R^1$ or

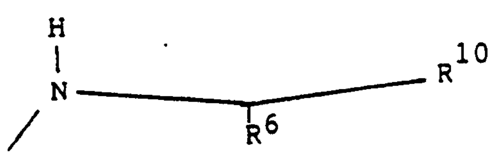

wherein $R^6$ is an aliphatic,
wherein $R^{10}$ is hydrogen or

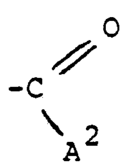 or 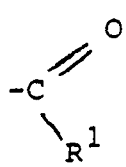 ,

wherein $A^2$ has the formula

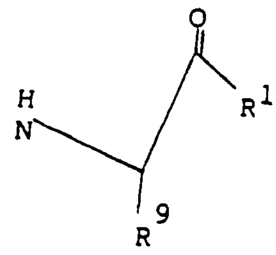

# THERAPEUTICS FOR AIDS BASED ON INHIBITORS OF HIV PROTEASE

## BACKGROUND OF THE INVENTION

### Field of the Invention

The present invention concerns therapeutics for AIDS based on inhibitors of HIV protease. The invention also is directed to novel compounds having pharmaceutical activity, to the production of such compounds, to intermediates, to compositions containing such novel compounds and to the use of such compounds in pharmacology. The invention also relates to a rapid, sensitive assay for HIV-protease activity.

### Background Information

Human Immunodeficiency Virus (HIV), the causative agent of the Acquired Immune Deficiency Syndrome (AIDS), is a member of the lentivirus family of retroviurs (M. A. Gonda, F. Wong-Staal and R. C. Gallo, "Sequence Homology and Morphological Similarity of HTLV III and Visna Virus, A Pathogenic Lentivirus", Science, 227, 173, (1985) and P. Sonigo and N. Alizon et al, "Nucleotide Sequence of the Visna Levtivirus: Relationship to the AIDS Virus", Cell, 42, 369, (1985)).

AIDS has already spread to 131 countries with the highest reported cases in the United States, reaching over 42,000 people. The high risk groups of adults/adolescents include homosexual/bisexual males (66%), intravenous (IV) drug abusers (16%), homosexual males and IV drug abusers (8%), hemophilia/coagulation disorder patients (1%), heterosexual cases (4%), transfusion, blood/components (2%) and undetermined ones (3%); while in the children's group, the highest incidence of 78% was from parents "at risk" of AIDS. The case fatality rate is 58%.

HIV, in common with other retroviruses, encodes a number of enzymes that are necessary for the viral life cycle (R. Weiss, N. Teich, H. Varmus and J. Coffin, eds., (1982), The Molecular Biology of TNA Tumor Viruses, 2nd edition. Cold spring Harbor Laboratory, New York and L. Ratner, W. Haseltine, et al, (1985), "Complete Nucleotide Sequence of the AIDS Virus, HTLV III", Nature, 313, 277, (1985)). In the absence of these enzymes, productive viral infection does not occur (R. Weiss, N. Teich, H. Varmus and J. Coffin, supra).

In view of the above, inhibitors of the aforementioned enzymes have therapeutic potential against AIDS. The only currently used therapeutic, AZT, is an inhibitor of the viral reverse transcriptase (H. Mitsuya and S. Broder, "Inhibition of the In Virto Infectivity and Cytopathic Effects of HTLV III", Proc. Natl. Acad. Sci. USA, 83, 1911 (1986)).

Other viral enzymes include an RNAse, an integrase and a protease, all of which are essential for the viral life cycle (H. Mitsuya and S. Broder, (1987), "Strategies for Antiviral Therapy in AIDS", Nature, 325, 773, (1987)).

The viral protease is responsible for cleavage of the gag and gag-pol polyproteins into mature peptides (L. Ratner, W. Haseltine, et al supra; R. A. Kramer, M D. Schaber, et al. "HTLV III Gag Protein is Processed in Yeast Cells by the virus Pol Protease", Science, 231, 1580, (1986) and W. G. Farmerie, D. D. Loeb et al, "Expression and Processing of the AIDS Virus Reverse Transcriptase in Escherichia coli", Science, 236, 305, (1987)). The amino acid sequence for the gag polyprotein is described in Ratner et al, Nature, 313, 277-284 (1985).

From the known location at the 5′ end of the pol gene and the inferred protease cleavage sites (L. H. Pearl and W. R. Taylor, "Sequence Specificity of Retroviral Proteases", Nature, 328, 482, (1987)), the predicted size of the protease is 99 amino acids. The HIV protease, along with the other retroviral proteases, have homology to cellular aspartyl proteases (I. Katoh, T. Yasunaga, Y. Ikawa and Y. Yoshinada, "Inhibition of Retroviral Protease Activity by an Aspartyl Proteinase Inhibitor", Nature, 329, 654, (1987)).

Some of the cleavage sites of the HIV protease have been determined (L. H. Pearl and W. R. Taylor, "Sequence Specificity of Retroviral Proteases", Nature, 328, 482 (1987)) and are listed below as follows:

(1) Ser-Phe-Asn-Phe-Pro-Gln-Val

(2) Thr-Leu-Asn-Phe-Pro-Ile-Ser

(3) Ser-Gln-Asn-Tyr-Pro-Ile-Val

Sequences (1) and (2) above are those that border the protease itself, while sequence (3) above is

located in the gag polyprotein.

The ability to obtain milligram amounts of recombinant HIV-protease in a highly pure form has aided in the development by the applicants herein of a rapid, sensitive, specific assay for HIV-protease activity using dansylated peptide substrates.

The isolation and purification techniques required for polypeptide products synthesized in E. coli are the subject of a review article by Marston, Biochem.J., (1986) 240, 1-12. This article describes purification of fusion proteins with and without cleavage. Although it broadly discloses a three stage process of cell lysis, denaturation and refolding, there is no suggestion of solubilization by increasing concentrations of buffered urea.

Marston et al also describes a method for solubilization of prochymosin (F.A.O. Marston, P.A. Lowe, S.W. Schoemaker and S. Angal, "Purification of Calf Prochymosin (prorennin) Synthesized in E. coli", Bio Technology, 2, pp 800-804 (1984)). Optimal solibilization was obtained using 8M urea or 6M guanidine HCl.

Partial purification of MS2-HIV-fusion proteins was achieved by the addition of acetone (-70 °C) to bacterial lystates obtained by French press treatment and sonification, and the precipitate dried under reduced pressure followed by a multistep purification procedure (Hansen et al, "Partial Purification and Substrate Analysis of Bacterially Expressed HIV Protease by Means of Monoclonal Antibody", EMBO J., 1785-1791, (1988)).

Gaim and Boros, J.Biol. Chem., 263, 14617-14620, (1988), describe purification of lacZ-pro fusion protein. To express large quantities of protease, they used an E. coli ribosomal RNA promotor and lac operator. The insoluble lacZ-pro proteins were solubilized and unfolded in an aqueous solution containing 8M urea and 50 mM DTT at concentrations of 0.2-0.4 mg/ml. To refold the protein, the suspension was diluted with 1 ml of a buffer containing 10mM Tris at pH 6.8, while allowing the suspension to stand at room temperature overnight.

The following method of purification of MS2-HIV fusion protease provides milligram quantities of a high purity preparation of HIV-protease migrating as a single band on SDS PAGE : (a) fractionating inclusion bodies containing MS2-HIV protease fusion protein by succesive protein extraction into solutions containing 2M, 4M, 6M, and 8M urea, each extraction being performed for 90 minutes, (b) refolding and autocatalytic cleaving of MS2-HIV protease fusion protein during a gradual decrease in urea concentration from 8M to 1.5M, under reducing conditions at pH8, and (c) precipitating remaining protein contaminants by dialysis. The contaminants can be dialysized in a buffer containing Triton X-100, 1.5M urea, glycerol, and dithiothreitol. The steps are conducted at room temperature and the soluble protease preparation has an overall purity of greater than 95%.

The use of dansylated peptide substrates has been reported in the development of non-HIV-protease enzyme assays. For example, the use of dansylated peptides has been reported for vertebrate collagenase, alpha-amidating enzyme and glutaminyl cyclase (Bond et al, "A Convenient Fluorescent Assay for Vertebrate Collagenase", Anal.Biochem, 155, 315-321 (1986); Jones et al, "A Fluorometric Assay for Peptidyl Alpha-amidation Activity Using High-Performance Liquid Chromatography", Anal. Biochem, 168, 272-279 (1988); and Consalvo et al, "A Rapid Fluorometric Assay for N-terminal Glutaminyl Cyclase Activity using High Performance Liquid Chromatography", Anal.Biochem, 175, 131-138 (1988)).

The above disadvantages are overcome by the assay of the present invention, such inventive assay can detect and quantify proteolytic activity of HIV-protease protein and the inhibitory potency of test compounds.

## SUMMARY OF THE INVENTION

An object of the present invention is to provide an active therapeutic compound to treat AIDS. Another object of the present invention is to provide a rapid sensitive assay for HIV protease activity. These objects and other objects, aims and advantages are provided by the present invention.

The present invention relates to an active compound having the formula

$(A')_k-(Z)_n-(Y)_m-A''$ (I)

,wherein

$A'$ is $R^1-\overset{O}{\overset{\|}{C}}$ or hydrogen,

$R^1$ is $OR^2$, $NR^2R^3$ or $CR^2R^3R^4$,

$R^2$, $R^3$ and $R^4$, independent of each other, are hydrogen, an unsubstituted or substituted aliphatic group and

1 to 20 carbon atoms or an unsubstituted or substituted aromatic group with 6 to 18 carbon atoms, wherein k is one (1) or zero (0), with the proviso that if Z is hydrogen, k is zero (0),

Z is hydrogen or amino acid residue Ser or Thr or

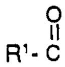 , wherein $R^1$ is defined as above wherein n is one (1) or zero (0), with the proviso that when Y is hydrogen, n is zero (0),

Y is hydrogen or

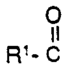 , wherein $R^1$ is defined as above or a group having the formula

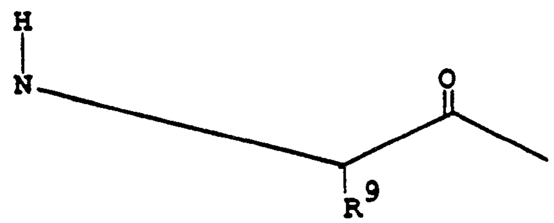

,

wherein $R^9$ is hydrogen or an unsubstituted or substituted $C_1$ - $C_{18}$ aliphatic group or an unsubstituted or substituted aromatic group with six to eighteen carbon atoms, wherein the substitutent is

-OH, 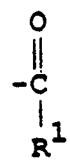 , -SH,

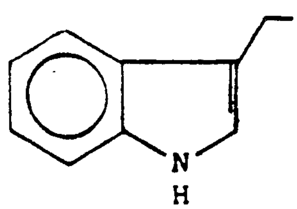 , $-NR^3R^2$ , 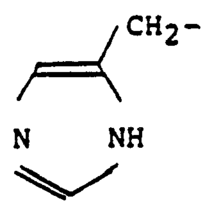 or 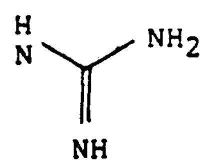

or an aromatic group with 6 to 18 carbon atoms,

wherein $R^1$ $R^2$ and $R^3$ are defined as before,

wherein m is one (1) or zero (0),

with the proviso that when $E^4$ is hydrogen, m is zero (0),

A″ is selected from the group consisting of

a) 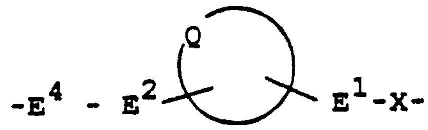 Q = S, $NR^2$, oxygen or $CR^2R^3$,

b) 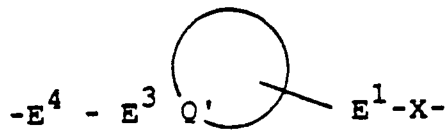 Q′ = N or $CR^2$ and

c) 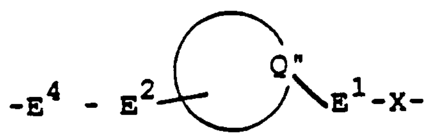 Q" = N or $CR^2$,

wherein the symbol

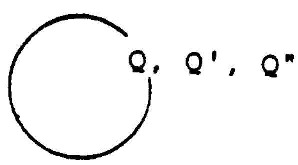

stands for a substituted or unsubstituted saturated ring system, which is 4 to 7 membered, wherein only Q, Q′ Q″ may be different from carbon, wherein the substituent is a $C_1$ - $C_{12}$ aliphatic group or an aromatic group with six to twelve carbon atoms,

wherein $E^1$ is 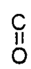 ;

wherein $E^3$ is

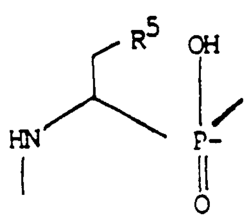 or 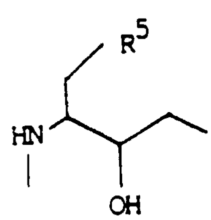 or 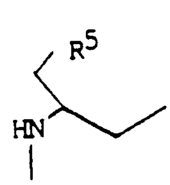

wherein $E^2$ is

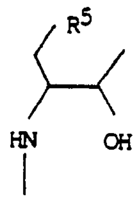 or 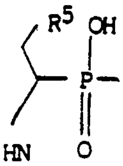 or 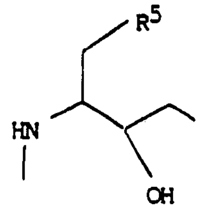 or 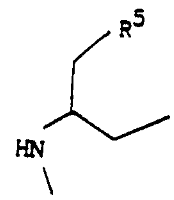

with the proviso if $E^2$ is

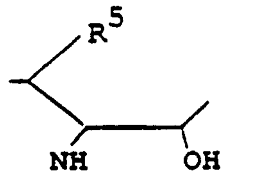 ,

$E^4$ is not $R^2-O-C(=O)-$, wherein $E^4$ is hydrogen or the amino acid residue Asn or $R^1-C(=O)-$, wherein $R^1$ is as defined above, it is recognized that other natural and unnatural amino acid residues can be substituted for Asn wherein $R^5$ is an unsubstituted or substituted aromatic group with six to eighteen carbon atoms or an unsubstituted or substituted aliphatic group with 1 to 18 carbon atoms, wherein X is hydrogen or $R^1$ or a group having the formula

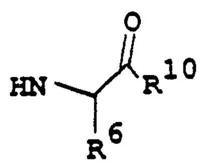

wherein $R^6$ is a substituted or unsubstituted $C_1$ - $C_{12}$ aliphatic group or a substituted or unsubstituted aromatic group with 6 to 12 carbon atoms, wherein the substitutent is

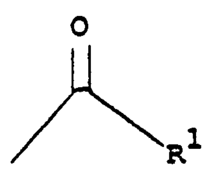

or a $C_1$ - $C_{12}$ aliphatic or aromatic group with 6 to 12 carbon atoms, wherein $R^1$ is as defined above, wherein $R^{10}$ is hydrogen or $-C(=O)-A^2$ or $-C(=O)-R^1$, wherein $A^2$ is a group having the formula,

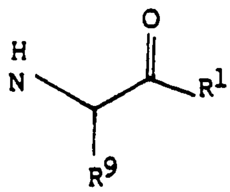

wherein $R^9$ is defined above, and wherein $R^1$ is defined above, and pharmaceutically acceptable salts or esters thereof.

The aforesaid compounds can exist in different stereochemical forms.

The invention relates both to the individual isomers (forms) and to their mixture.

The aforesaid active compounds inhibit the proteolytic activities of HIV-1 serotypes in the entire gag-pol polyprotein or in regions thereof.

The invention is also directed to a method of treating a patient infected with HIV, comprising administering to such patient an effective amount of the aforesaid therapeutic active compound, or a salt thereof, or an ester thereof, alone or in admixture with a diluent.

The present invention also relates to a rapid, sensitive assay for detecting HIV-protease activity. The assay comprises

(a) incubating HIV-protease with an N-Dansyl-peptide which can be cleaved proteolytically by the HIV-protease <u>in vitro</u>, the incubating being conducted under conditions in which the HIV-protease is catalytically active,

(b) subjecting a portion from the resultant mixture (a) to analysis by analytical RP-HPLC,

(c) resolving uncoverted peptide substrate from peptide products (resolving means completely separating the uncoverted substrate and product so as to enable their separate quantification) and

(d) determining the amount of peptide products formed over a known time interval to calculate the activity of HIV-protease.

## DETAILED DESCRIPTION OF THE INVENTION

The heretofore three cleavage sites of HIV protease have in common the sequence Thr(Ser)-X-Asn-Y-Pro-Z, wherein X is any amino acid, Z is a lipophilic amino acid and Y is either Tyr or Phe.

Based on these cleavage sites, analogs of high energy intermediates of the reaction having inhibitory activity can be designed.

From experience with other proteases, it is known that tetrahedral intermediates of high energy like (2) shown directly below are formed in the reaction pathway shown in below as follows:

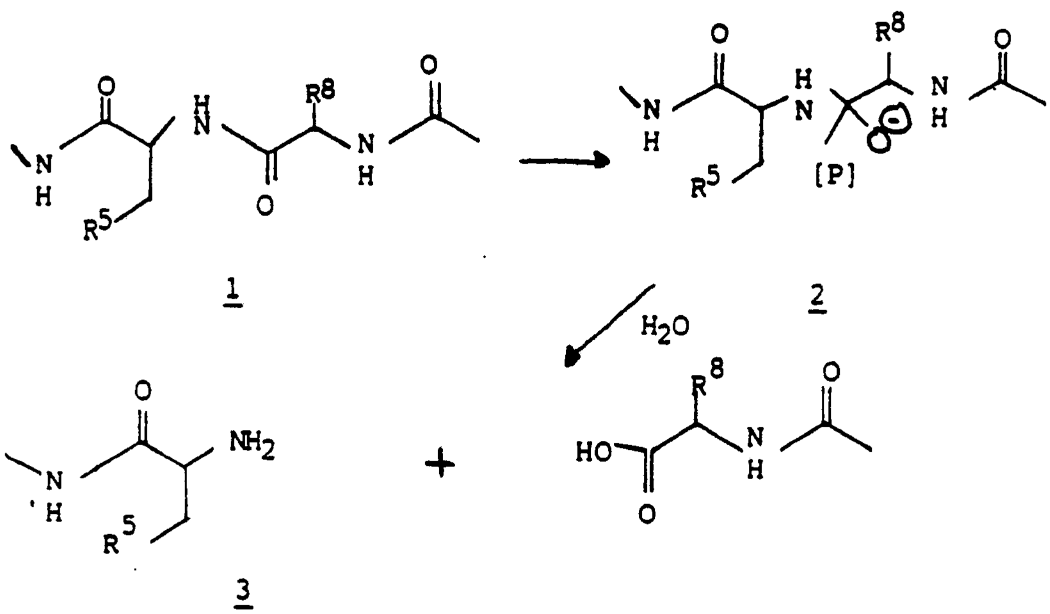


1 2 3

wherein [P] is a proteolytic enzyme with or without bound water molecules, wherein $R^5$ is as defined above, and $R^8$ is an aliphatic or aromatic residue.

According to the Transition State Analog Theory stable analogs of tetrahedral structures like (2) given directly herein above should bind tightly to protease and shown strong inhibition of protease activity.

In the compound according to the present invention having the formula $(A')_k-(Z)_n-(Y)_m-A''$ (I),

$A'$ is

$R^1-\overset{O}{\overset{\|}{C}}-$ or H, wherein $R^1$ is $OR^2$, $NR^3R^2$, or $CR^2R^3R^4$, wherein $R^2$, $R^3$ and $R^4$ are independent of each other, hydrogen, an aliphatic group with 1 to 20 carbon atoms or an aromatic group with 6 to 18 carbon atoms, preferably $R^2$, $R^3$ and $R^4$ are independent of each other, hydrogen, unsubstituted or substituted $C_1$-$C_{20}$-alkyl, unsubstituted or substituted $C_2$-$C_{20}$ alkenyl, unsubstituted or substituted $C_3$-$C_{20}$-alkynyl or $C_6$-$C_{18}$ aromatics, wherein the substituents for the alkyl-, alkenyl- and alkynyl-groups are selected from the group consisting of $C_1$-$C_5$-alkyl, $C_6$-$C_{12}$ aromatics, halogen (e.g., chlorine, fluorine, iodine or bromine); hydroxy; and an amino group of the formula $NR^8R^7$, wherein $R^8$ and $R^7$, independent of each other, are hydrogen, $C_1$-$C_{20}$-alkyl or oxygen. The substitutents for the aromatic group are $C_1$-$C_5$-alkyl, $C_6$-$C_{12}$-aromatics, halogen (e.g., fluorine, chlorine, bromine or iodine), hydroxy, the aforesaid amino group $NR^8R^7$, wherein $R^8$ and $R^7$ are defined as above, and $C_1$-$C_{20}$-alkyl.

In the above formula (I), k is one (1) or zero (0), with the proviso that when Z is hydrogen, k is zero (0).

Z is hydrogen or amino acid residue Ser or Thr or

$R^1-\overset{O}{\overset{\|}{C}}$ , wherein $R^1$ is as defined above.

In the above formula (I) n is one (1) or zero (0), with the proviso that when Y is hydrogen, n is zero (0).

Y is hydrogen or

$R^1-\overset{O}{\overset{\|}{C}}$ , wherein $R^1$ is as defined above, or one of the following amino acid residues:

| | | |
|---|---|---|
| aspartic acid | Asp | D |
| asparagine | Asn | N |
| threonine | Thr | T |
| serine | Ser | S |
| glutamic acid | Glu | E |
| glutamine | Gln | Q |
| proline | Pro | P |
| glycine | Gly | G |
| alanine | Ala | A |
| cysteine | Cys | C |
| valine | Val | V |
| methionine | Met | M |
| isoleucine | Ile | I |
| leucine | Leu | L |
| tyrosine | Tyr | Y |
| phenylalanine | Phe | F |
| tryptophan | Trp | W |
| lysine | Lys | K |
| histidine | His | H |
| arginine | Arg | R |

In the above compound, m is one (1) or zero (0) with the proviso that when $E^4$ is hydrogen, m is zero (0). $A''$ in formula (I) is selected from the group consisting of

a) 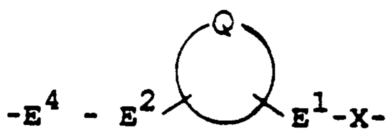 Q = S, $NR^2$, oxygen or $CR^2R^3$,

b) 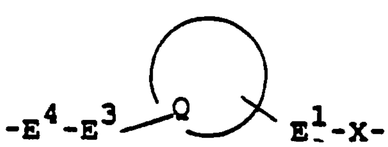 Q = N or $CR^2$ and

c) 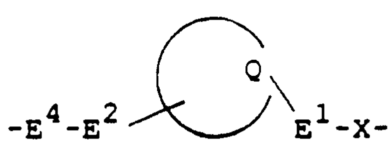 Q=N or $CR^2$,

wherein $E^1$ is 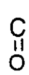 ;

wherein $E^3$ is

$E^2$ is 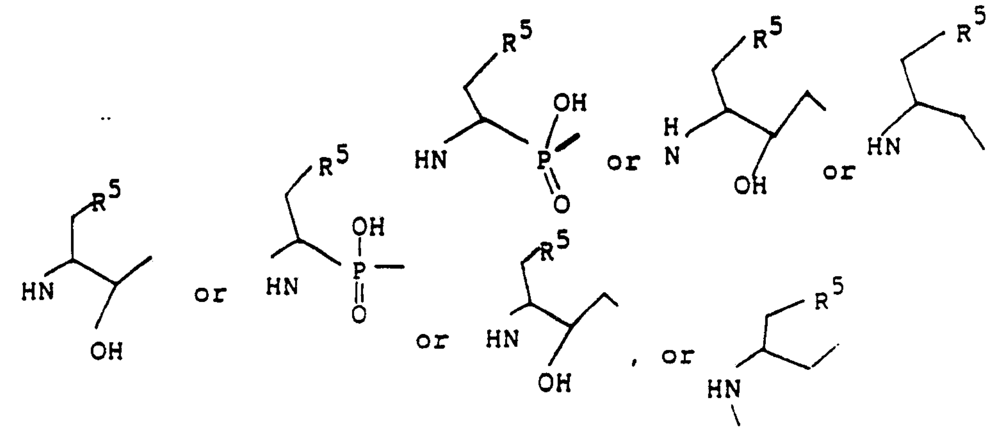 or or or or , or

with the proviso that if $E^2$ is

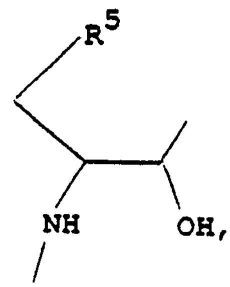

then $E^4$ cannot be

$R^2\text{-O-}\overset{\overset{O}{\|}}{C}$,

wherein $E^4$ is hydrogen or the amino acid residue Asn or

$R^1\text{-}\overset{\overset{O}{\|}}{C}\text{-}$, wherein $R^1$ is as defined above,

wherein the symbol

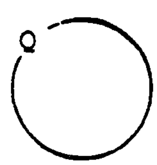

stands for an unsubstituted, saturated ring system, which is 4 to 7 membered, wherein only Q may be different from carbon,

wherein $R^5$ is an unsubstituted or substituted aromatic group with 6 to 18 carbon atoms or an unsubstituted or substituted aliphatic group with 1 to 18 carbon atoms and preferably with 1 to 10 carbon atoms, wherein the substitutents are selected from the group consisting of $C_1$-$C_5$-alkyl, $C_6$-$C_{12}$ aromatics, halogen (e.g., fluorine, chlorine, bromine or iodine), hydroxy and an amino group of the formula $-NR^8R^7$, wherein $R^8$ and $R^7$, independently of each other are hydrogen, $C_1$-$C_{20}$ alkyl, oxygen, $C_2$-$C_{20}$-alkenyl or $C_2$-$C_{20}$-alkynyl. More preferably, $R^5$ is phenyl, hydroxyphenyl, naphthyl, methyl, ethyl, isopropyl, butyl, isobutyl, sec.-butyl, cyclopentyl or cyclohexyl.

X is hydrogen, or $R^1$ or a group having the formula

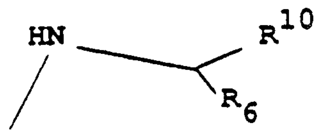

wherein $R^6$ is a substituted or unsubstituted $C_1$-$C_{12}$ aliphatic group or an unsubstituted or substituted aromatic group with 6 to 12 carbon atoms, preferably the aliphatic group for $R^6$ has one to seven carbon atoms,

wherein the substituent is $\overset{O}{\overset{\|}{C}}$-$R^1$ or a $C_1$-$C_{12}$ aliphatic group or an aromatic group with 6 to 12 carbon atoms. Preferably the substituent is $\overset{O}{\overset{\|}{C}}$-$R^1$ or phenyl, pyridyl, naphthyl, methyl, ethyl, propyl, isopropyl, butyl, sec.-butyl or isobutyl, wherein $R^1$ is defined as before.

Wherein $R^{10}$ is hydrogen or

- $\overset{O}{\overset{\|}{C}}$-$A^2$ or - $\overset{O}{\overset{\|}{C}}$-$R^1$,

$R^1$ is defined as before,

$A^2$ is one of the following amino acid residues

| | |
|---|---|
| aspartic acid | Asp |
| asparagine | Asn |
| threonine | Thr |
| serine | Ser |
| glutamic acid | Glu |
| glutamine | Gln |
| proline | Pro |
| glycine | Gly |
| alanine | Ala |
| cysteine | Cys |
| valine | Val |
| methionine | Met |
| isoleucine | Ile |
| leucine | Leu |
| tyrosine | Tyr |
| phenylalanine | Phe |
| tryptophan | Trp |
| lysine | Lys |
| histidine | His |
| arginine | Arg |

or a $C_1$-$C_{18}$ aliphatic or aromatic ester thereof with the aromatic ester having six to eighteen carbon atoms, and the aliphatic ester preferably having one to ten carbon atoms, or an $C_1$-$C_{18}$ aliphatic or aromatic amide thereof with the aromatic amide having six to eighteen carbon atoms and the aliphatic amide preferably having one to ten carbon atoms.

Preferably $E^1$ and $E^2$ are disposed adjacent to each other.

Preferred moieties for $A''$ include the following

(A)

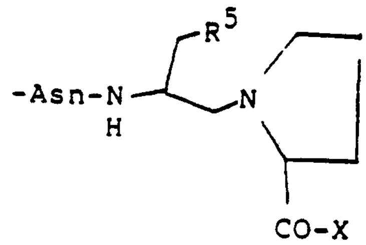

(B)

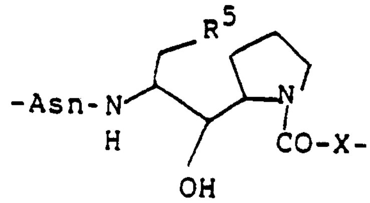

(C)

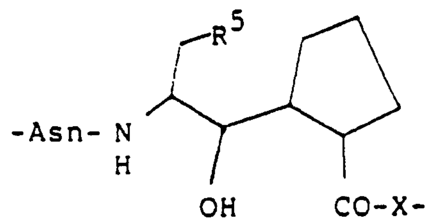

(D)

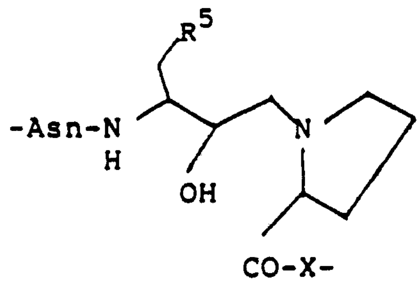

(E)

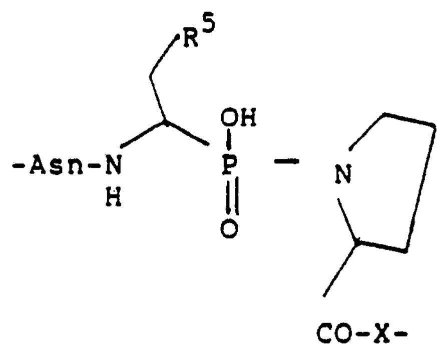

(F)

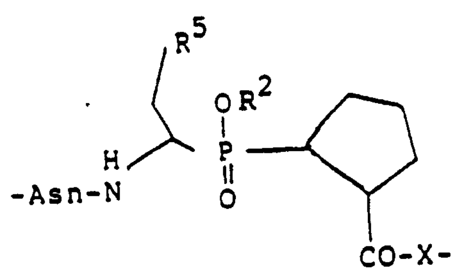

wherein $R^2$ is defined as above and $R^2$ is identical or different for A′ and A″,
wherein $R^5$ is phenyl, p-hydroxyphenyl or cyclohexyl,
wherein X is hydrogen or $R^1$ or a group having the formula

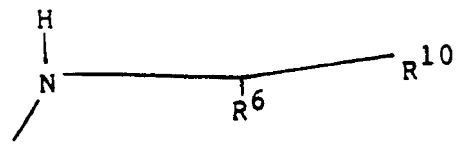

wherein $R^6$ is selected from the group consisting of methyl, ethyl, propyl, isopropyl, butyl, sec.-butyl, isobutyl, cyclohexyl, phenyl, pyridyl and is unsubstituted or substituted by a group which is selected from the group consisting of hydrogen,

$-\overset{O}{\overset{\|}{C}}-R^1$ or phenyl, pyridyl, naphthyl, methyl, ethyl, propyl, isopropyl, butyl, sec.-butyl or isobutyl, wherein $R^1$ is defined as before.

$R^{10}$ is hydrogen or

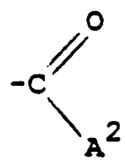 or 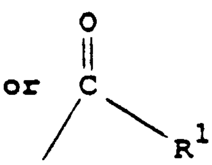

wherein $A^2$ and $R^1$ are defined as before.

The compounds according to the invention may be made by methods which are generally known in peptide chemistry for analogous compounds. In particular it it to be understood that reactive groups not involved in a particular reaction (e.g. amino, carboxyl ,hydroxyl, etc) may be protected by methods standard in peptide chemistry prior to reactions of other groups and subsequently deprotected.

The intermediates of use in production of the end-products are either known compounds or can be made of methods as described in the Examples.

Within the scope of the invention, the substituents in general have the following meaning:

Alkyl in general stands for a branched hydrocarbon radical having 1 to 20 carbon atoms. Lower alkyl having 1 to about 6 carbon atoms is preferred. Examples may include methyl,ethyl, propyl. isopropyl,butyl,isobutyl,pentyl,isopentyl hexyl,isohexyl,heptyl,isoheptyl,octyl, and isooctyl.

Alkenyl in general stands for a straight-chain or branched hydrocarbon radical having 2 to 20 carbon atoms and one or more,preferably one or two, double bonds. The lower alkenyl radical having 2 to about 6 carbon atoms and one double bond is preferred. An alkenyl radical having 2 to 4 carbon atoms and one double bond is particularly preferred. Examples include allyl,propenyl,isopropenyl,butenyl,isobutenyl,pentenyl, isopentenyl,hexenyl,isohexenyl,heptenyl,isoheptenyl, octenyl, and isooctenyl.

Alkinyl in general stands for a straight-chain or branched hydrocarbon radical having 2 to 20 carbon atoms and one or more, preferably one or two,triple bonds. The lower alkinyl radical having 2 to about 6 carbon atoms and one triple bond is preferred. Examples include propinyl,isopropinyl,butinyl, isobutinyl,pentinyl,isopentinyl,hexinyl,isohexinyl, heptinyl, isoheptinyl,octinyl, and isootcinyl.

Cycloalkyl in general stands for a cyclic hydrocarbon radical having 3 to 20 carbon atoms. The lower cycloalkyl radical having 3 to 8 carbon atoms in preferred. The cyclobutane, cyclopentane and cyclohexane ring are particularly preferred. Examples include cyclopentyl,cyclohexyl,cycloheptyl, and cyclooctyl.

Aryl in general stands for an aromatic radical having 6 to about 20 carbon atoms. The lower aryl radical having 6 to 10 carbon atoms is preferred. Particularly preferred aryl radicals are phenyl,naphthyl, and biphenyl.

Aralkyl in general for an aryl radical bonded via alkylene chain and having 7 to about 20 carbon atoms. Aralkyl radicals having 1 to 6 carbon atoms in the aliphatic part and 6 to 12 carbons in the aromatic part are preferred. Examples of aralkyl radicals include benzyl, naphthylmethyl, phenethyl, and phenylpropyl.

Alkoxy in general stands for a straight-chain or branched hydrocarbon radical bonded via an oxygen atom and having 1 to 20 carbon atoms. Lower alkoxy having 1 to about 6 carbon atoms is preferred. An

alkoxy having 1 to 4 carbons is particularly preferred. Examples include methoxy,ethoxy,propoxy,isopropoxy, butoxy, isobutoxy,pentoxy,isopentoxy,hexoxy,isohexoxy,heptoxy, isoheptoxy,octoxy, and isooctoxy.

Aryloxy in general stands for an aromatic radical having 6 to about 20 carbon atoms which are bonded via an oxygen atom. The lower aryloxyl radical having 6 to 10 carbon atoms is preferred. Particularly preferred aryloxy radicals are phenoxy or naphthyoxy. Aralkoxy in general stands for an aralkyl radical having 7 to about 20 carbon atoms, in which the alkylene chain is bonded via an oxygen atom. Aralkoy radicals having 1 to 6 carbon atoms in the aliphatic part and 6 to 12 carbons in the aromatic part are preferred. Examples of aralkoxy radicals include benzyloxy, naphthylmethyloxy, phenethyloxy, and phenylpropyloxy.

Acyl in general stands for phenyl or straight-chain or branched lower alkyl having 1 to about 6 carbon atoms which are bonded via a carbonyl group. Phenyl and alkyl radicals having up to 4 carbon atoms are preferred. Examples include benzoyl, acetyl,ethylcarbonyl,propylcarbonyl,isopropylcarbonyl, butylcarbonyl and isobutylcarbonyl.

Alkoxycarbonyl can be represented, for example, by the formula

- C -O-Alkyl
  ||
  O

where alkyl stands for a straight-chain or branched lower hydrocarbon radical having 1 to 12 carbon atoms. Lower alkoxycarbonyl having 1 to about 6 carbon atoms in the alkyl part is preferred. In particular, alkoxycarbonyl radicals having up to 4 carbon atoms are preferred Examples include methoxycarbonyl, ethoxycarbonyl,propoxycarbonyl, isopropoxycarbonyl, butoxycarbonyl and isobutoxycarbonyl.

Aryloxycarbonyl can be represented, for example, by the formula -COO-aryl. Here, aryl, in general stands for an aromatic radical having 6 to 12 carbon atoms. Examples include phenoxycarbonyl and naphthyloxycarbonyl.

Aralkoxycarbonyl can be represented, for example, by the formula -COO-aralkyl. Here, aralkyl in general stands for an aryl radical having 7 to 14 carbon atoms and bonded via an alkylene chain, aralkyl radicals having 1 to 6 carbons in the aliphatic part and 6 to 12 carbon atoms in the aromatic part are preferred. Examples include benzyloxycarbonyl or naphthylmethyloxycarbonyl.

Heteroaryl in the scope of the abovementioned definitions stand in general for a 5- to 6-membered aromatic ring which can contain oxygen,sulphur, and/or nitrogen as the hetero atom and onto which a further aromatic ring can be fused. 5- to 6-membered aromatic rings which contain one oxygen,one sulphur, and/or up to 2 nitrogen atoms and which are optionally fused to benzene are preferred. Particularly preferred heteroaryl radicals include:thienyl,furyl,pyridyl,pyrimidyl, pyrazinyl,pyridazinyl,quinolyl,isoquinolyl, quinazolyl, quinoxalyl, thiazolyl,benzothiazolyl,isothiazolyl, oxazolyl, benzoxazolyl, isoxazolyl, imidazolyl, benzimidazolyl, pyrazolyl and indolyl.

Halogen in general stands for fluorine, chlorine,bromine or iodine, preferably flourine, chlorine or bromine. Halogen particularly preferred stands for fluorine or chlorine.

Triarylsilyl in general stands for a silyl radical which can be substituted by three aryl radicals having 6 to 12 carbon atoms and which can be identical or different. Triphenylsilyl and trinaphthylsilyl are preferred.

Trialkylsilyl in general stands for a silyl radical which can be substituted by three alkyl radicals which can be identical or different. Substitution by lower alkyl radicals having 1 to about 6 carbon atoms is preferred. Methyl, ethyl, propyl, isopropyl, butyl, sec-butyl, isobutyl, tert.butyl, pentyl, neopentyl, hexyl and thexyl are particularly preferred.

The following description of intermediates defined by formula (III) for the production of therapeutic compounds according to formula (I) wherein $A''$ represents the preferred moiety (B) as defined above. It is also recognized that intermediates defined by formula (III) can be utilized for production of therapeutics compounds defined by formula (I) wherein $A''$ represents (c) and Q = N.

The present invention provides compounds described by the general formula III

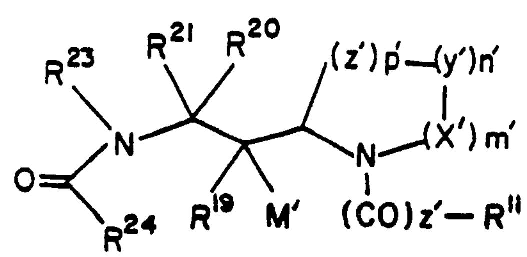

and salts thereof
wherein $R^{11}$ stands for hydrogen or $OR^{12}$ or $NR^{12}R^{13}$ or $CR^{12}R^{13}R^{14}$ or $SiR^{12}R^{13}R^{14}$ and $z'$ is zero (0) or one (1); if $z'$ is zero, $R^{11}$ is hydrogen, and $R^{11}$ and $R^{17}$ do not represent a single bond; if $z'$ is one, $R^{11}$ is not hydroxyl
wherein $R^{12}$,$R^{13}$,$R^{14}$ independently of each other stand for hydroxyl
or unsubstituted or substituted alkyl
or substituted or unsubstituted cycloalkyl
or unsubstituted or substituted aryl
or unsubstituted or substituted heteroaryl
or unsubstituted or substituted aralkyl
(wherein the substituents or said alkyl, cycloalkyl, aralkyl,aryl or heteroaryl are chosen from the group consisting of halogen, alkyl, cycloalkyl,aryl, (heteroaryl,$NR^{15}R^{16}$,hydroxy, alkoxy, aryloxy, aralkoxy
wherein $R^{15}$,$R^{16}$ independently of each other stand for hydrogen or alkyl or cycloalkyl or aryl or aralkyl or heteroaryl
or $R^{15}$ and $R^{16}$ together stand for an alkyl chain with two to ten carbon atoms which can be substituted by alkyl groups
or $R^{11}$ and $R^{17}$ together represent a single bond
(wherein $M'$ stands for $OR^{17}$
wherein $R^{17}$ stands for hydrogen or $CR^{12}R^{13}R^{14}$ or $SiR^{12}R^{13}R^{14}$ or $CO\text{-}R^{18}$
wherein $R^{12}$,$R^{13}$,$R^{14}$ are defined above but can be independently chosen
wherein $R^{18}$ stands for hydrogen or $OR^{12}$ or $NR^{12}R^{13}$ or $CR^{12}R^{13}R^{14}$
wherein $R^{12}$,$R^{13}$,$R^{14}$ are defined above
or $R^{17}$ and $R^{11}$ together represent a single bond
or $R^{17}$ and $R^{23}$ together represent a group $CR^{12}R^{13}$
wherein $R^{12}$,$R^{13}$ are defined above
or $M'$ and $R^{25}$ together represents a single bond
wherein $R^{19}$,$R^{20}$,$R^{21}$ stand for hydrogen or unsubstituted or substituted alkyl or unsubstituted or substituted cycloalkyl or unsubstituted or substituted aryl or unsubstituted or substituted heteroaryl or unsubstituted or substituted aralkyl
wherein the substituents are chosen from the group consisting of $COR^{22}$,$NR^{15}R^{16}$,$SR^{15}$,aryl, heteroaryl,alkyl,cycloalkyl, halogen, hydroxy,alkoxy,aryloxy,or a group having the formula
$R^{15}R^{16}NC(=NH)NH$-
wherein $R^{22}$ stands for hydrogen or $OR^{12}$ or $NR^{12}R^{13}$ or $CR^{12}R^{13}R^{14}$
wherein $R^{12}$,$R^{13}$,$R^{14}$ are defined above
wherein $R^{15}$ and $R^{16}$ are defined above
wherein $R^{23}$ stands for hydrogen or $SiR^{12}R^{13}R^{14}$ or $CR^{12}R^{13}R^{14}$ or $COR^{22}$ or aryl or heteroaryl
wherein $R^{12}$,$R^{13}$,$R^{14}$,$R^{22}$ are defined above
or $R^{23}$ and $R^{17}$ together represent a group $CR^{12}R^{13}$
wherein $R^{12}$ and $R^{13}$ are defined above
wherein $R^{24}$ stands for $OR^{25}$
wherein $R^{25}$ stands for unsubstituted or substituted alkyl or cycloalkyl or for unsubstituted or substituted aryl or unsubstituted or substituted heteroaryl or unsubstituted or substituted aralkyl
wherein the substituents of said groups are chosen from the group consisting of halogen,alkyl, cycloalkyl,aryl,heteroaryl, $NR^{15}R^{16}$,hydroxy,alkoxy,aryloxy, heteroaryloxy
wherein $R^{15}$,$R^{16}$ independently of each other stand for hydrogen, alkyl,cycloalkyl, aryl,aralkyl,heteroaryl
or $R^{24}$ and $R^{17}$ together stand for a single bond
wherein $X'$ stands for $CR^{12}R^{13}$ or $R^{12}C=CR^{13}$ or $C\equiv C$
wherein $R^{12}$ and $R^{13}$ are defined above
wherein $m'$ stands for a number between zero (0) and ten (10), and $2 \leq m' + n' + p' \leq 8$
wherein $Y'$ stands for O or S or $NR^{12}$ or $R^{12}R^{13}N^{+}$ or $SiR^{12}R^{13}$ or $CR^{12}R^{13}$ or $R^{12}C=CR^{13}$ or $C\equiv C$
wherein $R^{12}$,$R^{13}$ are defined above
wherein $n'$ stands for a number between zero (0) and ten (10), and $2 \leq m' + n' + p'' \leq 8$
wherein $Z'$ stands for $CR^{12}R^{13}$ or $R^{12}C=CR^{13}$ or $C\equiv C$
wherein $R^{12}$,$R^{13}$ are defined above
wherein $p'$ stands for a number between zero (0) and ten (10),and $2 \leq m' + n' + p' \leq 8$.

The present invention provides compounds of the general formula III and salts thereof
wherein $R^{11}$ stands for hydrogen or $OR^{12}$ or $NR^{12}R^{13}$ or $CR^{12}R^{13}R^{14}$ or $SiR^{12}R^{13}R^{14}$
$z'$ is zero (0) or one (1); if $z'$ is zero, $R^{11}$ is hydrogen and $R^{11}$ and $R^{17}$ do not represent a single bond; if $z'$

is one, $R^{11}$ is not hydroxyl
wherein $R^{12}$,$R^{13}$,$R^{14}$ independently of each other stand for hydrogen
or unsubstituted or substituted lower alkyl
or substituted or unsubstituted lower cycloalkyl
or unsubstituted or substituted lower aryl
or unsubstituted or substituted lower heteroaryl
or unsubstituted or substituted lower aralkyl
wherein the substituents of said alkyl, cycloalkyl, aralkyl,aryl or heteroaryl group are chosen from the group consisting of halogen, lower alkyl,lower cycloalkyl,lower aryl,lower heteroaryl,$NR^{15}$,$R^{16}$, hydroxy,lower alkoxy,lower aryloxy,lower aralkyloxy,
wherein $R^{15}$,$R^{16}$ independently of each other stand for hydrogen or lower alkyl or lower cycloalkyl or lower aryl or lower aralkyl or lower heteroaryl
or R15 and R16 together stand for an alkyl chain with two to six carbon atoms which can be substituted by lower alkyl
or $R^{11}$ and $R^{17}$ together represent a single bond
wherein $M'$ stands for $OR^{17}$
wherein $R^{17}$ stands for hydrogen or $CR^{12}R^{13}R^{14}$ or $SiR^{12}R^{13}R^{14}$ or $CO\text{-}R^{18}$
wherein $R^{12}$,$R^{13}$,$R^{14}$ are defined above but can be independently chosen
wherein $R^{18}$ stands for hydrogen or $OR^{12}$ or $NR^{12}R^{13}$ or $CR^{12}R^{13}R^{14}$
wherein $R^{12}$,$R^{13}$,$R^{14}$ are defined above
or $R^{17}$ and $R^{11}$ together represent a single bond
or $R^{17}$ and $R^{23}$ together represent a group $CR^{12}R^{13}$
wherein $R^{12}$,$R^{13}$ are defined above
or $M'$ and $R^{25}$ together represent a single bond
wherein $R^{19}$ and $R^{20}$ stand for hydrogen or lower alkyl or lower aryl or lower heteroaryl
wherein $R^{21}$ stands for hydrogen or unsubstituted or substituted lower alkyl
or unsubstituted or substituted lower cycloalkyl
or unsubstituted or substituted lower aryl
or unsubstituted or substituted lower heteroaryl
or unsubstituted or substituted lower aralkyl
wherein the substituents of said groups are chosen from the group consisting of $COR^{22}$,$NR^{15}$R16, $SR^{15}$,lower aryl, lower heteroaryl,lower alkyl,lower cycloalkyl,halogen,hydroxy, lower alkoxy,lower aryloxy,lower aralkyloxy, or a group having the formula
$R^{15}R^{16}NC(=NH)NH\text{-}$
wherein $R^{22}$ stands for hydrogen or $OR^{12}$ or $NR^{12}R^{13}$ or $CR^{12}R^{13}R^{14}$
wherein $R^{12}$,$R^{13}$,$R^{14}$ are defined above
wherein $R^{15}$ and $R^{16}$ are defined above
wherein $R^{23}$ stands for hydrogen or $SiR^{12}R^{13}R^{14}$ or $CR^{12}R^{13}R^{14}$ or $COR^{22}$ or lower aryl or lower heteroaryl
wherein $R^{12}$,$R^{13}$,$R^{14}$,$R^{22}$ are defined above or $R^{23}$ and $R^{15}$ together represent a group $CR^{12}R^{13}$
wherein $R^{12}$ and $R^{13}$ are defined above
wherein $R^{24}$ stands for $OR^{25}$
wherein $R^{25}$ stands for unsubstituted or substituted lower alkyl or lower cycloalkyl
or unsubstituted or substituted lower aryl
or unsubstituted or substituted lower heteroaryl
or unsubstituted or substituted lower aralkyl
wherein the substituents of said groups are chosen from the group consisting of lower alkyl,lower cycloalkyl,lower aryl,lower aralkyl,or lower heteroaryl
or $R^{24}$ and $R^{17}$ together stand for a single bond
wherein $X'$ stands for $CR^{12}R^{13}$ or $R^{12}C=CR^{13}$ or $C\equiv C$
wherein $R^{12}$ and $R^{13}$ are defined above
wherein $m'$ stands for a number between zero (0) and five (5),and $2\leq m'+n'+p'\leq 8$
wherein $Y'$ stands for O or S or $NR^{12}$ or $R^{12}R^{13}N^{+}$ or $SiR^{12}R^{13}$ or $CR^{12}R^{13}$ or $R^{12}C=CR^{13}$ or $C\equiv C$
wherein $R^{12}$,$R^{13}$ are defined above
wherein $n'$ stands for a number between zero (0) and five (5),and $2\leq m'+n'+p''\leq 8$
wherein $Z'$ stands for $CR^{12}R^{13}$ or $R^{12}C=CR^{13}$ or $C\equiv C$
wherein $R^{12}$,$R^{13}$ are defined above
wherein $p'$ stands for a number between zero (0) and five (5),and $2\leq m'+n'+p'\leq 8$.

The present invention provides compounds of the general formula III and salts thereof

wherein $R^{11}$ stands for hydrogen or $OR^{12}$ or $NR^{12}R^{13}$ or $CR^{12}R^{13}R^{14}$ or $SiR^{12}R^{13}R^{14}$
wherein $z'$ is zero (0) or one (1); if $z'$ is zero, $R^{11}$ is hydrogen and $R^{11}$ and $R^{17}$ do not represent a single bond; if $z'$ is one, $R^{11}$ is not hydroxyl
wherein $R^{12}$,$R^{13}$,$R^{14}$ independently of each other stand for hydrogen
or methyl,ethyl,propyl,isopropyl,butyl, isobutyl,sec-butyl,pentyl
or cyclopropyl,cyclobutyl,cyclopentyl
or phenyl,tolyl, naphtyl
or pyridyl,furyl,thienyl
or benzyl,naphtylmethyl,phenetyl,phenylpropyl
or $R^{11}$ and $R^{17}$ together represent a single bond
wherein $M'$ stands for $OR^{17}$
wherein $R^{17}$ stands for hydrogen or $CR^{12}R^{13}R^{14}$ or $SiR^{12}R^{13}R^{14}$ or $CO-R^{18}$
wherein $R^{12}$,$R^{13}$,$R^{14}$ are defined above but can be independently chosen
wherein $R^{18}$ stands for hydrogen or $OR^{12}$ or $NR^{12}R^{13}$ or $CR^{12}R^{13}R^{14}$
wherein $R^{12}$,$R^{13}$,$R^{14}$ are defined above
or $R^{17}$ and $R^{11}$ together represent a single bond
or $R^{17}$ and $R^{23}$ together represent a group $CR^{12}R^{13}$
wherein $R^{12}$,$R^{13}$ are defined above
$M'$ and $R^{25}$ together represent a single bond
wherein $R^{19}$ stands for hydrogen or methyl, ethyl, propyl, isopropyl, butyl, isobutyl, sec-butyl, pentyl, hexyl, phenyl, naphtyl
wherein $R^{20}$ stands for hydrogen
wherein $R^{21}$ stands for hydrogen or unsubstituted or substituted methyl,ethyl,propyl,isopropyl,butyl,isobutyl, sec-butyl,pentyl,hexyl,
or unsubstituted or substituted cyclopropyl,cyclobutyl,cyclopentyl, cyclohexyl,or unsubstituted or substituted phenyl, tolyl,naphtyl or unsubstituted or substituted pyridyl,thienyl, quinolyl,furyl,thiazolyl
or unsubstituted or substituted benzyl, naphtylmethyl, phenetyl,phenylpropyl
wherein the substituents of said groups are chosen from the group consisting of $COR^{22}$,$NR^{15}$,$R^{16}$,$SR^{15}$,phenyl,naphtyl, pyridyl,thienyl, quinolyl, furyl, thiazolyl, imidazoyl, benzimidazoyl, methyl, ethyl, propyl, isopropyl, butyl,isobutyl, sec-butyl,pentyl,cyclopropyl, cyclobutyl, cyclopentyl,cyclohexyl, halogen,hydroxy, methoxy,ethoxy,propoxy,butoxy,phenoxy, naphtoxy, benzyloxy, naphtylmethoxy or a group having the formula
$R^{15}R^{16}NC(=NH)NH-$
wherein $R^{22}$ stands for hydrogen or $OR^{12}$ or $NR^{12}R^{13}$ or $CR^{12}R^{13}R^{14}$
wherein $R^{12}$,$R^{13}$,$R^{14}$ are defined above
wherein $R^{15}$ and $R^{16}$ are independently of each other,hydrogen,methyl,ethyl, propyl, isopropyl,butyl,isobutyl, sec-butyl,pentyl,cyclopropyl, cyclobutyl,cyclopentyl,phenyl, naphtyl,benzyl,naphtylmethyl, phenetyl,phenylpropyl, pyridyl,thienyl,quinolyl,furyl, thiazolyl
or $R^{15}$ and $R^{16}$ together stand for an alkyl chain with two to five carbon atoms
wherein $R^{23}$ stands for hydrogen or $SiR^{12}R^{13}R^{14}$ or $CR^{12}R^{13}R^{14}$ or $COR^{22}$ or phenyl or naphyl
wherein $R^{12}$,$R^{13}$,$R^{14}$,$R^{22}$ are defined above
or $R^{23}$ and $R^{17}$ together represent a group $CR^{12}R^{13}$
wherein $R^{12}$ and $R^{13}$ are defined above
wherein $R^{24}$ stands for $OR^{25}$
wherein $R^{25}$ stands for methyl,ethyl,propyl, isopropyl,butyl,isobutyl,sec-butyl,cyclopropyl, cyclobutyl,cyclopentyl,cyclohexyl,phenyl, naphtyl,thienyl,furyl,pyridyl,fluorenylmethyl, benzyl,naphtylmethyl,phenetyl, phenylpropyl,
or $R^{24}$ and $R^{17}$ together stand for a single bond
wherein $X'$ stands for $CR^{12}R^{13}$ or $R^{12}C=CR^{13}$ or $C≡C$
wherein $R^{12}$ and $R^{13}$ are defined above
wherein $m'$ stands for a number between zero (0) and five (2), and $2 \leq m'+n'+p' \leq 6$
wherein $Y'$ stands for O or S or $NR^{12}$ or $R^{12}R^{13}N^{+}$ or $SiR^{12}R^{13}$ or $CR^{12}R^{13}$ or $R^{12}C=CR^{13}$ or $C≡C$
wherein $R^{12}$,$R^{13}$ are defined above
wherein $n'$ stands for a number between zero (0) and five (2),and $2 \leq m'+n'+p'' \leq 6$
wherein $Z'$ stands for $CR^{12}R^{13}$ or $R^{12}C=CR^{13}$ or C C
wherein $R^{12}$,$R^{13}$ are defined above
wherein $p'$ stands for a number between zero (0) and five (5),and $2 \leq m'+n'+p' \leq 8$.

Example 1 of formula III:

1-O-(t-butyldiphenylsilyl)oxy-4-butanol

A solution of 27 grams(0.300mol) of 1,4-butanediol in 200 ml anhydrous tetrahydrofuran was added dropwise to a vigorously stirred suspension of 9 grams of a 80% dispersion of sodium hydride in mineral oil (0.300mol) in 400 ml anhydrous tetrahydrofuran at 20°C under argon. The suspension was stirred for 2 hours, then a solution of 82.4 grams(0.300mol) of tert-butylchlorodiphenylsilane in 100 ml anhydrous tetrahydrofuran was added dropwise and stirred for 16 hours. The reaction was quenched by the addition of 450 ml sodium carbonate (10%w/w) and stirred for 10 minutes. 300 ml hexane were added and the layers separated. The organic layer was extracted with 450 ml B sodium carbonate (10%w/w), dried with sodium sulfate, filtered and concentrated in vacuo to give 97 grams (98.3%) of crude product as a residue. This was used without further purification.
Rf(1:1 petroleum ether/ethyl ether): 0.55
$C_{20}H_{28}O_2Si$ (328.42)

Example 2 of formula III

1-O-(t-butyldiphenylsilyl)oxy-4-O-(p-toluene sulfonyl)oxy-butane

A solution of 97 grams(0.295mol) of the preceding alcohol in 200 ml anhydrous dichloromethane was added dropwise to a vigorously stirred solution of 56.3 grams(0.295mol) p-toluenesulfonylchloride, 9 grams-(0.074mol) 4-dimethylaminopyridine, 45 ml(0.323mol) triethylamine in 500 ml anhydrous dichloromethane at 0°C under argon. The reaction was cooled to -20°C for 18 hours, then warmed to +20°C and 500 ml deionized water were added and stirred vigorously for 20 minutes. The pH was adjusted to 3 with concentrated hydrochloric acid and the layers separated. The organic phase was dried with magnesium sulfate, filtered and concentrated in vacuo to give 144 grams of yellow residue. The crude product was chromatographed on 800 grams silica gel(60µm) with toluene to give 120.6 grams(84.7%) of the product.
Rf(toluene): 0.51
$C_{27}H_{34}O_4SiS$ (482.62)

Example 3 of formula III

[4-O-(t-butyldiphenylsilyl)oxy]butyl-triphenyl phosphonium tosylate

65.6 grams(0.250mol) triphenylphosphine were added to a reaction vessel containing 120.6 grams-(0.250mol) of the preceding compound under argon. The vessel was heated to 100°C for 36 hours, then cooled to 20°C. 35 ml anhydrous toluene were added and heated to get the solidified material into solution. 250 ml petroleum ether were added and the reaction cooled to 20°C. The liquid phase was removed and the solid phase was dried in vacuo to yield 169.9 grams(91.2%) of the product as a white solid.
$C_{45}H_{49}O_4PSi$ (744.91)

Example 4 of formula III

2-N-(t-butoxycarbonyl)amino-3-phenylpropanal

9.33 grams(0.245mol) lithium aluminum hydride powder were added over 15 minutes to a vigorously stirred solution of 54.0 grams(0.175mol) tert-butoxycarbonyl-L-phenylalanine N-methoxy-N-methylamide in 1.8 liters ethyl ether at 20°C under argon. The suspension was stirred for 2 hours, then 900 ml of aqueous potassium hydrogen sulfate(0.34M) at 5°C was added and stirred for 10 minutes. The layers were separated and the aqueous phase was washed with 6 liters ethyl ether. Ether layers were combined,

concentrated in vacuo to 2 liters and extracted with 3N hydrochloric acid(3x650ml), saturated sodium hydrogen carbonate(2x1liter), saturated sodium chloride(1 liter). The ether layer was dried with magnesium sulfate, filtered and concentrated to yield 39.0 grams (89.3%) of crude product as an oil. According to $^1$H-NMR analysis the crude product contained 65% of the title compound. This product was used without further purification.
Rf(1:1 ethyl acetate/hexane): 0.71
$C_{14}H_{19}NO_3$ (249.31)

Example 5 of formula III

(Z)-2-N-(t-butoxycarbonyl)amino-7-O-(t-butyl diphenylsilyl)oxy-1-phenyl-3-heptene

A solution of 20.27 grams(0.105mol) sodium bis(trimethylsilyl)amide(95%) in 100 ml tetrahydrofuran was added over 40 minutes to a stirred solution of 78.2 grams(0.105mol) [4-O-(t-butyldiphenylsilyl)oxy]-butyl-triphenyl- phosphonium tosylate in 700 ml anhydrous tetrahydrofuran at -80° C under argon. After warming to 0° C for 100 minutes, the mixture was cooled to -80° C. This was added over 75 minutes to a stirred solution of 39.0 grams(0.101mol) of the preceding aldehyde in 100 ml anhydrous tetrahydrofuran at -80° C. The mixture was warmed to 20° C and stirred for 1 hour. 18 grams celite and 200 ml hexane were added and then filtered through 55 grams celite in 800 ml hexane. The pad was washed with 400 ml hexane. Filtrates were combined and concentrated in vacuo to yield 96 grams orange solid. The crude material was chromatographed on 500 grams silica gel(60µm) with 3:1 petroleum ether/dichloromethane to yield 32.2 grams(56%) of product.
Rf(toluene): 0.50
$C_{34}H_{45}NO_3Si$ (543.82)

Example 6 of formula III

(Z)-2-N-(t-butoxycarbonyl)amino-1-phenyl-3-heptene-7-ol

A solution of 19.88 grams(0.063mol) tetrabutylammoniumfluoride trihydrate in 150 ml tetrahydrofuran was added over 5 minutes to a stirred solution of 30.55 grams(0.056mol) of the preceding compound in 200 ml tetrahydrofuran at 0° C under argon. After 5 hours 0.063N acetic acid(100ml) and 200 ml hexane were added. Layers were separated and the aqueous layer was extracted with 100 ml hexane. Organic layers were combined and extracted with saturated sodium hydrogen carbonate(125 ml), saturated sodium chloride(75 ml), dried with magnesium sulfate, filtered and concentrated in vacuo to 33.22 grams yellow residue. The crude product was chromatographed on 150 grams silica gel(60µm) with 10:1 dichloromethane/methanol to yield 12.7 grams(74%) of product as an off white solid.
Rf(1:1 petroleum ether/ethyl ether): 0.32
$C_{18}H_{27}NO_3$ (305.56)

Example 7 of formula III

(Z)-2-N-(t-butoxycarbonyl)amino-1-phenyl-7-O-(p-toluenesulfonyl)oxy-3-heptene

7.55 ml(0.054mol) triethylamine, 1.27 grams (0.010mol) 4-dimethylaminopyridine were added to a stirred solution of 12.7 grams(0.042mol) of the preceding alcohol in 250 ml anhydrous dichloromethane at -1° C under argon. 8.90 grams (0.046mol) p-toluenesulfonylchloride in 100 ml anhydrous dichloromethane were added and stirred for 15 hours. 25 ml deionized water were added and stirred vigorously for 20 minutes. 0.24N hydrochloric acid(100ml) was added. Layers were separated and the organic phase was extracted with 0.12N hydrochloric acid(100ml), saturated sodium hydrogen carbonate(100ml), dried with magnesium sulfate, filtered and concentrated to 19.9 grams yellow residue. The crude material was chromatographed on 130 grams silica gel(60µm) with 10:1 dichloromethane/methanol to yield 17.3 grams-(90%) of product as an off white solid
Rf(1:1 petroleum ether/ethyl ether): 0.57
$C_{25}H_{33}NO_5S$ (459.67)

Example 8 of formula III

2-N-(t-butoxycarbonyl)amino-3,4-epoxy-1-phenyl-7-O-(p-toluenesulfonyl)oxy-heptane

1.4 liters 3-chloroperbenzoic acid(0.12N in dichloromethane) were added to 17.3 grams (0.038mol) of the preceding compound and stirred at 20°C under argon. After all starting material had been used up (TLC control), 1N sodium thiosulfate(500ml) was added and stirred for 15 minutes. The layers were separated and the organic phase was extracted with saturated sodium hydrogen carbonate(250ml), 50% saturated sodium hydrogen carbonate(400ml), saturated sodium chloride(600ml), dried with magnesium sulfate, filtered and concentrated in vacuo to a yellow residue. The crude product was chromatographed on 700 grams silica gel(60µm) equilibrated to petroleum ether. The column was eluted with petroleum ether/dichloromethane/ethyl ether gradient to yield 9.76 grams(55%) of product as a light yellow solid.
Rf(1:1 petroleum ether/ethyl ether): 0.29
$C_{25}H_{33}NO_6S$ (475.67)

Example 9 of formula III

7-azido-2-N-(t-butoxycarbonyl)amino-3,4-epoxy-1-phenyl-heptane

1.09 grams(0.022mol) lithium azide were added to a stirred solution of 9.6 grams(0.020mol) of the preceding compound in 60 ml anhydrous dimethylformamide at 20°C under argon. After 18 hours the reaction was concentrated in vacuo to a black residue, dissolved in 200 ml anhydrous dichloromethane and extracteo with saturated sodium hydrogen carbonate (4x200ml), saturated sodium chloride(125ml), dried over magnesium sulfate, filtered and concentrated in vacuo to yield 7.35 grams(105%) of crude product as a light brown solid. According to NMR analysis the crude product contained 95% of the title compound and was used without further purification.
Rf(1:1 petroleum ether/ethyl ether): 0.50
$C_{18}H_{26}N_4O_3$ (346.43)

Example 10 of formula III

2-[2-N-(t-butoxycarbonyl)amino-1-hydroxy-3-phenyl]propyl-pyrrolidine

0.339 grams 5% palladium on calcium carbonate were added to a solution of 0.97 grams(0.0028mol) of the preceding compound in 50 ml ethanol (200proof). The reaction vessel was evacuated and filled 3 times with hydrogen gas and stirred vigorously. Hydrogen gas was bubbled through the vessel at 1 atmosphere pressure for 1 hours, then the reaction mixture was filtered through 5 grams celite. The pad was washed with 30 ml dichloromethane. Filtrates were combined and concentrated to 1.23 grams black residue. The crude product was chromatographed on 89 grams silica gel(60µm) with dichloromethane/methanol/ ammonia gradient to yield 0.44 grams(50%) product as a white solid.
Rf(5:1 dichloromethane/methanol): 0.15
$C_{18}H_{26}N_2O_3$ (320.43)
$^{13}C$-NMR 300MHz($CD_2Cl_2$) $\delta$ = 26.69; 28.72; 28.97; 40.12; 46.44; 54.09; 61.77; 73.06; 79.32; 126.73; 128.88; 130.06; 139.49; 156.33

The following description of intermediates defined by formula (IV) for the production of therapeutic compounds according to formula (I) wherein A″ represents the preferred moiety (C) as defined above. It is also recognized that intermediates defined by formula (IV) can be utilized for the production of therapeutic compounds defined by formula (I) wherein formula A″ represents (a) and (c) wherein if A″ is (c), $Q = CR^2$

and $R^2$ is defined above.

The present invention provides compounds described by the general formula IV

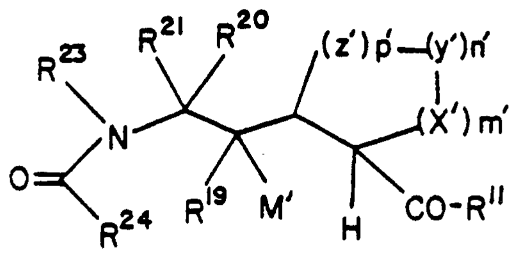

and salts thereof
wherein $R^{11}$ stands for hydrogen or $OR^{12}$ or $NR^{12}R^{13}$ or $CR^{12}R^{13}R^{14}$ or $SiR^{12}R^{13}R^{14}$
wherein $R^{12}$,$R^{13}$,$R^{14}$ independently of each other stand for hydrogen
or unsubstituted or substituted alkyl
or substituted or unsubstituted cycloalkyl
or unsubstituted or substituted aryl
or substituted or unsubstituted heteroaryl
or unsubstituted or substituted aralkyl
wherein the substituents of said alkyl, cycloalkyl, aralkyl,aryl or heteroaryl are chosen from the group consisting of halogen, alkyl,cycloalkyl, aryl, heteroaryl,$NR^{15}R^{16}$,hydroxy, alkoxy, aryloxy, aralkoxy
wherein $R^{15}$,$R^{16}$ independently of each other stand for hydrogen or alkyl or cycloalkyl or aryl or aralkyl or heteroaryl or $R^{15}$ and $R^{16}$ together stand for an alkyl chain with two to ten carbon atoms which can be substituted by alkyl groups
or $R^{11}$ and $R^{17}$ together represent a single bond
wherein $M'$ stands for $OR^{17}$
wherein $R^{17}$ stands for hydrogen or $CR^{12}R^{13}R^{14}$ or $SiR^{12}R^{13}R^{14}$ or CO-$R^{18}$
wherein $R^{12}$,$R^{13}$,$R^{14}$ are defined above but can be independently chosen
wherein $R^{18}$ stands for hydrogen or $OR^{12}$ or $NR^{12}R^{1}3$ or $CR^{12}R^{13}R^{14}$
wherein $R^{12}$,$R^{13}$,$R^{14}$ are defined above
or $R^{17}$ and $R^{11}$ together represent a single bond
or $R^{17}$ and $R^{23}$ together represent a group $CR^{12}R^{13}$
wherein $R^{12}$,$R^{13}$ are defined above or $M'$ and $R^{25}$ together represent a single bond wherein $R^{19}$,$R^{20}$,$R^{21}$ stand for hydrogen or unsubstituted or alkyl or unsubstituted or substituted cycloalkyl unsubstituted or substituted aryl or unsubstituted or substituted heteroaryl or unsubstituted or substituted aralkyl
wherein the substituents are chosen from the group consisting of $COR^{22}$,$NR^{15}R^{16}$,$SR^{15}$,aryl, heteroaryl,alkyl, cycloalkyl, halogen, hydroxy,alkoxy,aryloxy,or a group having the formula
$R^{15}R^{16}NC(=NH)NH$-
wherein $R^{22}$ stands for hydrogen or $OR^{12}$ or $NR^{12}R^{13}$ or $CR^{12}R^{13}R^{14}$
wherein $R^{12}$,$R^{13}$,$R^{14}$ are defined above
wherein $R^{15}$ and $R^{16}$ are defined above
wherein $R^{23}$ stands for hydrogen or $SiR^{12}R^{13}R^{14}$ or $CR^{12}R^{13}R^{14}$ or $COR^{22}$ or aryl or heteroaryl
wherein $R^{12}$,$R^{13}$,$R^{14}$,$R^{22}$ are defined above or $R^{23}$ and $R^{17}$ together represent a group $CR^{12}R^{13}$
wherein $R^{12}$ and $R^{13}$ are defined above
wherein $R^{24}$ stands for $OR^{25}$
wherein $R^{25}$ stands for unsubstituted or substituted alkyl or cycloalkyl or for unsubstituted or substituted aryl or unsubstituted or substituted heteroaryl or unsubstituted or substituted aralkyl
wherein the substituents of said groups are chosen from the group consisting of halogen, alkyl, cycloalkyl,aryl,heteroaryl, $NR^{15}R^{16}$,hydroxy,alkoxy,aryloxy, heteroaryloxy
wherein $R^{15}$,$R^{16}$ independently of each other stand for hydrogen,alkyl,cycloalkyl, aryl,aralkyl,heteroaryl
or $R^{24}$ and $R^{17}$ together stand for a single bond
wherein $X'$ stands for $CR^{12}R^{13}$ or $R^{12}C=CR^{13}$ or $C\equiv C$
wherein $R^{12}$ and $R^{13}$ are defined above
wherein $m'$ stands for a number between zero (0) and ten (10), and $2 \leq m' + n' + p' \leq 8$
wherein $Y'$ stands for O or S or $NR^{12}$ or $R^{12}R^{13}N^{+}$ or $SiR^{12}R^{13}$ or $CR^{12}R^{13}$ or $R^{12}C=CR^{13}$ or $C\equiv C$
wherein $R^{12}$,$R^{13}$ are defined above

wherein $n'$ stands for a number between zero (0) and ten (10),and $2 \leq m' + n' + p'' \leq 8$
wherein $Z'$ stands for $CR^{12}R^{13}$ or $R^{12}C = CR^{13}$ or $C \equiv C$
wherein R12,$R^{13}$ are defined above
wherein $p'$ stands for a number between zero (0) and ten (10), and $2 \leq m' + n' + p' \leq 8$.

This invention provides compounds described by the general formula IV and salts thereof
wherein $R^{11}$ stands for hydrogen or $OR^{12}$ or $NR^{12}R^{13}$ or $CR^{12}R^{13}R^{14}$ or $SiR^{12}R^{13}R^{14}$
wherein $R^{12}$,$R^{13}$,$R^{14}$ independently of each other stand for hydrogen
or unsubstituted or substituted lower alkyl
or substituted or unsubstituted lower cycloalkyl
or unsubstituted or substituted lower aryl
or unsubstituted or substituted lower heteroaryl
or unsubstituted or substituted aralkyl
wherein the substituents of said alkyl, cycloalkyl, aralkyl,aryl or heteroaryl group are chosen from the group consisting of halogen, lower alkyl,lower cycloalkyl,lower aryl,lower heteroaryl, $NR^{15}R^{16}$, hydroxy,lower alkoxy,lower aryloxy, lower aralkyloxy,
wherein $R^{15}$,$R^{16}$ independently of each other stand for hydrogen or lower alkyl or lower cycloalkyl or lower aryl or lower aralkyl or lower heteroaryl
or $R^{15}$ and $R^{16}$ together stand for an alkyl chain with two to six carbon atoms which can be substituted by lower alkyl
or $R^{11}$ and $R^{17}$ together represent a single bond
wherein $M'$ stands for $OR^{17}$
wherein $R^{17}$ stands for hydrogen or $CR^{12}R^{13}R^{14}$ or $SiR^{12}R^{13}R^{14}$ or CO-$R^{18}$
wherein $R^{12}$,$R^{13}$,$R^{14}$ are defined above but can be independently chosen
wherein $R^{18}$ stands for hydrogen or $OR^{12}$ or $NR^{12}R^{1}3$ or $CR^{12}R^{13}R^{14}$
wherein $R^{12}$,$R^{13}$,$R^{14}$ are defined above
or $R^{17}$ and $R^{11}$ together represent a single bond
or $R^{17}$ and $R^{23}$ together represent a group $CR^{12}R^{13}$
wherein $R^{12}$,$R^{13}$ are defined above
or $M'$ and $R^{25}$ together a single bond
wherein $R^{19}$ and $R^{20}$ stand for hydrogen or lower alkyl
wherein $R^{21}$ stands for hydrogen or unsubstituted or lower alkyl
or unsubstituted or substituted lower cycloalkyl
or unsubstituted or substituted lower aryl
or unsubstituted or substituted lower heteroaryl
or unsubstituted or substituted lower aralkyl
wherein the substituents of said groups are chosen from the group consisting of $COR^{22}$, $NR^{15}R^{16}$,$SR^{15}$,lower aryl, lower heteroaryl, lower alkyl, lower cycloalkyl,halogen,hydroxy,lower alkoxy, lower aryloxy,lower aralkyloxy, or a group having the formula $R^{15}R^{16}NC(=NH)NH$-
wherein $R^{22}$ stands for hydrogen or $OR^{12}$ or $NR^{12}R^{13}$ or $CR^{12}R^{13}R^{14}$
wherein $R^{12}$,$R^{13}$,$R^{14}$ are defined above
wherein $R^{15}$ and $R^{16}$ are defined above
wherein $R^{23}$ stands for hydrogen or $SiR^{12}R^{13}R^{14}$ or $CR^{12}R^{13}R^{14}$
or $COR^{22}$ or lower aryl or lower heteroaryl
wherein $R^{12}$,$R^{13}$,$R^{14}$,$R^{22}$ are defined above
or $R^{23}$ and $R^{15}$ together represent a group $CR^{12}R^{13}$
wherein $R^{12}$ and $R^{13}$ are defined above
wherein $R^{24}$ stands for $OR^{25}$
wherein $R^{25}$ stands for unsubstituted or substituted lower alkyl or lower cycloalkyl or for unsubstituted or substituted lower aryl or unsubstituted or substituted lower heteroaryl or unsubstituted or substituted lower aralkyl
wherein the substituents of said groups are chosen from the group consisting of lower alkyl,lower cycloalkyl,lower aryl,lower aralkyl,or lower heteroaryl
or $R^{24}$ and $R^{17}$ together stand for a single bond
wherein $X'$ stands for $CR^{12}R^{13}$ or $R^{12}C = CR^{13}$ or C C
wherein $R^{12}$ and $R^{13}$ are defined above
wherein $m'$ stands for a number between zero (0) and five (5), and $2 \leq m' + n' + p' \leq 8$
wherein $Y'$ stands for O or S or $NR^{12}$ or $R^{12}R^{13}N^{+}$ or $SiR^{12}R^{13}$ or $CR^{12}R^{13}$ or $R^{12}C = CR^{13}$ or C C

wherein $R'^{2}$,$R^{13}$ are defined above
wherein $n'$ stands for a number between zero (0) and five (5), and $2 \leq m' + n' + P'' \leq 8$
wherein $Z'$ stands for $CR^{12}R^{13}$ or $R^{12}C = CR^{12}$ or C C
wherein R12.$R^{13}$ are defined above
wherein $p'$ stands for a number between zero (0) and five (5), and $2 \leq m' + n' + p' \leq 8$.

This invention provides compounds described by the general formula IV and salts thereof
wherein $R'^{1}$ stands for hydrogen or $OR^{12}$ or $NR^{12}R^{13}$ or $CR^{12}R^{13}R^{14}$ or $SiR^{12}R^{13}R^{14}$
wherein $R'^{2}$,$R^{13}$,$R^{14}$ independently of each other stand for hydrogen
or methyl,ethyl,propyl,isopropyl,butyl, isobutyl,sec-butyl, pentyl
or cyclopropyl,cyclobutyl,cyclopentyl
or phenyl,tolyl,naphtyl
or pyridyl,furyl,thienyl
or benzyl,naphtylmethyl,phenetyl,phenylpropyl
or $R^{11}$ and $R'^{7}$ together represent a single bond
wherein $M'$ stands for $OR'^{7}$
wherein $R'^{7}$ stands for hydrogen or $CR^{12}R'^{3}R^{14}$ or $SiR'^{2}R'^{3}R^{14}$ or CO-$R^{18}$
wherein $R'^{2}$,$R^{13}$,$R^{14}$ are defined above but can be independently chosen
wherein $R'^{8}$ stands for hydrogen or $OR^{12}$ or $NR^{12}R^{13}$ or $CR^{12}R^{13}R^{14}$
wherein $R'^{2}$,$R^{13}$,$R^{14}$ are defined above
or $R'^{7}$ and $R^{11}$ together represent a single bond
or $R'^{7}$ and $R^{23}$ together represent a group $CR'^{2}R^{13}$
wherein $R'^{2}$,$R^{13}$ are defined above
or $M'$ and $R^{25}$ together represent a single bond
wherein $R'^{9}$ stands for hydrogen or methyl, ethyl, propyl, isopropyl, butyl, sec-butyl, isobutyl, pentyl, hexyl, phenyl, naphtyl,
wherein $R^{20}$ stands for hydrogen
wherein $R^{2'}$ stands for hydrogen or unsubstituted or substituted methyl,ethyl, propyl,isopropyl,butyl,isobutyl sec-butyl,pentyl,hexyl,
or unsubstituted or substituted cyclopropyl,cyclobutyl, cyclopentyl,cyclohexyl,
or unsubstituted or substituted phenyl,tolyl,naphtyl
or unsubstituted or substituted pyridyl,thienyl, quinolyl,furyl,thiazolyl
or unsubstituted or substituted benzyl, naphtylmethyl, phenetyl,phenylpropyl
wherein the substituents of said groups are chosen from the group
consisting of $COR^{22}$,$NR^{15}R^{16}$.$SR^{15}$,phenyl, naphtyl,pyridyl,thienyl,quinolyl,furyl, thiazolyl,imidazoyl methyl,ethyl,propyl,isopropyl,butyl,isobutyl, sec-butyl,pentyl,cyclopropyl,cyclobutyl, cyclopentyl,cyclohexyl, halogen,hydroxy,methoxy,ethoxy, propoxy,butoxy,phenoxy,naphtoxy,benzyloxy, naphtylmethoxy
or a group having the formula
$R'^{5}R'^{6}NC(=NH)NH$-
wherein $R^{22}$ stands for hydrogen or $OR^{12}$ or $NR'^{2}R^{13}$ or $CR^{12}R^{13}R^{14}$
wherein $R'^{2}$,$R^{13}$,$R^{14}$ are defined above
wherein $R'^{5}$ and $R'^{6}$ are independently of each other hydrogen,methyl,ehtyl,propyl, isopropyl,butyl,isobutyl,sec-butyl, pentyl,cyclopropyl,cyclobutyl, cyclopentyl,phenyl,naphtyl,benzyl, naphtylmethyl,phenetyl,phenylpropyl, pyridyl,thienyl,quinolyl,furyl, thiazolyl
or $R'^{5}$ and $R'^{6}$ together stand for an alkyl chain with two to five carbon atoms
wherein $R^{23}$ stands for hydrogen or $SiR^{12}R^{13}R^{14}$ or $CR^{12}R^{13}R^{14}$
or $COR^{22}$ or phenyl or naphtyl
wherein $R'^{2}$,$R^{13}$,$R^{14}$,$R^{22}$ are defined above
or $R^{23}$ and $R'^{7}$ together represent a group $CR'^{2}R^{13}$
wherein $R'^{2}$ and $R^{13}$ are defined above
wherein $R^{24}$ stands for $OR^{25}$ wherein $R^{25}$ stands for methyl,ethyl,propyl,isopropyl, butyl,isobutyl,sec-butyl,cyclopropyl,cyclobutyl, cyclopentyl,cyclohexyl,phenyl,naphtyl,thienyl, furyl,pyridyl,fluorenylmethyl,benzyl, naphtylmethyl,phenetyl,phenylpropyl,
or $R^{24}$ and $R'^{7}$ together stand for a single bond
wherein $X'$ stands for $CR'^{2}R^{13}$ or $R^{12}C = CR^{13}$ or C≡C
wherein $R'^{2}$ and $R'^{3}$ are defined above
wherein $m'$ stands for a number between zero (0) and five (2), and $2 \leq m' + n' + p' \leq 6$
wherein $Y'$ stands for O or S or $NR^{12}$ or $R^{12}R'^{3}N^{+}$ or $SiR^{12}R^{13}$ or $CR^{12}R^{13}$ or $R^{12}C = CR^{13}$ or C≡C
wherein $R'^{2}$,$R^{13}$ are defined above

wherein n′ stands for a number between zero (0) and five (2), and $2 \leq m' + n' + p'' \leq 6$
wherein Z′ stands for $CR^{12}R^{13}$ or $R^{12}C = CR^{13}$ or C≡C
wherein $R^{12}$,$R^{13}$ are defined above
wherein p′ stands for a number between zero (0) and

The present invention provides a process for the preparation of compounds described by formula IV characterized in that compounds of the general formula V

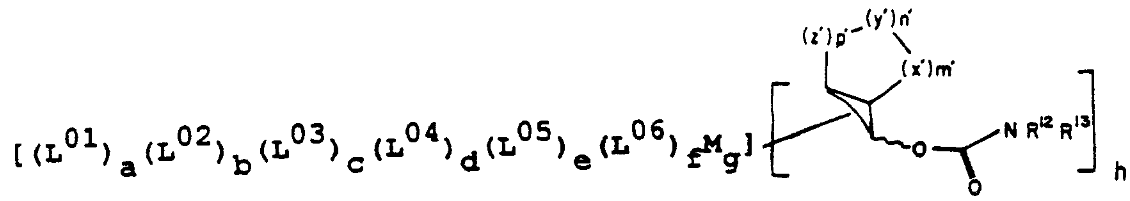

wherein X′,Y′,Z′ are defined above
wherein m′,n′,p′ are defined above
wherein $R^{12}$,$R^{13}$ are defined above
wherein $L^{01}$, $L^{02}$, $L^{03}$, $L^{04}$, $L^{05}$, $L^{06}$ independently of each other stand for hydrogen or halogen or $OR^{12}$ or $NR^{12}R^{13}$ or $CR^{12}R^{13}R^{14}$ or $PR^{12}R^{13}R^{14}$ or $NR^{12}R^{13}R^{14}$
or substituted or unsubstituted cyclopentadienyl
or unsubstituted or substituted lower aryl
or unsubstituted or substituted lower heteroaryl
wherein $R^{12}$,$R^{13}$,$R^{14}$ are defined above
or two (2) of the mentioned substituents together stand for $L^{08}(L^{09})_jL^{10}$
wherein $L^{08}$,$L^{10}$ independently of each other stand for $OR^{12}$,$NR^{12}$,$R^{13}$,$PR^{12}R^{13}$,$NR^{12}$
wherein $R^{12}$,$R^{13}$ are defined above
wherein L09 stands for $CR^{12}R^{13}$
wherein $R^{12}R^{13}$ are defined above
and if j>1 the substituents of the different carbon atoms can be chosen independently
wherein j stands for a number between one (1) and five (5)
wherein a, b, c, d, e, f, stand independently of each other for a number between zero (0) and six (6),
and $a + b + c + d + e + f + h = n$
wherein n stands for a number between two (2) and ten (10),
and n must not exceed the maximal number of ligands for a given metal-nucleus,
wherein M stands for lithium, sodium, potassium boron, magnesium, aluminum, silicon, titanium, vanadium, chromium, manganese, germanium, zirconium,niobium, molybdenum, tin, antimony, lanthanides, hafnium, tantalum, tungsten, rhenium
wherein g stands for a number between one (1) and five (5),
and if g>1 M can represent more than one metal
wherein h stands for a number between one (1) and five (5)
are reacted with aldehydes or ketones of formula VI

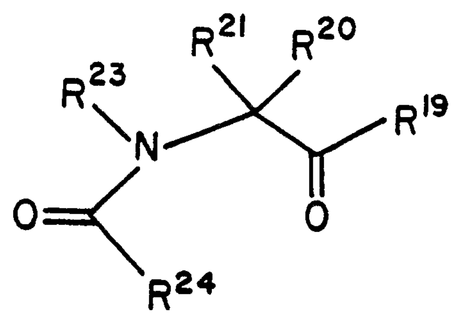

wherein $R^{19}$, $R^{20}$,$R^{21}$,$R^{23}$,$R^{24}$ are defined above
in inert solvents which is followed by oxidation under acidic conditions.

The compounds according to formula V employed as starting materials are known or can be prepared

by known methods, for example by the reaction of esters of the formula VII or VIII with a base which can be followed by the treatment with a metal containing compound of the formula IX

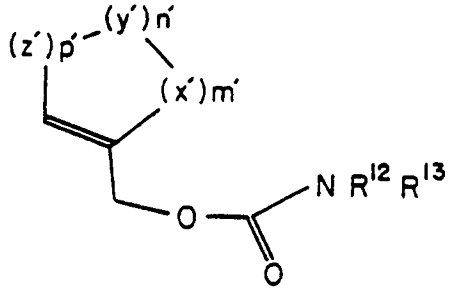

VII

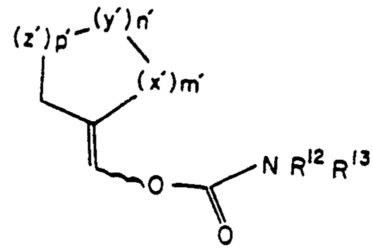

VIII

$(L^{01})_a(L^{02})_b(L^{03})_c(L^{04})_d(L^{05})_e(L^{06})_f M_g(L^{07})_i$ IX

wherein $R^{12}$,$R^{13}$ are defined above
wherein X′,Y′,Z′ are defined above
wherein m′,n′,p′ are defined above
wherein $L^{01}$, $L^{02}$, $L^{03}$, $L^{04}$, $L^{05}$, $L^{06}$ are defined above
wherein M is defined above
wherein $L^{07}$ stands for halogen or $OR^{12}$ or $NR^{12}R^{13}$ or $CR^{12}R^{13}R^{14}$ or $PR^{12}R^{13}R^{14}$ or $NR^{12}R^{13}R^{14}$
wherein $R^{12}$, $R^{13}$,$R^{14}$ are defined above
wherein a, b, c, d, e, f, g are defined above
wherein i stands for a number between zero (0) and five (5).
and $a+b+c+d+e+f+i=u$
wherein n stands for a number between two (2) and ten (10) and u must not exceed the maximal number of ligands for a given metal nucleus

It is particularly understood that the allyl group in compounds of formula V can be bound to the metal nucleus not only in a trihapto ($h^3$) but also in a monohapto ($h^1$) manner.

The carbonyl compounds of formula VII employed as starting materials are known or can be prepared by known methods, for example by the reaction of alcohols of formula X with Carbamic acid halogenides of formula XI wherein X′, Y′, Z′, m′, n′, p′, $R^{12}$, $R^{13}$ are defined above.

The carbonyl compounds of formula VIII employed as starting materials are known or can be prepared by known methods, for example by the reaction of enolates of formula XII with carbamic acid halogenides of formula XI

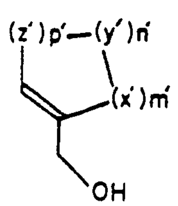

X

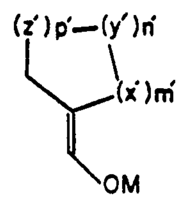

XII

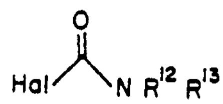

XI

wherein X′,Y′,Z′ are defined above
wherein m′,n′,p′ are defined above
wherein $R^{12}$,$R^{13}$ are defined above
wherein Hal stands for halogen
wherein M is defined above

The aldehydes or ketones of formula VI are known or can be prepared by known methods [Synthesis 676 (1983)]. Oxidations under acidic conditions which are suitable here are processes which utilize known

oxidizing agents at a $p_H < 7$

Inert solvents which are suitable here are organic solvents which are not changed under the reaction conditions. These preferably include ethers such as diethyl ether, butyl methyl ether, dioxane, tetrahydrofuran, glycol-dimethyl ether, or hydrocarbons such as benzene, toluene, xylene or mineral oil fractions, or ureas such as DMEU (N,N-dimethyl-ethylene-urea) or DMPU (N,N-dimethyl-propylene-urea) or amines such as trimethylamine, triethylamine, tetramethylethylendiamine. In addition it is possible to use mixtures of the solvents mentioned.

Alkali metal alkoxides or alkali metal alkyls or alkali metal amides are in general used as bases, preferably lithiumalkyls and lithium amides.

The reaction is in general carried out at atmospheric, elevated or reduced pressure (for example in the pressure range from 0.1 to 10 bar). In general, the reaction is carried out at atmospheric pressure.

The reaction is in general carried out in a temperature range from -125° C to + 50° C, preferably from -100° to + 20° C.

Example 1 of formula IV

1-Cyclopentenylmethanol (1)

9.61 g (0.1 mol) 1-Cyclopentenecarboxaldehyde(L.F. Tietze, T. Eicher: Reacktionen und Synthesen im organisch-chemischen Praktikum, S. 231, Thieme Verlag,Stuttgart, New York 1981) was dissolved in 150 ml n-hexane and cooled to 0° C. Then 120 ml of a 1 M diisobutylaluminumhydride solution in hexane was added at such a rate that the temperature did not rise above 5° C. After the addition, the solution was stirred for 30 minutes at 0° C. Then 20 ml MeOH followed by 20 ml saturated $NH_4Cl$ solution and 100 ml 2 N HCl was added to the reaction mixture at room temperature. The aqueous phase was separated and extracted 3 times with 150 ml ether, the combined organic phases shaken 2 times with 150 ml saturated $NaHCO_3$ solution and dried over $MgSO_4$. After removal of solvent in vacuo and fractionation of residual liquid there was obtained 9.32g (95%) 1, bp 58° C./10 Torr).

Example 2 of formula IV

1-Cyclopentenylmethyl-N,N-diisopropylcarbamate (2) was prepared from 1 and N,N-Diisopropylcarbamoyl chloride according to the literature, (D. Hoppe,R. Hanko, A. Broenneke, F. Lichtenberg, Angew.Chem.**93** (1981) 1106:Angew. Chem., Int. Ed. Engl.**20**, (1981) 1024; D. Hoppe,R.Hanko, A. Broenneke: Angew. Chem.**92** (1980) 637, Angew.Chem. Int. Ed. Engl.**19**(1980) 625.

Example 3 of formula IV

(S)-2-(t.-Butoxycarbonyl)amino-3-phenylpropanal (4) was prepared from (S)-2-tert.-Butoxycarbonylamino-N-methoxy-N-methyl-3-phenyl-propanamide according to the literature (J.-A.Fehrentz, B.Castro, Synthesis (1983) 676.

Example 4 of formula IV

[1S,1(1S,2Z),2S] and [1R,1(1R,2Z),2S]-2-tert.-Butoxycarbonyl-amino-1-(N,N-diisopropylcarbamoyloxymethylene)] cyclopentyl-3-phenyl-1-propanol (3a) and (3b)

9.02 g (40mmol) 2 and 5.1g (44mmol) N,N,N′,N′-Tetramethylethylendiamin was di ssolved in 120 ml. ether and cooled to -78° C. Then 26.2 ml (44mmol) of a 1.68M n-Buli solution in n-hexane was slowly added so that the temperature did not rise above -70° C. After crystallization of the deprotonated 2, the reaction mixture was allowed to stand 20 minutes,then 47.6 ml (160 mmol) tetraisopropoxytitanium was added (reaction temp. during addition held below -60° C), whereby the precipitate again dissolved. After 30 minutes stirring at -78° C, 4.98 g (20 mmol) 4 suspended in 30 ml ether was added dropwise and the

mixture stirred an additonal 30 minutes. To complete the reaction the mixture was allowed to warm to room temperature, then diluted with 100 ml. ether and added to 800 ml. 2N HCl. After separation of the clear phases and 2x extraction with 150 ml ether, the organic phase was shaken with sat. $NaHCO_3$ solution to remove acid then dried over $MgSO_4$. Removal of solvent in vacuo and chromatography on silica with ethylacetate/n-hexane gave 3.949 g (41.6%) **3a** and 4.699 g (49.5%) 3b.

3a: Oil, $R_f$ = 0.38 (Ethylacetate/n-Hexane 1:3)

IR (Film) .3600-3300 (OH,NH), 1700 $cm^{-1}$ (N-C=0).

[ $]^{20}_D$ = +16.8°(c=2,$CH_2Cl_2$).

| $C_{27}H_{42}O_5N_2$ (474.64). | | | |
|---|---|---|---|
| Calc.: | C=68.33<br>H= 8.92 | Found: | C=68.28<br>H= 9.03 |

3b.: White solid, mp 107°C(Hexane),

$R_f$=0.23 (EtoAc/n-Hexan 1:3).

IR (KBr): 3600-3300 (OH, NH), 1700 $cm^{-1}$ (N-C=0).

[ $]^{20}_D$ = +94.2° (c=2, $CH_2Cl_2$).

| $C_{27}H_{42}O_5N_2$ (474.64). | | | |
|---|---|---|---|
| Calc.: | C=68.33<br>H= 8.92 | Found: | C=68.45<br>H= 8.97 |

Example 5 of formula IV

[4S,4S,5(1S.2Z)]-and[4S,5R,5(1R,2Z)]-4-Benzyl-5-[2-(N,N-diisopropyl-carbamoyloxymethylene)cyclopentyl]-1,3-oxazoldin-2-one (5a) and (5b).

237 mg (0.5mmol) 3a and 71 mg (0.25mmol) Tetraisopropoxytitanium in 5 ml tetrahydrofuran was heated 15 h under reflux. To the reaction mixture was added 10 ml 2N HCl and the aqueous phase was extracted 3 times with 10 ml ether. The organic phase was shaken 2 times with 10 ml. saturated $NaHCO_3$ solution to remove acid and after drying over $MgSO_4$ the solvent was removed in vacuo.

Chromatography of the residue on silica with EtOAc/n-hexane gave 168 mg (84%) 5a.

5a: Oil, $R_f$=0.28 (EtOAc/n-Hexane 1:1).

IR (Film): 3300 (NH), 1755 (Oxazolidin C=0), 1710 $cm^{-1}$ (Carbamoyl C=0).

[ $]^{20}_D$ = +69.8° (c=2, $CH_2Cl_2$).

| $C_{23}H_{32}O_4N_2$ (400.52). | | | |
|---|---|---|---|
| Calc.: | C=68.97<br>H= 8.05 | Found: | C=69.21<br>H= 8.28 |

In the same way as described for 5a, one obtained from 237mg (0,5mmol) 3b 172 mg (86%) 5b.

5a White Solid, mp. 149°C (Hexan), $R_f$=0.20 (EtOAc/n-Hexan 1:1).

IR (KBr):3360(NH),1755 (Oxazolidin C=0), 1685 $cm^{-1}$(Carbamoyl C=0).

[ $]^{20}_D$=-222.0° (c=2, $CH_2Cl_2$).

| $C_{23}H_{32}O_4N_2$ (400.52). | | | |
|---|---|---|---|
| Calc.: | C = 68.97<br>H = 8.05 | Found: | C = 69.22<br>H = 7.87 |

Example 6 of formula IV

[4S,5S,5(1S,2Z)]-and{4S,5R,5(1R,2Z)]-4-Benzyl-3-tert.-butoxycarbonyl-5-[2-(N,N-diisopropylcarbamoyloxymethylene)cyclopentyl]-2,2-dimethyl-1,3-oxazoldine (6a) and (6b)

To 237 mg (0.5 mmol) **3a** in 3ml N,N-Dimethylformamid at 0°C was added 120 ul (1.25 mmol) 2-methoxypropene and one drop of $POCl_3$. The reaction mixture was stirred 15 h at room temperature. Then 10 ml $H_2O$ was added, the aqueous phase extracted 3 times with 10 ml ether and the organic phases dried over $MgSO_4$. The solution was concentrated in vacuo and chromatographed on silica with EtOAc/hexane. One obtained 218mg (85%) 6a.
6a: Oil, $R_f$ = 0.56 (EtOAc/n-Hexan 1:4).
IR (Film): 1700 $cm^{-1}$ (C = 0).
$[\ ]^{20}_D$ = + 164°(c = 2.5,$CH_2Cl_2$).

| $C_{30}H_{46}O_5N_2$ (514.71). | | | |
|---|---|---|---|
| Calc.: | C = 70.01<br>H = 9.01 | Found: | C = 69.90<br>H = 8.94 |

In the same way as described for 3a. one obtained 232 mg 6b. (92%) from 237 mg. 3b.
6b: White Solid, mp 58°C (Hexan), $R_f$ = 0.44 (EtOAc/n-Hexan 1:4).
IR (KBr); 1700 $cm^{-1}$ (C = 0).
$[\ ]^{20}_D$ = -97.6°(c = 2, $CH_2Cl_2$).

| $C_{30}H_{46}O_5N_2$ (514.71) | | | |
|---|---|---|---|
| Calc.: | C = 70.01<br>H = 9.01 | Found: | C = 69.92<br>H = 9.03 |

Example 7 of formula IV

[1R,2S,2(4S,5S)] and [1S,2S,2(4S,5S)]-2-(4-Benzyl-3-tert.-butoxycarbonyl-2,2-dimethyl-1,3-oxazoldine-5-yl)-cyclopentane-methanol (7a) and (7b)

To a solution of 514mg (1mmol) **6a** in 5 ml Tetrahydrofuran at 0°C was added dropwise 190ul (2mmol) borane-dimethysulfide complex over the course of 5 minutes.

After 30 minutes the reaction mixture was allowed to warm to room temperature, then stirred for 15 h. Then 8 ml EtOH was added followed by 1 ml 30% $H_2O_2$ and 2.2 ml 30% NaOH. The solution was heated 1 h at 50°C. To the cooled reaction mixture was then added 20 ml $H_2O$ and 20 ml ether. the phases were separated and the aqueous phase extracted 3 times with 10 ml ether. After drying over $MgSO_4$ the solvent was removed on the rotary evaporator while under observation as a precautionary measure. One obtains 421 mg colorless oil that was used without further purification in subsequent reactions. For characterization by chromatography on silica and obtained 217 mg (56%) of an unseparable mixture of C-1 epimers 7a and

7b.
7a and 7b: 6a: Oil, $R_f = 0.42$ (EtOAc/n-Hexan 1:1).
IR (KBr): 3460 (OH), 1670 $cm^{-1}$ (C=0).

| $C_{23}H_{35}O_4N$ (389.54). | | | |
|---|---|---|---|
| Calc.: | C=70.92<br>H= 9.06 | Found: | C=71.08<br>H= 8.94 |

Example 8 of formula IV

[1R,2R,2(4S,5R)]-and[1S,2R,2(4S,5R)]-2-(4-Benzyl-3-tert.-butoxycarbonyl-2,2-dimethyl-1,3-oxazolidine-5-yl)-cyclopentanemethanol (8a) and (8b)

From 514 mg 6b one obtained in the same way as described under 7 418 mg 8a and 8b as crude product (colorless oil), that without further purification was used in the following reaction. For characterization by chromatography on silica there was obtained 260 mg (67%) of an inseparable mixture of the C-1 epimers 8a and 8b.
8a and 8b: White solid mixture, MP range 90-100° (Hexan), $R_f = 0.55$ (EtOAc/n-Hexan 1:1).
IR (KBr): 3480 (OH), 1715 $cm^{-1}$ (C=0).

| $C_{23}H_{35}O_4N$ (389.54). | | | |
|---|---|---|---|
| Calc.: | C=70.92<br>H= 9.06 | Found: | C=70.89<br>H= 9.00 |

Example 9 of formula IV

[1R,2R,2(4S,5R)]-and[1S,2R,2(4S,5R)]-2-(4-Benzyl-3-tert.-butoxycarbonyl-2,2-dimethyl-1,3-oxazolidine-5-yl)-cyclopentane-carbaldehyde (9a) and (9b).

To 418 mg of mixture of 8a and 8b (crude product from 7) in 10 ml $CH_2Cl_2$ was added 752 mg (2mmol)
pyridiniumdichromate. After 15h stirring at room temperature the mixture was heated for 1 hour at 40°C to complete the reaction. The solvent was removed in vacuo, the residue taken up in 40ml ether and filtered through a 5cm layer of silica gel. The filtrate was washed consecutively with 10ml 2N and 10ml saturated $NaHCO_3$ solution and dried over $MgSO_4$. After removal of the solvent in vacuo there remained 385 mg 9a and 9b as crude product (yellow oil) that was used without purification in the next reaction.
9a and 9b: Oil, $R_f = 0.55$ (EtOAc/n-Hexan 1:3).

Example 10 of formula IV

Epimerization of 9b to 9a.

385 mg 9a and 9b (crude product from 9) was dissolved in 5ml MeOH, 108 mg (2 mmol) sodium-methoxide was added and the solution was stirred 15h at room temperature. Then 10ml 2N HCl and 20ml

ether was added to the reaction mixture. The organic phase was deacidified by shaking with 10ml sat. $NaHCO_3$ solution and dried over $MgSO_4$. Removal of solvent in vacuo gave 355 mg 9a as a yellow oil, that was used without purification in further reactions.
9a: 0:1, $R_f = 0.55$ (EtOAc/n-Hexan 1:3).

Example 11 of formula IV

[1R,2R,2(4S,5R)]-2-(4-Benzyl-3-tert.-butoxycarbonyl-2,2-dimethyl-1,3-oxazolidine-5-yl)-cyclopentanecarboxylic acid (10). (B.S. Bal,W.E. Childers,H.W. Pinnick, Tetrahedron 37(1981) 2091.

To 355 mg 9a (crude product from 10) and 3ml 2-methyl-2-butene in 12 ml MeOH was added dropwise a solution of 630 mg (7mmol) $NaClO_2$ and 600 mg (5mmol) $NaH_2PO_4$ in 7 ml $H_2O$ over a 10 minute period. After 30 minutes stirring at room temperature, the solvent was mostly removed and the residue taken up in 20 ml ether and 10 ml 2N HCl. The aqueous phase was extracted 3 times with 10 ml ether and the combined organic phases dried over $MgSO_4$. The solvent was removed in vacuo and the crude product chromatographed on silica with EtOAc/n-hexane. There was obtained 185mg (46%, based on 6b) 10. 10: White solid, mp 48-50°C, $R_f = 0.29$ (EtOAc/n-Hexan 1:3).
IR (KBr): 1700 $cm^{-1}$ (C=0).
$[\ ]^{20}_D$ = -64.5°(c=2, $CH_2Cl_2$).

| $C_{23}H_{33}O_5N$ (403.52) | | | |
|---|---|---|---|
| Calc.: | C=68.46<br>H= 8.24 | Found: | C=68.38<br>H= 8.35 |

Example 12 of formula IV

[1R,2R,2(4S,5R)]-2-(4-Benzyl-3-tert.-butoxycarbonyl-2,2-dimethyl-1,3-oxazolidine-5-yl)-cyclopentanecarboxylic acid methylester (11). ( D.R. Williams, F.D. Klinger, E.E. Allen,F.W. Lichtenthaler, Tetrahedron Letters(1988) 5087.

To a solution of 355 mg 9a (crude product from 10) in 2 ml $MeOH/H_2O$ (9:1) was added 1.68g (20mmol) $NaHCO_3$. Then 0.2ml (4mmol) $Br_2$ in 1 ml $MeOH/H_2O$(9:1) was added dropwise over the course of 5 minutes at a reaction temperature of 0°C. The reaction mixture was stirred an additional 5 hours at room temperature. After removal of excess $Br_2$ with $Na_2S_2O_3$, 30 ml $H_2O$ and 20 ml ether was added, the phases separated and the aqueous phase extracted 3 times with 15 ml ether. The combined organic phases were dried over $MgSO_4$, the solvent removed in vacuo and the residue chromatographed on silica with EtOAc/hexane. there was obtained 209mg (50% based on 6b) 11.
11: White solid, mp 90°(Hexan), $R_f = 0.55$(EtOAc/n-Hexan 1:3).
IR (KBr): 1730 (COOMe), 1700 $cm^{-1}$ (N-C=0).
$[\ ]^{20}_D$ = -65°(c=1, $CH_2Cl_2$).

| $C_{24}H_{35}O_5N$ (418.56) | | | |
|---|---|---|---|
| Calc. | C=68.87<br>H= 8.43 | Found: | C=69.28<br>H= 8.51 |

Example 13 of formula IV

[1R,4S,4(1S), 5S]-4-(1-tert.-Butoxycarbonylamino-2-phenylethyl)-3-oxabicyclo[3.3.0]-octan-2-one (12)

To a solution of 237mg (0.5mmol) 3a in 2.5ml THF was added dropwise at 0°C 192 ul (2mmol) borane-dimethylsulfide complex over a 5 minute period. After 30 minutes the reaction mixture was allowed to warm to room temperature, then stirred for 15 hours. Then 4ml EtOH, 4ml 30% $H_2O_2$ and 2.2 ml 30% NaOH was added consecutively and the solution heated 1 hour at 50°C. The cooled solution was added to 10 ml $H_2O$ and 10 ml ether, the phases separated and the aqueous phase extracted 3 times with 10 ml ether. After drying over $MgSO_4$, the solvent was removed on the rotary evaporator while observing as a precautionary measure. The crude product was then dissolved in 2ml $CH_2Cl_2$, 376 mg (1mmol) pyridinium dichromate was added and the solution stirred 15 hours at room temperature. The solvent was removed in vacuo, the residue taken up in 40 ml ether and filtered through a 5 cm layer of silica gel. The filtrate was consecutively washed with 10 ml 2N HCl and 10 ml saturated $NaHCO_3$ solution and dried over $MgSO_4$. Removal of solvent in vacuo and chromatography on $SiO_2$ with EtOAc/n-Hexan gave 83 mg (48%) 12.

12: White solid, mp 140°C (Hexan), $R_f$ = 0.6 (EtOAc/n-Hexan 1:1).
IR (KBr): 3400-3340 (NH), 1750 (Lacton C = 0), 1700 $cm^{-1}$ (t.-Butoxycarbonyl (C = 0).
$[\ ]^{20}_D$ = -4.8°(c = 1.5, $CH_2Cl_2$).

| $C_{20}H_{27}O_4N$ (345.44) | | | |
|---|---|---|---|
| Calc.: | C = 69.54<br>H = 7.88 | Found: | C = 69.43<br>H = 8.09 |

Example 14 of formula IV

(S)-2-Benzyloxycarbonylamino-3-phenylpropanal (13) was synthesized from (S)-Benzyloxycarbonylamino-N-methoxy-N-methyl-3-phenylpropanamide according to the literature.

Example 15 of formula IV

[1S,1(1S.2Z),2S]-and[1R,1(1R,2Z),2S]-Benzyloxycarbonyamino-1-[2-(N,N-diisopropylcarbamoyloxymethylene)cyclopentyl]-3-phenyl-1-propanol (14a) and (14b)

From 9.020g (40mmol) 2 and 5.660g (20mmol) 13 one obtained in the same way as described under 4 2.400 g(23%) 14a and 5.030g (49.5%) 14b which were separated by chromatography on $SiO_2$ with EtOAc/hexane.

14a: Oil, $R_f$ = 0.4(EtOAc/n-Hexan 1:3).
IR(KBr) 3600-3300 (NH, OH), 1710 $cm^{-1}$ (N-C = 0).
$[\ ]^{20}_D$ = 63.2°(c = 2, $CH_2Cl_2$).

| $C_{30}H_{40}N_2O_5$ (508.66). | | | |
|---|---|---|---|
| Calc.: | C = 70.84<br>H = 7.96 | Found: | C = 70.87<br>H = 7.84 |

14b: Oil, $R_f$ = 0.25(EtOAc/n-Hexan 1:3).
IR(Film) 3600-3300 (NH, OH), 1710 $cm^{-1}$ (N-C = 0).
$[\ ]^{20}_D$ = -96.5°(c = 2, $CH_2Cl_2$).

| $C_{30}H_{40}N_2O_5$ (508.66). | | | |
|---|---|---|---|
| Calc.: | C = 70.87<br>H = 7.96 | Found: | C = 70.86<br>H = 8.03 |

Example 16 of formula IV

Conversion of 15a into 5b

In the same was as described under 5 one obtained from 127 mg (0.25 mmole) 14a 95 mg (95%) 5a

Example 17 of formula IV

Conversion of 14b into 5b

In the same way 127mg (0.25mmole) 14b were converted into 59mg (59%) 5b.

Example 18 for formula IV

[1S,2S,2(1S),4S,5R]-and[1S,2S,2(1S),4R,5R]-2-(1-Benzyloxycarbonyl amino-2-phenylethyl)-4-methoxy-3-oxabicyclo[3.3.0]octane (15a) and (15b).

To 508mg (1mmol) 14a and 32 mg (0.1 mmol) mercury (II) acetate in 5ml MeOH was added dropwise at 0°C 128ul (2 mmol) methanesulfonic acid over a 5 minute period. At the end of the addition the reaction mixture was stirred 15 hours at room temperature. The reaction mixture was neutralized with $NaHCO_3$, the solvent removed in vacuo and the residue taken up in 30 ml ether. The solution was washed with 10 ml $H_2O$, dried over $MgSO_4$ and the solvent removed in vacuo to leave 408mg of colorless oil. This was used without purification in the next reaction. Alternatively, chromatography of the crude product on $SiO_2$ with StOAc/hexane gave 185 mg (46.8%) 15a and 60 mg (15.2%) 15b.
15a: Oil, $R_f$ = 0.77 (EtOAc/n-Hexan 1:1).
IR(Film): 3450-3250 (NH), 1715 $cm^{-1}$ (C = 0).
$[\ ]^{20}_{D}$ = + 59.93(c = 1.5,$CH_2Cl_2$).

$C_{24}H_{29}NO_4$ (395.48).

| Calc.: | C=72.89 | Found: | C=72.79 |
|---|---|---|---|
| | H= 7.39 | | H= 7.41 |

15b: Oil, $R_f$ = 0.72(EtOAc/n-Hexan 1:1).
IR(Film):3400-3250 (NH), 1715 $cm^{-1}$ (C = 0).
$[\ ]^{20}_{D}$ = -55.84

| $C_{24}H_{29}NO_4$ (395.48) | | | |
|---|---|---|---|
| Calc.: | C = 72.89<br>H = 7.39 | Found: | C = 72.79<br>H = 7.35 |

Example 19 of formula IV

[1R,4S,4(1S),4S]-4-(1-Benzyloxycarbonylamino-2-phenylethyl)-3-oxabicyclo[3,3,0)-octane-2-one (16).

To 408 mg 15a and 15b (crude product from 18) and 237 mg (1.1 mmol) 80% m-chloroperbenzoic acid in 5 ml $CH_2Cl_2$ was added dropwise at 0°C 280 ul (1 mmol) borontrifluoride-diethylether complex and the reaction mixture was stirred 15 hours at room temperature. The excess peracid was then reduced with 50 ul dimethylsulfide and after 30 minutes the reaction mixture was added to 30 ml ether and 10 ml $H_2O$. The organic phase was deacidified with 10ml saturated $NaHCO_3$ solution and dried over $MgSO_4$. Removal of the solvent in vacuo and chromatogrpahy of the residue on $SiO_2$ with EtOAc/hexane gave 201 mg (53% based on 14a) 16.
16: White solid, mp:129°C (Ether), $R_f$ = 0.64 (EtOAc/n-Hexan 1:1).
IR(KBr): 3320 (NH,) 1770 (Lacton C = 0), 1690 $cm^{-1}$ (N-C = 0).
[ $]^{20}_{D}$ = -16°(c = 1,$CH_2Cl_2$).

| $C_{23}H_{25}NO_4$ (379.44). | | | |
|---|---|---|---|
| Calc.: | C = 72.81<br>H = 6.64 | Found: | C = 73.02<br>H = 6.72 |

The compounds of the present invention can exist as optical isomers and both racemic and diastereomeric mixtures of these isomers which may exist for certain compounds, as well as the individual optical isomers are all within the scope of the present invention. While the racemic mixtures can be separated into their individual isomers through well-known techniques such as, for example, the separation of diastereomeric salts formed with optically active adjuncts, e.g., acids or bases followed by conversation back to the optically active substrates; in most instances, for the compounds of the present invention,
the preferred optical isomer can by synthesized by means of sterospecific reactions, beginning with the appropriate steroisomer of the desired starting material.

The present invention also pertains to pharmaceutically acceptable non-toxic salts of these compounds, containing, for example $Na^+$, $Li^+$, $K^+$, $Ca^{++}$ and $Mg^{++}$. Such salts may include those derived by combination of appropriate cations such as alkali and alkaline earth metal ions or ammonium and quaternary amino ions with a compound of this invention. Examples of metal salts which can be prepared in this way are salts containing $Li^+$, $Na^+$, and $K^+$. A less soluble metal salt can be precipitated from the solution of a more soluble salt by addition of the suitable metal compound. In addition, salts may be formed from acid addition of certain organic and inorganic acids, e.g. HCl, HBr, $H_2SO_4$ or organic sulfonic acids. Finally, it is to be understood that compounds of the present invention in their un-ionized, as well as zwitterionic form, and/or in the form of solvates are also considered part of the present invention.

The present invention also encompasses pharmaceutically acceptable esters of the inventive compounds containing OH groups. Such esters are the esterification products of an OH group of an inventive compound with an acid such as, for example, a carboxylic acid, a sulphonic acid or a phosphoric acid. Non-limiting examples of specific acids to make esters according to the invention include $C_1$-$C_{10}$-alkylcarboxylic acid, unsubstituted or substituted benzoic acid or $C_1$-$C_6$-alkylsulphonic acid.

The active compounds of the present invention may be used as follows:

(a) for the treatment or propylaxis of diseases caused by HIV (virus of human immune deficiency) e.g., HIV I, HIV II and HIV III and the associated AIDS stages such as ARC (AIDS related complex) and LAS (lymph adenopathy syndrome) and the immune weakness and encephalopathy caused by this virus.

(b) for the treatment or prophylaxis of an HTLV I or HTLV II infection.

(c) for the treatment or propylaxis of the AIDS carrier state (AIDS transmitter state); and

(d) for the treatment or prophylaxis of diseases caused by hepatitis B virus.

The present invention includes pharmaceutical formulations which, in addition to non-toxic, inert pharmaceutically suitable excipients, contain one or more compounds of the invention.

The present invention also includes pharmaceutical formulations in dosage units. This means that the formulations are present in the form of individual parts, for example, tablets, dragees, capsules, caplets, pills, suppositories and ampoules, the active compound content of which corresponds to a fraction or a multiple of an individual dose. The dosage units can contain, for example, 1 ,2, 3 or 4 individual doses or 1/2, 1/3 or 1/4 of an individual dose. An individual dose preferably contains the amount of active compound which is given in one administration and which usually corresponds to a whole, one half, one third or one quarter of a daily dose.

By non-toxic, inert pharmaceutically suitable excipients there are to be understood solid, semi-solid or liquid diluents, fillers and formulation auxiliaries of all types.

Preferred pharmaceutical formulations which may be mentioned are tablets, dragees, capsules, caplets, pills, granules, suppositories, solutions, suspensions and emulsions, pastes, ointments, gels, creams, lotions, dusting powders and sprays. Tablets, dragees, capsules, caplets, pills and granules can contain the active compound or compounds in addition to the customary excipients, such as (a) fillers and extenders, for example, starches, lactose, sucrose, glucose, mannitol and silicic acid, (b) binders, for example, carboxymethylcellulose, alginates, gelatin and polyvinylpyrrolidone, (c) humectants, for example, glycerol, (d) disintegrating agents, for example, agar-agar, calcium carbonate and sodium carbonate, (e) solution retarders, for example, paraffin and (f) absorption accelerators, for example, quaternary ammonium compounds, (g) wetting agents, for example, cetyl alcohol and glycerol monostearate, (h) absorbents, for example, kaolin and bentonite and (i) lubricants, for example, talc, calcium stearate, magnesium stearate and solid polyethylene glycols, or mixtures of the substances listed under (a) to (i) directly hereinabove.

The tablets, dragees, capsules, caplets, pills and granules can be provided with the customary coatings and shells, optionally containing opacifying agents and can also be of such composition that they release the active compound or compounds only or preferentially in a certain part of the intestinal tract, optionally in a delayed manner. Examples of embedding compositions which can be used are polymeric substances and waxes.

The active compound or compounds can also be present in microencapsulated form, if appropriate with one or more of the abovementioned excipients.

Suppositories can contain, in addition to the active compound or compounds, the customary watersoluble or water-insoluble excipients, for example, polyethylene glyclos, fats, for example, cacao fat and higher esters (for example, $C_{14}$-alcohol with $C_{16}$-fatty acid), or mixtures of these substances.

Ointments, pastes, creams and gels can contain, in addition to the active compound or compounds, the customary excipients, for example, animal and vegetable fats, waxes, paraffins, starch, tragacanth, cellulose derivatives, polyethylene glycols, silicones, bentonites, silicic acid, talc and zinc oxide, or mixtures of these substances.

Dusting powders and sprays can contain, in addition to the active compound or compounds, the customary excipients, for example, lactose, talc, silicic acid, aluminum hydroxide, calcium silicate and polyamide powder, or mixtures of these substances. Sprays can additionally contain customary propellants, for example, chlorofluorohydrocarbons.

Solutions and emulsions can contain, in addition to the active compound, customary excipients, such as solvents, solubilizing agents and emulsifiers, for example, water, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, castor oil and sesame oil, glycerol, glycerol formal, tetrahydrofurfuryl alcohol, polyethylene glycols and fatty acid esters or sorbitan, or mixtures of these substances.

For parenteral administration, the solutions and emulsions can also be in a sterile form which is isotonic with blood.

Suspensions can contain, in addition to the active compond, customary excipients, such as liquid diluents, for example, water, ethyl alcohol or propylene glycol and suspending agents, for example, ethoxylated isostearyl alcohol, polyoxyethylene sorbitol and sorbitan esters, microcrystalline cellulose, aluminum metahydroxide, bentonite, agar-agar and tragacanth, or mixtures of these substances.

The formulation forms mentioned can also contain coloring agents, preservatives and smell- and taste-improvement additives, for example, peppermint oil and eucalyptus oil and sweeteners, for example, saccharine or aspartame.

The active compounds of the invention should preferably be present in the abovementioned pharmaceutical formulations in a concentration of about 0.1 to 99.5, preferably about 0.5 to 95% by weight of the total mixture.

The abovementioned pharmaceutical formulations can also contain other pharmaceutical active compounds in addition to the compounds of the invention.

The aforementioned pharmaceutical formulations are prepared in the customary manner by known methods, for example, by mixing the active compound or compounds with the excipient or excipients.

The formulations mentioned can be used on humans and animals either orally, rectally, bucally, parenterally (intravenously, intramuscularly or subcutaneously), intracisternally, intravaginally, intraperitoneally or locally (dusting powder, ointment or drops) and for the therapy of infections in hollow spaces or body cavities. Suitable formulations are injection solutions, solutions and suspensions for oral therapy, gels, pour-on formulations, emulsions, ointments or drops. Ophthalmological and dematological formulations, silver salts and other salts, ear drops, eye ointments, powders or solutions can be used for local therapy. It is furthermore possible to use gels, powders, dusting powders, tablets, sustained release tablets, premixes, concentrates, granules, pellets, capsules, caplets. aerosols, sprays and inhalates on humans and animals. The compounds according to the invention can furthermore be incorporated into other carrier materials, such as, for example, plastics (e.g, chains of plastic for local therapy). collagen or bone cement.

In general, it is advantageous both in human and in veterinary medicine to administer the active compound or compounds of the invention in total amounts of about 0.5 to 500, preferably 5 to 100 mg/kg of body weight every 24 hours, if appropriate in the form of several individual doses, to achieve the desired results. An individual dose preferably contains the active compound or compounds in amounts of about 1 to about 80, in particular 3 to 30 mg/kg of body weight. However, it may be necessary to deviate from the dosages mentioned and in particular to do so as a function of the nature and body weight of the subject to be treated, the nature and severity of the disease, the nature of the formulation and of the administration of the medicament and the period of interval within which administration takes place.

Thus in some cases it may be sufficient to manage with less that the abovementioned amount of active compound, while in other cases, the abovementioned amount of active compounds can easily be determined by any expert on the basis of his expert knowledge.

The present invention also is directed to a Reverse Phase High Performance Liquid Chromatography (RP-HPLC) in vitro enzyme assay based on the proteolytic activity catalyzed by the HIV-protease. Thin layer chromatography could be used in place of RP-HPLC, but RP-HPLC results in more precise quantitation. The enzyme is incubated with a N-Dansyl-peptide which can be s cleaved by the protease in vitro. The amino acid sequences present in such N-dansyl-peptide substrates include, but are not limited to those present in the proteolytic cleavage site of the HIV gag and pol polyproteins. The incubation of the protein with such peptides is performed under conditions in which the protease is catalytically active. After the incubation period, an aliquot of the reaction mixture is removed and analysed by analytical RP-HPLC. Any uncoverted peptide substrate is resolved from peptide product(s) and then detected by an inline fluorescence monitor. The amount of product formed over a known time interval may then be used to calculate the activity of the enzyme under the specific assay conditions. The amount of fluorescence detected in the eluted peak from HPLC can be related to the amount of peptide in the peak by prior HPLC analysis of the fluorescence obtained from HPLC analysis of standardized samples. The amount of peptide in the pure, standardized samples is determined accurately by amino acid analysis.

In the examples cited herein, an assay based on the cleavage of N-dansyl SQNYPIV to yield N-dansyl-SQNY is described. The following peptides can also be employed in the aforementioned assay:

| Substrate | Product |
|---|---|
| N-dansyl-ARVLAEA | N-dansyl-ARVL |
| N-dansyl-ATIMMQR | N-dansyl-ATIM |
| N-dansyl-PGNFLQS | N-dansyl-PGNF |
| N-dansyl-SFNFPQI | N-dansyl-SFNF |
| N-dansyl-TLNFPIS | N-dansyl-TLNF |
| N-dansyl-RKILFLD | N-dansyl-RKIL. |

The rapid analysis time allows for analysis of large numbers of samples, such as encountered in the monitoring of column chromatography during enzyme purification. The isochratic nature of the HPLC separation allows for a comparatively simple and inexpensive solvent delivery system. Additionally, the

selectivity in detection enables easy development of competition assays featuring other nonfluorescent ligands, such as authentic gag polyproteins. It provides a direct method of determining inhibitory effect of compounds.

A p24 capture assay (see Example 50 hereinbelow) provides an "in vivo" method for determining HIV-protease activity. The inhibitor is added to cells infected with HIV, andOne the effect on syntheses of viral protein is the examined. Inhibition of propagation of HIV-protease in cells is measured by the absence or presence of p24, which is generated by HIV protease.

The invention will now be described with reference to the following non-limiting examples.

Example 1: 2-[(1,1-Dimethylethoxy carbonyl]amino-1-phenyl-but-3-ene

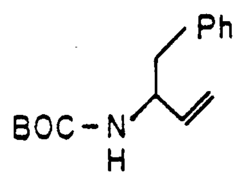

A suspension of 87.5 g (0.21 mol) of methyltriphenyl phosphonium bromide + sodium amide (FLUKA "instant ylide") in 500 ml of dry THF was stirred at room temperature for 20 minutes. Then a solution of 52.6 g (0.21 mol) of Boc-L-phenylalaninal in 250 ml of dry THF was added under ice cooling, and the mixture was stirred at room temperature overnight. The suspension was poured onto crushed ice and extracted four times with brine, dried ($Na_2SO_4$), and concentrated. The residue was chromatographed on silica gel with toluene/petrol either (gradient 1:2 → 5:1) to give 26.6 g (51.3%) of the product

$R_F$ (20:1 dichloromethane/methanol): 0.68

Example 2: [1-[(1.1-Dimethylethoxy)carbonyl]amino-2-phenylethyl]oxiran

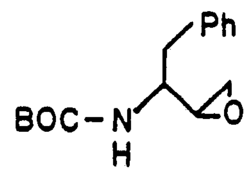

A solution of 42.6 g (0.21 mol) of 85% m-chloroperbenzoic acid in a mixture of 200 ml of chloroform and 50 ml of dichloromethane was added at 0°C to a solution of 26.6g (0.1 mol) of the compound of Example 1 in 250 ml of chloroform. After stirring at room temperature overnight, the solution was washed twice with 5% aqueous $Na_2S_2O_5$ and three times with 5% aqueous $Na_2CO_3$, dried ($Na_2SO_4$), and concentrated. The residue was chromatographed on silica gel with 3:1 n-hexane/ethyl acetate to give 18.3 g (69.5%) of the product, mainly as the 2R, 3S diastereomer ($^1H$ NMR).

FAB-MS:m/z 264 (M+H)

$R_F$ (3:1 n-hexane/ethyl acetate): 0.34

Example 3: 2-[1,1-Dimethylethoxy)carbonyl]amino-1-cyclohexyl-but-3-ene

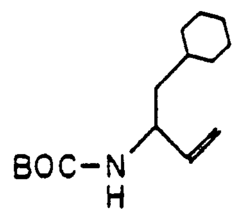

A suspension of 82.9g (0.2 mol) of methyltriphenyl phosphonium bromide + sodium amide (FLUKA "instant ylide") in 500 ml of dry THF was stirred at room temperature for 20 minutes. Then a solution of 46.8 g (0.2 mol) of Boc-L-cyclohexylalaninal in 250 ml of dry THF was added under ice cooling, and the mixture was stirred at room temperature overnight. The suspension was poured onto crushed ice and extracted three times with n-hexane. The combined extracts were washed twice with brine, dried ($Na_2SO_4$), and concentrated. The residue was chromatographed on silica gel with toluene/petrol ether (gradient 1:1→10:1) to give 24.4 g (50.7%) of the product.
DCI-MS:m/z 254 (M + H)
$F_F$ (10:1 toluene/ethyl acetate):0.37

Example 4: [1-[1,1-Dimethylethoxy)carbonyl]amino-2-cyclohexyl-ethyl]oxiran

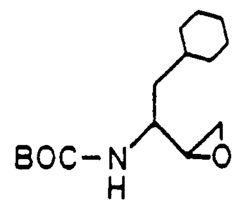

A solution of 24.2 g (0.12 mol) of 85% m-chloroperbenzoic acid in a mixture of 200 ml of dichloromethane and 50 ml of chloroform was added at 0° C to a solution of 14.4 g (0.06 mol) of the compound of Example 3 in 200 ml of dichloromethane. After stirring at room temperature overnight, the solution was washed twice with 5% aqueous $Na_2S_2O_5$ and three times with 5% aqueous $Na_2CO_3$, dried ($Na_2So_4$), and concentrated. The residue was chromatographed on silica gel with toluene/ethyl acetate (gradient 50:1→10:1) to give 12.7 g(78.6%) of the product, mainly as the 2R, 3S diastereomer ($^1H$ NMR). DCI-MS m/z 270 (M + H)
$R_F$ (10:1 toluene/ethyl acetate): 0.28

Example 5: N2-[(1,1-Dimethylethoxy)carbonyl]-N-[2-(2-pyridinyl)ethyl]-L-prolineamide

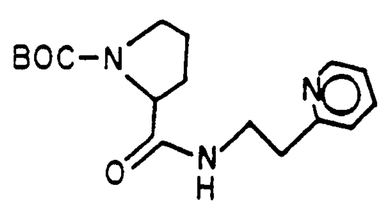

To a stirred solution of 15.06 g (70 mmol) of Boc-L-proline in 100 ml of dichloromethane at 0° C were added 9.2 ml (77 mmol) of 2-(2-aminoethyl)-pyridine, 13.4 grams (87.5 mmol) of 1-hydroxybenzotriazole and 16.61 g (80.5 mmol) of dicyclohexylcarbodiimide. The mixture was stirred at room temperature overnight, then filtered, and the filtrate concentrated. The residue was taken up in ethyl acetate, washed twice with saturated aqueous $NaHCO_3$ and with brine, dried ($Na_2SO_4$), and concentrated. The residue was chromatographed on silica gel with 10:1 dichloromethane/methanol to give 20.6 g (92%) of the product.

MS: m/z 319 ($M^+$)
$R_F$ (10:1 dichloromethane/methanol): 0.50

Example 6: N-[2-(2-pyridinylethyl)]-L-prolineamide

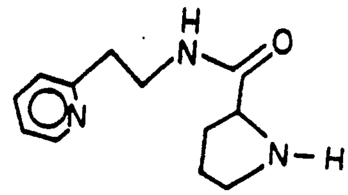

A solution of 20.6 g (65 mmol) of the compound of Example 5 in 150 ml of a 4 N solution of gaseous HCl in dioxane, containing 30 ml of dry methanol, was stirred at 0°C for 8 hours. The solution was concentrated, and the oily residue dried in vacuo. It was then dissolved in 10% aqueous $Na_2CO_3$ (pH 10), the solution was concentrated, and the residue vigorously triturated with warm dichloromethane. The organic phase was dried ($Na_2SO_4$) and concentrated to give 12.4 g (87%) of the product.
$R_F$ (9:1:0.1 dichloromethane/methanol/concentrated aqueous $NH_3$): 0.34

Example 7: N2-[3-[1,1-Dimethylethoxy)carbonyl]amino-2-hydroxy-4-phenyl-butyl]-N-[2-(2-pyridinyl)ethyl)-L-prolineamide

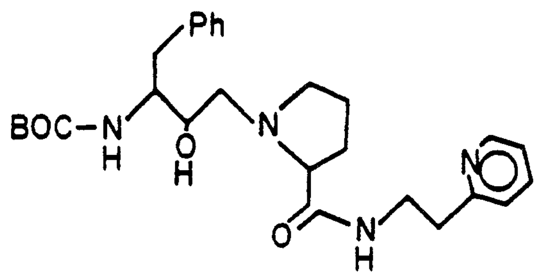

A solution of 4.13 g (15.7 mmol) of the compound of Example 2 and 3.79 g (17.3 mmol) of the compound of Example 6 in 50 ml of isopropanol was stirred at 70°C for 7 hours. The solution was then concentrated, and the residue chromatographed on silica gel with 12:1:0.1 dichloromethane/methanol/concentrated aqueous $NH_3$ to give 4.82 g (63.6%) of the product.
FAB-MS: m/z 483 (M+H)
$R_f$ (9:1:0.1 dichloromethane/methanol/concentrated aqueous $NH_3$): 0.64

Example 8: N2-[3-[1,1-Dimethylethoxy)carbonyl]amono-2-hydroxy-4-cyclohexyl-butyl]-N-2-(2-pyridinyl)-ethyl]-L-prolineamide

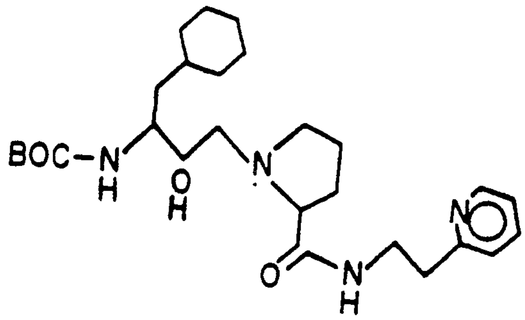

A solution of 4.16 g (15.4 mmol) of the compound of Example 4 and 3.73 g (17 mmol) of the compound of Example 6 in 50 ml of isopropanol was stirred at 70 °C for 7 hours. The solution was then concentrated, and the residue chromatographed on silica gel with 12:1:0.1 dichloromethane/methanol/concentrated aqueous $NH_3$ to give 6.17 g (81.9%) of the product.
FAB-MS: m/z 489 (M + H)
$R_F$ (9:1:0.1 dichloromethane/methanol concentrated aqueous $NH_3$): 0.65

Example 9: N2-[3-amino-2-hydroxy-4-phenyl-butyl]-N-[2-pyridinyl)-L-prolineamide hydrochloric salt (1:3)

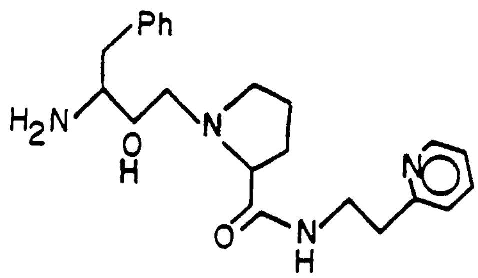

A solution of 4.32 g (8.95 mmol) of the compound of Example 7 in 50 ml of a 4 N solution of gaseous HCl in dioxane, containing 3 ml of dry methanol, was stirred at 0 °C for 2 hours. The solution was then concentrated, the residue repeatedly triturated with dry ether and again concentrated, and finally dried *in vacuo* to give 4.5 g (100%) of the product.
$R_F$ (9:1:0.1 dichloromethane/methanol/concentrated aqueous $NH_3$): 0.29

Example 10: N2-[3-amino-2-hydroxy-4-cyclohexyl-butyl)-N-[2-(2-pyridinyl)ethyl]-L-prolineamide, hydrochloric acid salt (1:3)

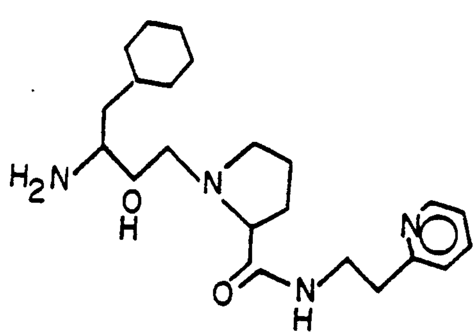

A solution of 5.67 g (11.6 mmol) of the compound of Example 8 in 60 ml of a 4 N solution of gaseous

HCl in dioxane, containing 1 ml of dry methanol, was stirred at 0° C for 2 hours. The solution was then concentrated, the residue repeatedly triturated with dry ether and again concentrated, and finally dried in vacuo to give 5.9 g (100%) of the product.
$R_F$ (9:1:0.1 dichloromethane/methanol/concentrated aqueous $NH_3$): 0.26

Example 11: N2-[N2-(1,1-Dimethylethoxy)carbonyl]-L-asparagyl]amino-2-hydroxy-4-phenyl-butyl]-N-[2-(2-pyridinyl) ethyl]-L-prolineamide

To a stirred solution of 4.5 g (9 mmol) of the compound of Example 9 in 30 ml of DMF at 0° C were added 4.1 ml (32.17 mmol) of N-ethylmorpholine, 2.35 g (10.1 mmol) of Boc-L-asparagine, 1.76 g (11.49 mmol) of 1-hydroxybenzotriazole, and 2.27 g (11 mmol) of dicyclohexylcarbodiimide. The mixture was stirred at room temperature overnight, then filtered, and the filtrate concentrated. The residue was taken up in ethyl acetate, again filtered, and the filtrate washed twice with saturated aqueous $NaHCO_3$ and with brine. The organic phase was dried ($Na_2SO_4$) and concentrated. The residue was dissolved in 9:1:0.1 dichlormethane/methanol concentrated aqueous $NH_3$, and the product precipitated by addition of ether. After filtrate, it was washed with ether and dried in vacuo to give 2.27 g (41.4%).
FAB-MS: m/z 597 (M+H)
$R_F$ (10:1 dichloromethane/methanol): 0.34

Example 12: N2[3-[N2[1,1-Dimethylethoxy)carbonyl)-L-asparagyl]amino-2-hydroxy-4-cyclohexyl-butyl]-N-[2-(2-pyridinyl)ethyl]-L-prolineamide

To a stirred solution of 5.69 g (11 mmol) of the compound of Example 10 in 30 ml of DMF at 0° C were added 5.1 ml (40 mmol) of N-ethylmorpholine, 2.92 g (12.5 mmol) of Boc-L-asparagine, 2.18 g (14.27 mmol) of 1-hydroxybenzotriazole, and 2.83 g (13.7 mmol) of dicyclohexylcarbodiimide. The mixture was stirred at room temperature overnight, then filtered, and the filtrate concentrated. The residue was taken up in dichloromethane, washed twice with saturated aqueous $NaHCO_3$ and with brine, dried ($Na_2SO_4$), and concentrated. The residue was chromatographed on silica gel with dichloromethane/methanol/concentrated aqueous $NH_3$ (gradient 15:1:0.1→9:1:0.1) to give 2.01 g (29.2%) of the product.
FAB-MS: m/z 603 (M+H)
$R_F$ (9:1:0.1 dichloromethane/methanol/concentrated aqueous $NH_3$): 0.43

Example 13: N2-[3-[-L-asparagyl]amino-2-hydroxy-4-phenylbutyl]-N-[2-(2-pyridinyl)ethyl]-L-prolineamide hydrochloric acid salt (1:3)

A solution of 1.95 g (3.27 mmol) of the compound of Example 11 in 35 ml of a 4 N solution of gaseous HCl in dioxane, containing 10 ml of dry methanol was stirred at 0° C for 1 hour and at room temperature for 1 hour. The solution was then cencentrated, the residue repeatedly triturated with dry ether and again concentrated, and finally dried in vacuo to give 1.93 g (97.5%) of the product.
$R_F$ (9:1:0.1 dichloromethane/methanol/concentrated aqueous $NH_3$): 0.15

Example 14: N2-[3-[L-asparagyl]amino-2-hydroxy-4-cyclohexyl-butyl]-N-[2-(-pyridinyl)ethyl]-L-prolineamide hydrochloric acid salt (1:3)

A solution of 1.71 g (2.83 mmol) of the compound of Example 12 in 30 ml of a 4 N solution of gaseous HCl in dioxane, containing 3 ml of dry methanol, was stirred at 0° C for 1 hour and at room temperature for 1 hour. The solution was then concentrated, the residue repeatedly triturated with dry ether and again concentrated, and finally dried in vacuo to give 1.75 g (100%) of the product.
$R_F$ (9:1:0.1 dichloromethane/methanol/concentrated aqueous $NH_3$): 0.17

Example 15: N2-[3-[N2-[N-[(1,1-Dimethylethoxy)carbonyl]-L- phenylalanyl]-L-asparagyl]amino-2-hydroxy-4-cyclohexylbutyl]-N-[2-(2-pyridinyl)ethyl]-L-prolineamide

To a solution of 925 mg (3.49 mmol) of Boc-L-phenylalanine in 15 ml of dichloromethane at 0 °C were added 588 mg (3.84 mmol) of 1-hydroxybenzotriazole and 756 mg (3.67 mmol) of dicyclohexylcarbodiimide. After stirring at 0 °C for 2 hours, a solution of 1.75 g (2.85 mmol) of the compound of Example 14 and 1.3 ml (10.1 mmol) of N-ethylmorpholine in 20 ml of DMF was added, and the mixture was stirred at room temperature overnight. The mixture was filtered and the residue repeatedly triturated with 9:1:0.1 dichloromethane/methanol/concentrated aqueous $NH_3$. The combined filtrates were concentrated, the residue taken up in dichloromethane (plus a few ml of methanol), and washed twice with saturated aqueous $NaHCO_3$ and with brine.

Example 15a:

N2-[3-[N2-[N-[(1,1-Dimethylethoxy)carbonyl]-L-phenylalanyl]-L-histidyl]amino-2-hydroxy-4-cyclohexyl-butyl]-N-[2-(2-pyridinyl) ethyl]-L-prolineamide.

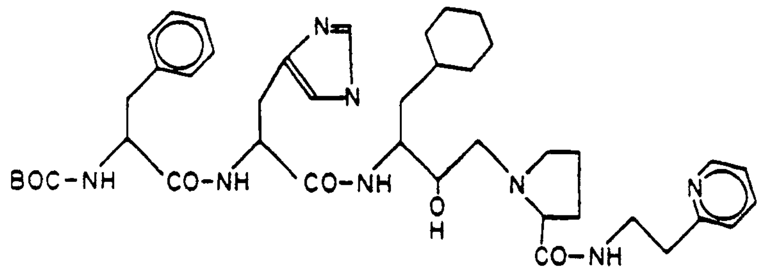

This compound was prepared by standard procedures similar in principle to the sequential steps involving synthesis of the compound in Example 15 from the compound of Example 10.

Example 16: N2-[3-[N2-[N-[(1,1 Dimethylethoxy)carbonyl]-L-phenylalanyl]-L-asparagyl]amino-2-hydroxy-4-phenylbutyl]-N-[2-(2-pyridinyl)ethyl]-L-prolineamide acetic acid salt (1:2)

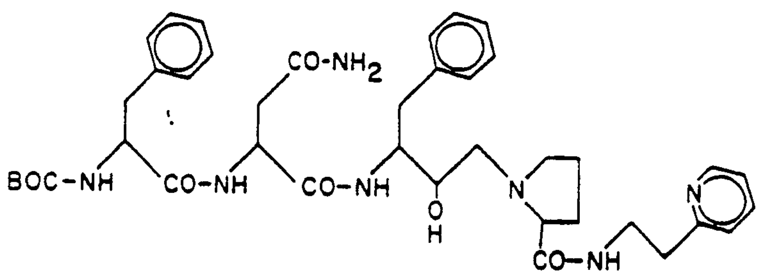

To a solution of 929 mg (3.5 mmol) of Boc-L-phenylalanine in 15 ml of dichloromethane at 0 °C were added 590 mg (3.85 mmol) of 1-hydroxybenzotriazole and 758 mg (3.68 mmol) of dicyclohexylcarbodiimide. After stirring at 0 °C for 2 hours, a solution of 1.93 g (3.18 mmol) of the compound of Example 13 and 1.3 ml (10.1 mmol) of N-ethylmorpholine in 30 ml of KMF was added, and the mixture was stirred at room temperature overnight. The mixture was filtered, the filtrate concentrated, the residue taken up in dichloromethane and washed twice with saturated aqueous $NaHCO_3$ and with brine. The organic phase was dried ($Na_2SO_4$) and concentrated. The residue was combined with the first residue from the dichloromethane/DMF - reaction mixture (containing dicyclohexyl urea as well as some product) and was vigorously stirred with ether. The suspension was filtered, and the residue again vigorously stirred with 10% acetic acid. The mixture was filtered, and the filtrate lyophilized to give 1.80 g (65.5%) of the product.

FAB-MS: m/z 744 (M+H)

$R_F$ (9:1:0.1 dichlormethane/methanol/concentrated aqueous $NH_3$): 0.45

Example 17: N-[N-[(1,1-Dimethylethoxy)carbonyl]-L-phenylalanyl]-L-prolinemethylester

A solution of 26.5 g (0.1 mol) of Boc-L-phenylalanine and 16.5 g (0.1 mol) prolinemethylester in 150 ml of dry dimethylforamide was stirred at 0°C and 60 ml of N-ethylmorpholine was added. After stirring for 6 hours all the solvents were evaporated and the residue was dissolved in 100 ml ethyl acetate and washed three times with saturated sodium bicarbonate, water and a 1N solution of citric acid, dried over $MgSO_4$ and concentrated. The residue was chromatographed on silica gel (dichloromethane/methanol 10:1) to yield 35.0 g (93%) of the title compound. Rf: 0.9 (Dichloromethane/methanol 10:1.5)

Example 18:

N-[2-[(1,1-Dimethylethoxy)carbonyl]amino-3-phenylthiopropionyl]-L-prolinemethylester

26.6 g (79 mmol) of N-[N-(1,1-Dimethylethoxy) carbonyl]-L-phenylalanyl]-L-prolinemethylester were dissolved in 100 ml toluene and added to a solution of 15.9 g (38 mmol)2,4-bis(4-methoxyphenyl)-1,3-dithio-2,4-diphosphetane-2,4disulfide (Lawesson's reagent) in 600 ml toluene at 80°C. After stirring for 8 hours another 1.6 g (4 mmol) of the Lawesson's reagent were added. After stirring for another 12 hours the reaction mixture was cooled down and extracted three times with $H_2O$, dried over $MgSO_4$ and concentrated. The residue was chromatographed on silica gel (dichloromethane/petroleumether/acetone 10:10:0.3 to yield 23.5 g (76.3%) of the title compound. Rf: 0.8 (dichloromethane/acetone 10:1)

Example 19: N-[2-[(1,1-Dimethylethoxy)carbonyl]amino-3-phenyl-propyl]-L-prolinemethylester hydrochloric salt (1:1)

56 g of an alloy nickel/aluminum 50/50 were suspended in 500 ml $H_2O$ under argon and 80 g of sodium hydroxide were slowly added. The mixture was heated to 80°C for 30 minutes, cooled down, decanted and washed three times with water. The residue was washed three times with methanol and finally 25.5 g (70 mmol) of N-[2-[(1,1-Dimethylethoxy)carbonyl]amino-3-phenylthiopropionyl]-L-prolinemethylester in 300 ml of methanol were added. The resulting mixture was stirred at room temperature for three hours, filtrated and concentrated. 70 ml hydrochloric acid were added and the solution was concentrated to yield 14.9 g(53%) of the title compound. RF: 0.23 (dichloromethane/acetone 20:1)

Example 20: N-[2-[(1,1-Dimethylethoxy)carbonyl]amino-3-phenyl-propyl]-L-proline

14.8 g (37 mmol) N-[2-[(1,1-Dimethylethoxy) carbonyl]amino-3-phenyl-propyl]-L-prolinemethylester were dissolved in 100 ml methanol and 50 ml tetradydrofuran and 3.1 g (74 mmol) lithium hydroxide-monohydrate in 40 ml $H_2O$ were added. The resulting mixture was stirred for 4 hours at 0°C. After adding 1.55 g (37 mmol) lithium hydroxide in 20 ml $H_2O$ the solution was maintained at 0°C for 16 hours, extracted with 150 ml hexane and acidified to pH of 2.5. The resulting crystals were collected and dried to yield 11.3 g (88%) of the title compound. Rf: 0.67 (chloroform/methanol/$H_2O$/acetic acid 65:25:4:3)

Example 21: N-[N-[2-[(1,1-Dimethylethoxy)carbonyl]amino-3-phenyl-propyl]-L-prolyl]-L-isoleucinbenzylester citric acid salt (1:1)

10.1 g (29 mmol) N-2[-[(1,1-Dimethylethoxy)-carbonyl]amino-3-phenyl-propyl]-L-proline and 13.1 g (33 mmol) isoleucinbenzylester, toluenesulfonic acid salt (1:1) were dissolved in 65 ml dimethylformamide and maintained at 0°C. 15.8 g (137 mmol) N-Ethylmorpholine and 25.5 g (40 mmol) of 1-propanephosphonic acid cyclic anhydride (50% solution in dichloromethane) were added at this temperature and the resulting mixture was stirred at 0°C for five hours. The reaction mixture was concentrated in vacuo and the residue dissolved in ethylacetate and water, washed three times with saturated sodium bicarbonate, then with water, then three times with 1-molar citric acid, dried over $MgSO_4$ and concentrated in vacuo. The residue was chromatographed on silica gel (dichloromethane/methanol (20:1) to yield 12.9 g (82%) of the title compound. Rf: 0.73 (dichloromethane/methanol 10:1).

Example 22: N-[N-[2-amino-3-phenyl-propyl]-L-prolyl]-L-isoleucinbenzylester hydrochloric acid salt (1:2)

8.7 g (15.8 mmol)N-[N-[(1,1-Dimethylethoxy)carbonyl]amino-3-phenyl-propyl]-L-prolyl]-L-isoleucinben zylester were mixed with 5 ml ethyl acetate followed by the addition of 20 ml of a 4M solution of hydrogen chloride in ethyl acetate. The mixture is stirred at 0 °C compound in quantitative yield. Rf: 0.26 (Dichloromethane/Methanol 10:1)

Example 23: N-[N-[2-[N2-[(1,1-Dimethylethoxy)carbonyl]-L-asparagyl]amino-3-phenyl-propyl]-L-prolyl]-L-isoleucinbenzylester

2.8 g (6.2 mmol) N-[N-[2-amino-3-phenyl-propyl]-L-prolyl-L-isoleucinbenzylester and 1.45 g (6.3 mmol) of Boc-L-asparagine were dissolved in 15 ml of dimethylformamide followed by the addition of 0.72 g (6.3 mmol) N-ethylmorpholine, 0.83 g (6.1 mmol) 1-hydroxybenzotriazole hydrate and 1.42 g dicyclohexylcarbodiimide. The resulting mixture is stirred at 0 °C for one hour and then at room temperature for 15 hours. The solution is concentrated in vacuo and the residue is chromatographed on silica gel (dichloromethane/methanol 19:1) to yield 3.16 g (77%) of the title compound. Rf: 0.60 (dichloromethane/methanol 20:3).

Example 24: N-[N-[2-[-L-asparagyl]amino-3-phenyl-propyl]-L-prolyl-L-isoleucinbenzylester hydrochloric acid salt (1:2)

4 g (6.0 mmol) N-[N-[2-[$N^2$-[(1,1-Dimethylethoxy) carbonyl]-L-asparagyl]amino-3-phenyl-propyl]-L-prolyl]-L-isoleucinbenzylester were dissolved in 6 ml of a 4M solution of hydrogen chloride in ethyl acetate and stirred at 0 °C for 4 hours. The solution was concentrated in vacuo and dried to yield 3.75 g (98%) of the title compound. Rf: 0.55 (dichloromethane/methanol 50:4).

Example 25: N-[N-[2-[[N2-[N-[(1,1-Dimethylethoxy)carbonyl]-L-phenylalanyl]-L-asparagyl]amino]-3-phenylpropyl]-L-prolyl]-L-isoleucinbenzylester

3.6 g (5.6 mmol) N-[N-[2-[-L-asparagyl]amino-3-phenyl-propyl]-prolyl-L-isoleucinbenzylester and 1.85 g (7 mmol) Boc-L-phenylalanine were dissolved in 13 ml dimethylformamide followed by the addition of 3.7 ml (29 mmol) N-ethylmorpholine and 4.9 g (8 mmol) 1-propane-phosphonic acid cyclic anhydride (50% in dichloromethane). The resulting mixture was stirred at 5 °C for 2.5 hours and concentrated in vacuo. The residue was dissolved in ethyl acetate and water and washed three times with 1M citric acid, with water, three times with saturated sodium bicarbonate, dried over $MgSO_4$ and concentrated in vacuo. The residue was chromatographed on silica gel (dichloromethane/acetone 4:1) to yield 3.88 g (85%) of the title compound. Rf: 0.71 (dichloromethane/methanol 50:4).

Example 26: N-[N-[2-[[N2-[-L-phenylalanyl]-L-asparagyl]-amino]-3-phenyl-propyl]-L-prolyl]-L-isoleucinbenzylester hydrochloric acid salt (1:1)

2.5 g (3.1 mmol) N-[N-[2-[[N-[(1,1-Dimethylethoxy)carbonyl]-L-phenylalanyl]-L-asparagyl]amino]-3-phenylpropyl]-L-prolyl]-L-isoleucinbenzylester were dissolved in 7 ml of a 4M solution of hydrogen chloride in ethyl acetate and stirred for 3 hours at 0 °C. The solvent was stripped of to yield 2.4 g (99%) of the title compound. Rf: 0.36 (dichloromethane/methanol 20:3).

Example 27:

N-[N-[2-[[N2-[N-[N-[(1,1-dimethylethoxy)carbonyl]-L-seryl]-L-phenylanyl]-L-asparagyl]-amino]-3-phenylpropyl]-L-prolyl]-L-isoleucinbenzylester

2 g (2.5mmol) of title compound in example 26 and 0.65 g (3.2mmol) of BOC-serine were dissolved in

12 ml dimethylformamide followed by the addition of 1.9 ml (14.9 mmol) N-ethylmorpholine and 2.2 g (3.6 mmol)1-propane-phosphonic acid cyclic anhydride (50% in dichloromethane). The resulting mixture was stirred at 5° C for 18 hours and concentrated in vacuo. The residue was dissolved in ethyl acetate and water and washed 3 times with 1M citric acid, with water, 3 times saturated sodium bicarbonate, dried with magnesium sulfate and concentrated in vacuo. The residue was chromatographed on silica gel (dichloromethane/methanol 10:1) to yield 1.8 g (80%) of the title compound. Rf: 0.39 (dichloromethane/methanol/ammonia 10:1:0.1)

Example 28:

N-[N-[2-[[N2-[N-[N-L-seryl]-L-phenylalanyl]-L-asparagyl]-amino]-3-phenyl-propyl]-L-prolyl]-L-isoleucinbenzylester hydrochloric acid salt (1:2)

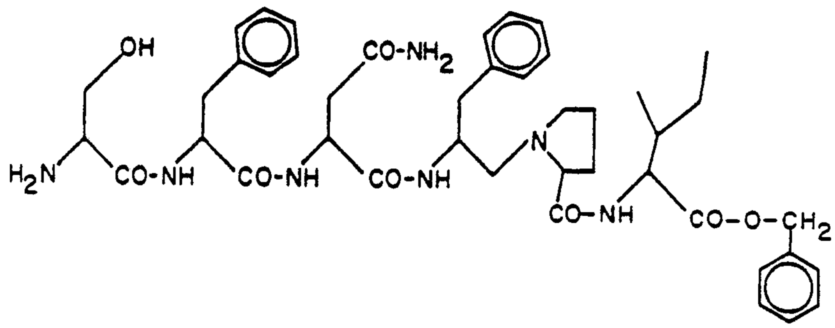

1.5 g (1.7 mmol) of title compound in example 27 were dissolved in 2.5 ml of 4M hydrogen chloride in ethyl acetate and stirred at 0° C for 2 hours. The solution was concentrated in vacuo. The residue was dissolved in ethyl acetate, concentrated and dried in vacuo to yield 1.43 g (96%) of the title compound. Rf: 0.21 (dichloromethane/methanol/ammonia 10.1:0.1).

Example 29: N-[N-[2-[[N2-[N-[N-[N-[(1,1dimethyl ethoxy)carbonyl]-L-valyl]-L-seryl]-L-phenylanyl]-L-asparagyl]-amino]-3-phenyl-propyl]-L-prolyl]-L-isoleucinebenzylester

1.4 g (1.6 mmol) of title compound in example 28 and 0.44 g (2 mmol) of BOC-valine were dissolved in 8 ml dimethylformamide and cooled to 0° C. 1.1 ml (8.6 mmol) N-ethylmorpholine and 1.6 g (2.6 mmol) 1-propane phosphonic acid cyclic anhydride (50% in dichloromethane) were added and the mixture was stirred for 18 hours and concentrated in vacuo. The residue was dissolved in 100 ml dichloromethane and extracted 3 times with 1M citric acid, with water, 2 times with saturated sodium bicarbonate, dried with magnesium sulfate, and concentrated in vacuo. The residue was chromatographed on silica gel (dichloromethane/acetone/ethanol 10:7:1) to yield 0.45 g (28%) of the title compound. Rf:0.38 (dichloromethane/acetone/ethanol 10:7:1).

Example 30:

1-[(Benzyloxycarbonyl)amino]-2-phenylethylphosphinic acid

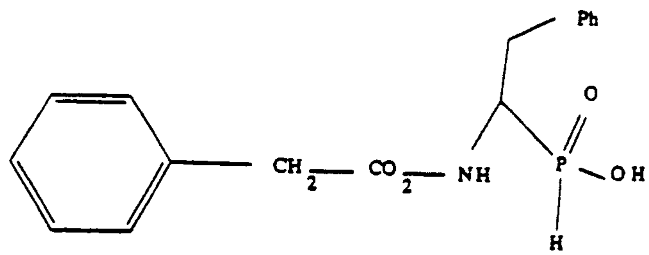

A solution of 30 g (0.162 mol) of 1-amino-2-phenylethylphosphinic acid (J.Chem. Soc. Perkin Trans. I, 2845 (1984)) in 460 ml of dioxane and 175 ml of 1 N NaOH was cooled to 0 °C and stirred vigorously while 47.7 ml (0.32 mol) of benzyl chloroformate and 345 ml of 1 N NaOH was cooled to 0 °C and stirred vigorously while 47.7 ml (0.32 mol) of benzyl chloroformate and 345 ml of 1 N NaOH were added simultaneously. The reaction mixture was vigorously stirred at room temperature for 20 hours. Most of the dixoane was then removed in vacuo and the aqueous solution extracted twice with ether. The ether layer was discarded and the aqueous layer acidified with aqueous $KHSO_4$ to pH 1-2. The resulting suspension was extracted five times with ethyl acetate, the combined extracts dried ($Na_2SO_4$), and concentrated. The residue was vigorously stirred with ether, filtered and dried in vacuo to give 49.3 g (95.3%) of the product as a white solid.
MS: m z 342 (M + Na)
$R_F$ (140:80:20:1 chloroform/methanol/water/glacial acetic acid): 0.53

Example 31:

Methyl-1-[(Benzyloxycarbonyl)amino]-2-phenylethylphosphinate

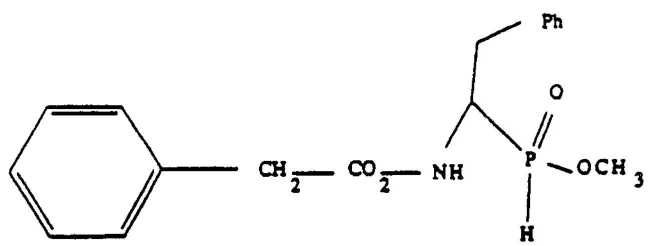

A solution of 9 g (28.2 mmol) of the phosphinic acid in 300 ml of ethyl acetate and 25 ml of methanol was added at 0 °C to a freshly prepared etheral diazomethane solution (excess). The solution was stirred at room temperature for 1 hour and then excess diazomethane destroyed by addition of a few ml of glacial acetic acid. The solution was concentrated and the residue chromatographed on silica gel with 30:1 dichloromethane/methanol to give 4.84 g (51.5%) of the product.
$R_F$ (20:1 dichloromethane/methanol): 0.52

Example 32:

2-[[1-[(Benzyloxycarbonyl)amino]-2-phenylethyl](methoxy)phosphinyl]cyclopentanecarbonylmethylester

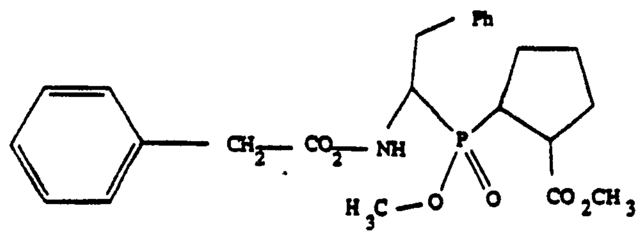

A solution of 4.83 g (14.49 mmol) of the phosphinate in 40 ml of dry methanol was cooled to 0° C, and 978 mg (18.11 mmol) of sodium methylate were added. The mixture was stirred at 0° C for 10 minutes, at which time 2.28 g (18.11 mmol) of methyl cyclopentene-1-carboxylate were added all at once. The mixture was stirred at 0° C for 30 minutes and then at room temperature overnight. Another 98 mg (1.81) mmol) of sodium methylate were added, and stirring was continued at room termperature for 3 hours. The solution was then concentrated, the residue treated with 0.1 N HCl, and the aqueous solution extracted three times with ethyl acetate. The combined extracts were dried ($Na_2SO_4$), concentrated, and the residue chromatographed on silica gel with 30:1 dichloromethane/methanol to give 3.68 g (55.3%) of the product.

MS: m/z 460 (M + H)

$R_F$ (20:1 dichloromethane/methanol): 0.51

Example 33

2-[[1-[(Benzyloxycarbonyl)amino]-2-phenylethyl](methoxy)phosphinyl]cyclopentanecarboxylic acid

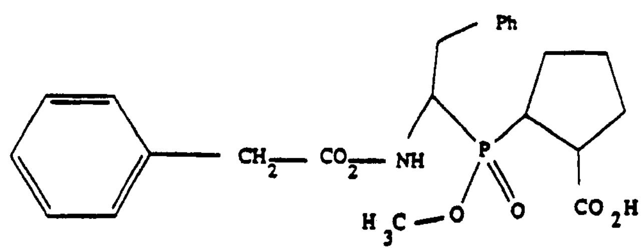

2.82 g (6.14 mmol) of the ester were dissolved in 12 ml of dioxane and 6.4 ml of 1 N NaOH, and the solution stirred at room temperature overnight. The mixture was extracted twice with ethyl acetate, the combined extracts dried ($Na_2SO_4$), and concentrated to give 2.66 g (97.3%) of the product.

MS: m/z 446 (MH)

$R_F$ (9:1:0.1 dichloromethane/emthanol/formic acid) : 0.39

Example 34:

N-[2-[[1-[(Benzyloxycarbonyl)amino]-2-phenylethyl](methoxy)phosphinyl]cyclopentanecarbonyl]-L-isoleucine-methylester

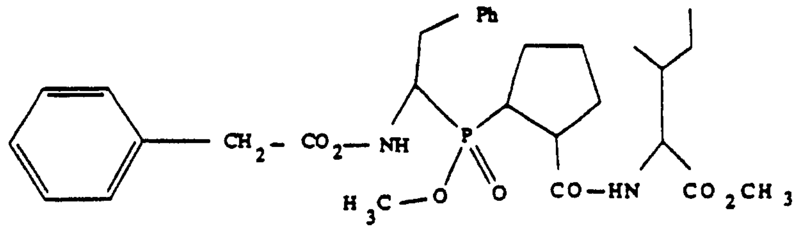

To a stirred solution of 2.64 g (5.93 mmol) of the acid and 1.238 g (6.82 mmol) of L-isoleucine methyl ester hydrochloride in 50 ml of dichloromethane were added at 0°C 1.74 ml (13.63 mmol) of N-ethylmorpholine, 0.962 g (7.12 mmol) of 1-hydroxybenzotriazole, and 1.346 g (6.52 mmol) of dicyclohexylcarbodiimide. The mixture was stirred at 0°C for 30 minutes, and then at room temperature overnight. The solution was filtered, and the filtrate was washed twice with saturated aqueous $NaHCO_3$ and with brine. The organic layer was dried ($Na_2SO_4$), concentrated, and the residue chromatographed on silica gel with 30:1 dichloromethane/ methanol to give 2.645 g (77.9%) of the product.

MS: m/z 573 (M+H)

$R_F$ (20:1 dichloromethane/methanol) : 0.53

Example 35:

N-[2-[[1-[(Benzyloxycarbonyl)amino]-2-phenylethyl](methoxy)phosphinyl]cyclopentanecarbonyl]-L-isoleucine-(2-pyridyl)methylamide

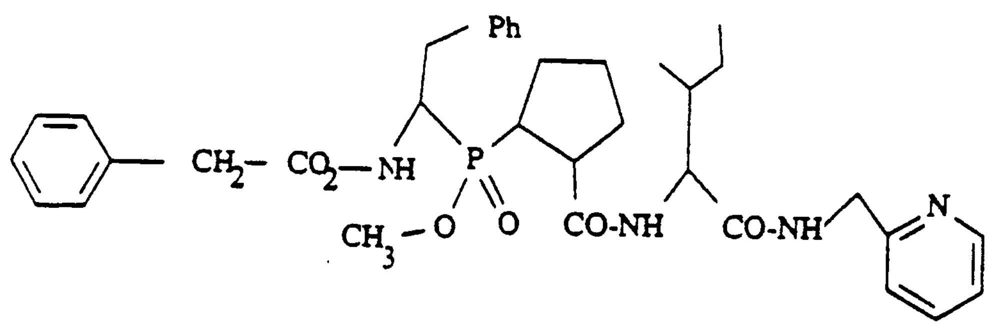

To a stirred solution of 2.93 g (6.58 mmol) of the acid and 2.226 g (7.56 mmol) of L-isoleucine 2-aminomethyl- pyridyl amide dihydrochloride in 55 ml of dichloromethane were added 0°C 2.9 ml (22.7 mmol) of N-ethylmorpholine, 1.07 g (7.89) mmol) of 1-hydroxybenzotriazole, and 1.493 g (7.24 mmol) of dicyclohexylcarbodiimide. The mixture was stirred at 0°C for 30 minutes, and then at room temperature overnight. The solution was filtered, and the filtrate was washed twice with saturated aqueous $NaHCO_3$ and with brine. The organic layer was dried ($Na_2SO_4$), concentrated, and the residue chromatographed on silica gel with 30:1 dichloromethane/methanol to give 2.56 g (60%) of the product.

MS: m/z 649 (M+H)

$R_F$ (20:1 dichloromethane/methanol): 0.35

Example 36:

N-[2-[(1-amino-2-phenylethyl) (methoxy)phosphinyl]cyclopentanecarbonyl]-L-isoleucine-(2-pyridyl)-methylamide

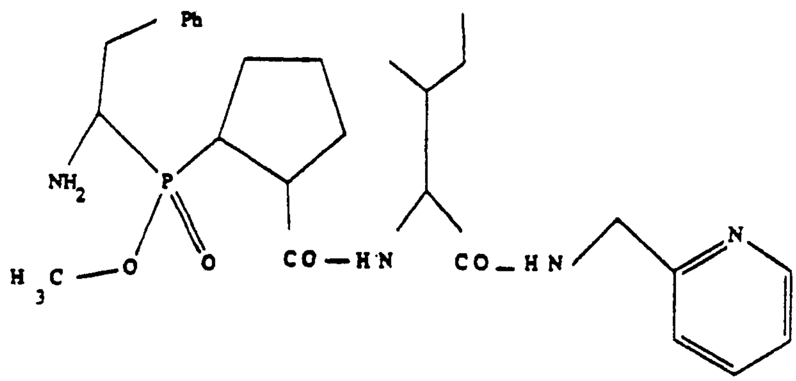

To a solution of 2.54 g (3.92 mmol) of the urethane in 60 ml of methanol were added 2.46 g (39.2 mmol) of ammonium formate and 0.65 g of 10% palladium on charcoal. The mixture was heated under reflux in an argon atomsphere for 1 hour, then cooled to room temperature and filtered. The filtrate was concentrated, the residue taken up in dichloromethane, re-filtered, and the filtrate again concentrated to give 1.98 g (98.2%) of the product.
MS: m/z 515 (M+H)
$R_F$ (9:1:0.1 dichloromethane/methanol/concentrated aqueous $NH_3$) : 0.50

Example 37:

N-[2-[(1-amino-2-phenylethyl)methoxy)phosphinyl]cyclopentanecarbonyl]-L-isoleucinemethylester

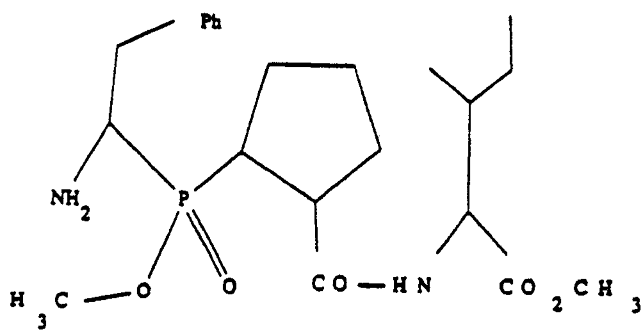

To a solution of 2.45 g (4.28 mmol) of the urethane in 55 ml of methanol were added 2.7 g (42.8 mmol) of the ammonium formate and 0.60 g of 10% palladium on charcoal. The mixture was heated under reflux in an argon atmosphere for 1 hour, then cooled to room temperature and filtered. The filtrate was concentrated, the residue taken up in dichloromethane, re-filtered, and the filtrate again concentrated to give 1.87 g (99.6%) of the product.
MS: m/z 439 (M+H)
$R_F$ (20:1 dichloromethane/methanol): 0.32 and 0.25

Example 38:

N-[2-[[1-[[$N^2$-(tert.-Butoxycarbonyl)-L-asparagyl]amino]-2-phenylethyl] (methoxy)phosphinyl]cyclopentanecarbonyl]-L-isoleucine-(2-pyridyl)methylamide

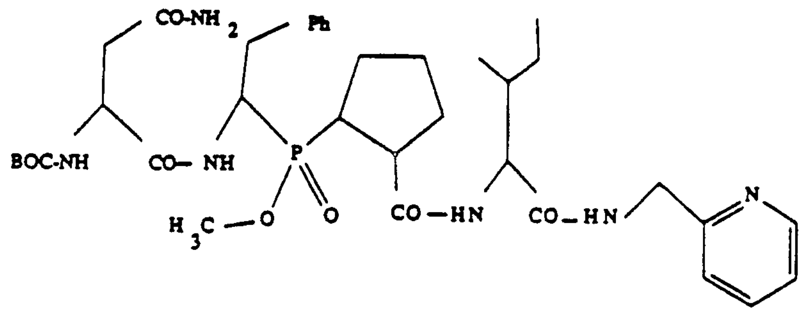

To a stirred solution of 1.97 g (3.83 mmol) of the amine and 0.98 g (4.21 mmol) of Boc-L-asparagine in 12 ml of DMF were added at 0 °C 0.65 g (4.79 mmol) of 1-hydroxybenzo-triazole and 0.908 g (4.4 mmol) of dicyclohexylcarbodiimide. The mixture was stirred at 0 °C for 30 minutes, and then at room temperature overnight. The solution was filtered, and th DMF removed in vacuo.

The residue was taken up in ethyl acetate and washed twice with saturated aqueous $NaHCO_3$ and with brine. The organic layer was dried ($Na_2SO_4$), concentrated, and the residue chromatographed on silica gel with 15:1:0:1 dichloromethane/methanol/concentrated aqueous $NH_3$ to give 1.29 g (46.2%) of the product.

MS: m z 729 (M+H)

$R_F$ (9:1:0.1 dichloromethane/ethanol/concentrated aqueous $NH_3$): 0.48

Example 39:

N-[2-[[1-[[$N^2$-(tert.-Butoxycarbonyl)-L-asparagyl]amino]-2-phenylethyl] (methoxy)phosphinyl]cyclopentane-carbonyl]-L-isoleucinemethylester

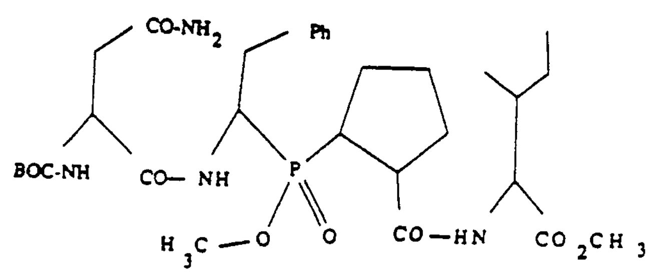

To a stirred solution of 1.86 g (4.24 mmol) of the amine and 1.09 g (4.7 mmol) of Boc-L-asparagine in 13 ml of DMF were added at 0 °C 0.72 g (5.3 mmol) of 1-hydroxybenzo-triazole and 1.0 g (4.88 mmol) of dicyclohexylcarbodiimide. The mixture was stirred at 0 °C for 30 minutes, and then at room temperature overnight. The solution was filtered, and the DMF removed in vacuo. The residue was taken up in ethyl acetate and washed twice with saturated aqueous $NaHCO_3$ and with brine. The organic layer was dried ($Na_2SO_4$), concentrated, and the residue chromatographed on silica gel with 15:1:0.1 dichloromethane/methanol/concentrated aqueous $NH_3$ to give 1.94 g (70.1%) of the product.

MS: m/z 653 (M+H)

$R_F$ (9:1:0.1) dichloromethane/methanol/concentrated aqueous $NH_3$): 0.52

Example 40:

N-[2-[[1-[(L-Asparagyl)amino]-2-phenylethyl] (methoxy)phosphinyl]cyclopentanecarbonyl]-L-isoleucine(2-pyridyl)methylamide

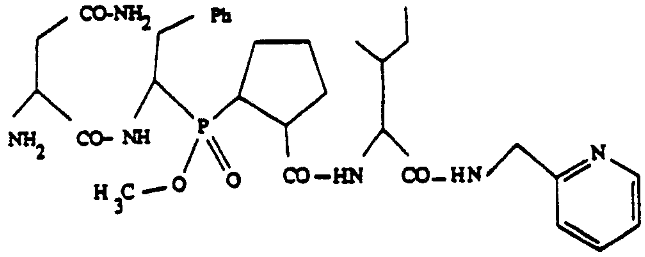

A solution of 1.11 g (1.52 mmol) of the urethane in 16 ml of a 4 N solution of gaseous HCl in dioxane and 3 ml of dry methanol was stirred at 0 °C for 1 hour and at room temperature for 1 hour. The solution was then concentrated, the residue repeatedly triturated with dry ether and again concentrated, and finally dried in vacuo to give 1.07 g (100%) of the product.
MS: m/z 629 (M + H)
$R_F$ (9:1:0.1 dichloromethane/methanol/concentrated aqueous $NH_3$) : 0.20

Example 41:

N-[2-[[1-[N-(L-asparagyl)amino]-2-phenylethyl] (methoxy) phosphinyl]cyclopentanecarbonyl]-L-isoleucine-methylester

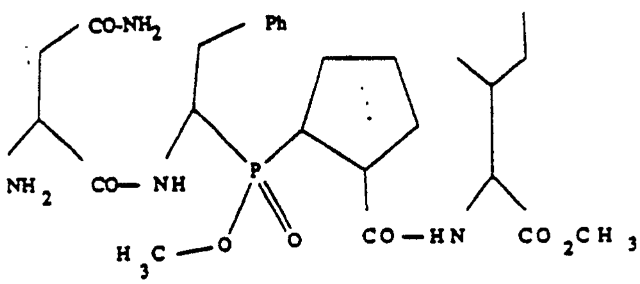

A solution of 1.67 g (2.56 mmol) of the urethane in 27 ml of a 4N solution of gaseous HCl in dioxane and 5 ml of dry methanol was stirred at 0 °C for 1 hour and at room temperature for 1 hour. The solution was then concentrated, the residue repeatedly triturated with dry ether and again concentrated, and finally dried in vacuo to give 1.51 g (100%) of the product. MS: m/z 553 (M + H)
$R_F$ (9:1:0.1 dichloromethane/methanol/concentrated aqueous $NH_3$): 0.29

Example 42:

N-[2-[[1-[[$N^2$-[N-[tert.-Butoxycarbonyl)-L-phenylalanyl]-L-asparagyl]amino]-2-phenylethyl] (methoxy)-phosphinyl] cyclopentanecarbonyl]-L-isoleucine-(2-pyridyl)methylamide

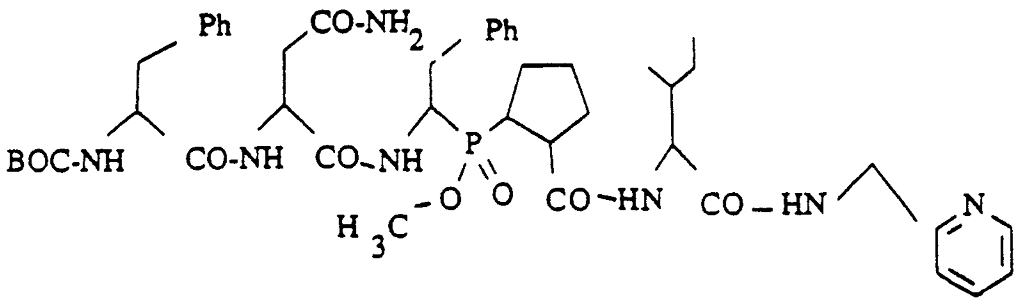

To a solution of 445 mg (1.68 mmol) of Boc-L-phenylalanine in 7 ml of dichloromethane at 0° C were added 247 mg (1.83 mmol) of 1-hydroxybenzotriazole and 361 mg (1.75 mmol) of dicyclohexylcarbodiimide. After stirring at 0° C for 2 hours, a solution of 1.07 g (1.52 mmol) of the amine and 0.58 ml (4.57 mmol) of N-ethylmorpholine in 10 ml of DMF was added, and the mixture was stirred at room temperature overnight. The solution was filtered, and the DMF removed in vacuo. The residue was taken up in dichloromethane and washed twice with saturated aqueous $NaHCO_3$ and with brine. The organic layer was dried ($Na_2SO_4$), concentrated, and the residue chromatographed on silica gel with 15:1:0:1 dichloromethane/methanol/concentrated aqueous $NH_3$ to give 0.90 g (67.5%) of the product.
MS: m z 876 (M+H)
$R_F$ (9:1:0.1 dichloromethane/methanol/concentrated aqueous $NH_3$) 0.42

Example 43:

N-[2-[[1-[[N²-[N-(tert.-Butoxycarbonyl)-L-phenylalanyl]-L-asparagyl]amino]-2-phenylethyl] (methoxy) phosphinyl]cyclopentanecarbonyl]-L-isoleucinemethylester

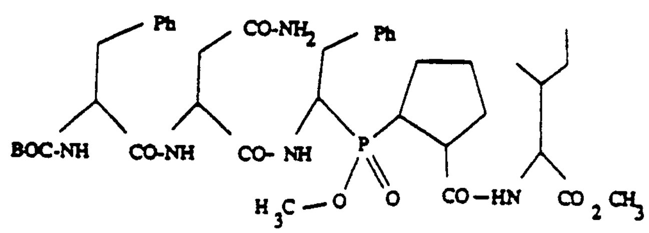

To a solution of 747 mg (2.82 mmol) of Boc-L-phenylalanine in 12 ml of dichloromethane at 0° C were added 415 mg (3.07 mmol) of 1-hydroxybenzotriazole and 607 mg (2.94 mmol) of dicyclohexylcarbodiimide. After stirring at 0° C for 2 hours, a solution of 1.51 g (2.56 mmol) of the amine and 0.65 ml (5.12 mmol) of N-ethylmorpholine in 16 ml of DMF was added, and the mixture was stirred at room temperature overnight. The solution was filtered, and the DMF removed in vacuo. The residue was taken up in ethyl acetate and washed twice with saturated aqueous $NaHCO_3$ and with brine. The organic layer was dried ($Na_2SO_4$), concentrated, and the residue chromatographed on silica gel with 15:1:0.1 dichloromethane/methanol/concentrated aqueous $NH_3$ to give 1.51 g (73.7%) of the product.
MS: m/z 800 (M+H)
$R_F$ (9:1:0.1 dichloromethane/methanol/concentrated aqueous $NH_3$): 0.56

Example 44:

N-[2-[[1-[[N²-[N-(tert.-Butoxycarbonyl)-L-phenylalanyl]-L-asparagyl]amino]-2-phenylethyl]phosphinyl]-cyclopentanecarbonyl]-L-isoleucinemethylester

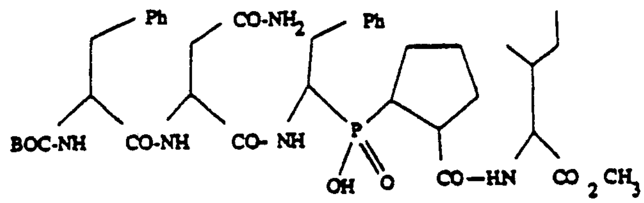

To a solution of 350 mg (0.44 mmol) of the methyl phosphinate in 3 ml of dry THF were added 118 mg (0.88 mmol) of lithium iodide. The mixture was stirred at room temperature for 36 hours, then another 59 mg (0.44 mmol) of lithium iodide were added, and stirring was continued at room temperature for 24 hours. The solution was concentrated and the residue was partitioned between ether and 5% aqueous $NaHCO_3$. The ether layer (containing some starting material) was discarded and the aqueous layer acidified with 1 N HCl to pH 1-2. The mixture was extracted three times with ethyl acetate, the combined extracts dried ($Na_2SO_4$), and concentrated. The residue was stirred with ether, filtered and dried in vacuo to give 173 mg (50%) of the product.
MS: m/z 786 (M+H)
$R_F$ (140:80:20:1 chloroform/methanol/water/glacial acetic acid): 0.69

Example 45:

N-[2-[[1-[[$N^2$-(tert.-Butoxycarbonyl)-L-phenylalanyl]-L-asparagyl]amino]-2-phenylethyl]phosphinyl]-cyclopentanecarbonyl]-L-isoleucine-(2-pyridyl)methylamide

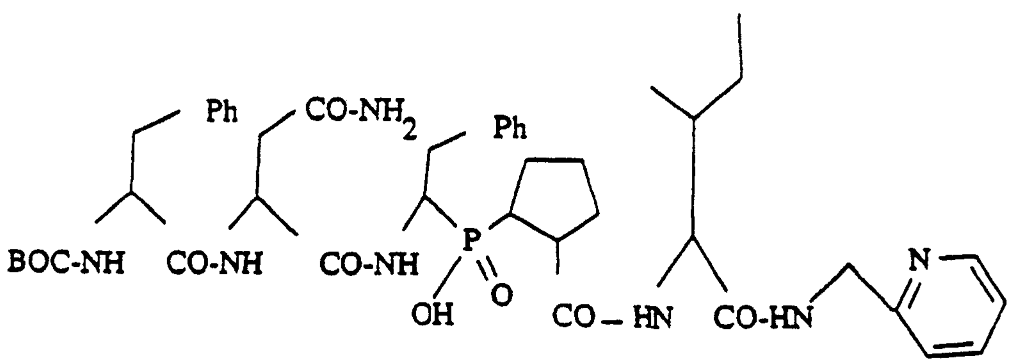

To a solution of 400 mg (0.46 mmol) of the methyl phosphinate in 3 ml of dry THF were added 123 mg (0.92 mmol) of lithium iodide. The mixture was stirred at room temperature for 24 hours, then another 123 mg (0.92 mmol) of lithium iodide were added, and stirring was continued at room temperature for two days. The solution was concentrated, and the residue was partitioned between ether and 5% aqueous $NaHCO_3$. The ether layer was discarded and the aqueous layer acidified with 1 N HCl to pH 4-5. The mixture was extracted twice with dichloromethane, the combined extracts dried ($Na_2SO_4$), and concentrated. The residue was stirred with ether, filtered and dried in vacuo to give 90 mg (22.7%) of the product.
MS: m/z 862 (M+H)
$R_F$ (140:80:20:1 chloroform/methanol/water/glacial acetic acid): 0.70

Example 46:

N-[2-[[1-[[$N^2$-(-L-Phenylalanyl)-L-asparagyl]amino]-2-phenylethyl] (methoxy)phosphinyl]-cyclopentanecarbonyl]-L-isoleucinemethylester

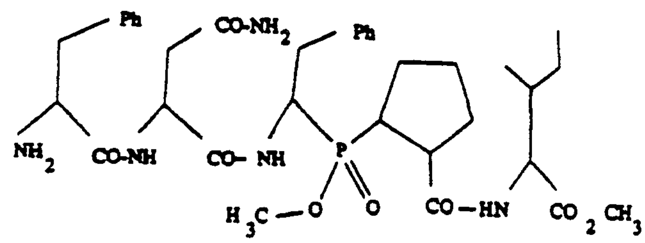

A solution of 0.8 g (1 mmol) of the urethane in 11 ml of a 4 N solution of gaseous HCl in dioxane and 5 ml of dry methanol was stirred at 0° C for 1 hour and at room temperature for 1 hour. The solution was then concentrated, the residue repeatedly triturated with dry ether and again evaporated, and finally dried in vacuo to give 0.75 g (100%) of the product.
MS: m/z 700 (M + H)
$R_F$ (9:1:0:1 dichloromethane/methanol/concentrated aqueous $NH_3$) : 0.40

Example 47:

N-[2-[[1-[[N²-[N-[N-(tert.-Butoxycarbonyl)-L-serinyl]-L-phenylalanyl]-L-asparagyl]amino]-2-phenylethyl]-(methoxy)phosphinyl]cyclopentanecarbonyl]-L-isoleucinemethylester

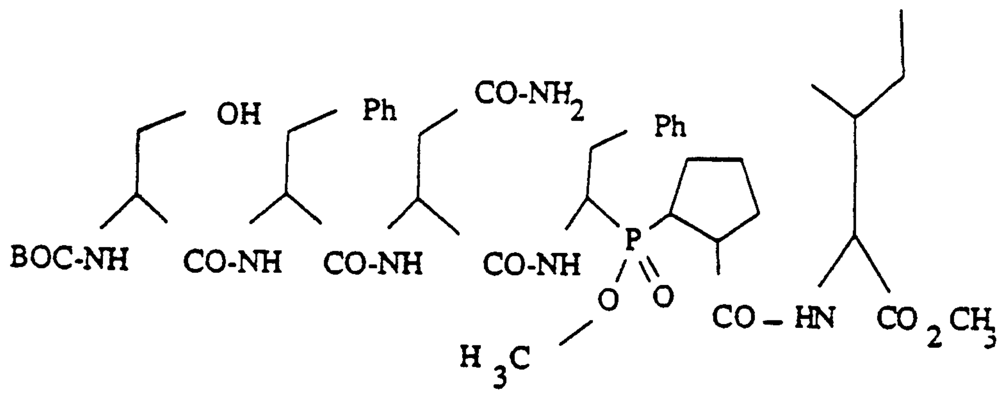

To a solution of 226 mg (1.1 mmol) of Boc-L-serine in 5 ml of dichloromethane at 0° C were added 162 mg (1.2 mmol) of 1-hydroxybenzotriazole and 237 mg (1.15 mmol) of dicyclohexylcarbodiimide. After stirring at 0° C for 12 hours, a solution of 0.75 g (1 mmol) of the amine and 0.25 ml (2 mmol) of N-ethylmorpholine in 6 ml of DMF was added, and the mixture was stirred at room temperature for two days. The mixture was filtered, the filtrate concentrated, the residue taken up in ethyl acetate, and washed twice with saturated aqueous $NaHCO_3$ and with brine. The organic layer was dried ($Na_2SO_4$), concentrated, and the residue chromatographed on silica gel with 15:1:0.1 dichloro- methane/methanol/concentrated aqueous $NH_3$): 0.46
MS: m/z 887 (M + H)
$R_F$ (9:1:0.1 dichloromethane/methanol/concentrated aqueous $NH_3$): 0.46

Example 48

[IR,2R,2(4S,5R)]-[2-(4-Benzyl-3-tert.-butoxycarbonyl-2,2-dimethyl-1,3-oxazolidine-5-yl)cyclopentancarbonyl]-L-isoleucine-O-benzylester

241 mg (90.53 mmol) of the compound of example 11 of formula IV and 239 mg (0.61 mmol)

isoleucinebenzylester-toluenesulfonic acid salt were dissolved in 3ml N,N-dimethylformamide. After addition of 369 mg (3.21 mmol) N-ethylmorpholine the solution was cooled to 0° C and 530 mg of a 50% solution of propanephosphonicacid-anhydride (0.83 mmol) in dichloromethane were added. The resulting mixture was stirred at 0° C for 4h and the at 20° C for 24h. All the solvents were stripped off and the residue was taken up in dichloromethane and ethylacetate (9:1; 15 ml), extracted with citric acid ( 1M ; 10 ml), saturated sodiumhydrogencarbonate ( 10 ml ) saturated sodium-chloride ( 5 ml ) and dried over MgSO4. The solvents were stripped off and the residue was chromatographed on silica gel (20µm) with ethylacetate/hexane 1:1 to give 246 mg of the title compound (76%).

$C_36H_{51}N_2O_6$ (607.79)

$R_f$(hexane/ethylacetate 5:1) : 0.20

Example 49

N-[2-(2-amino-1-hydroxy-3-phenyl-propyl)cyclopentancarbonyl]-L-isoleucine-O-benzylester hydrochloric salt

190 mg (313µmol) of the preceding compound were dissolved in 1 ml ethylacetate. The resulting solution was evaporated to dryness and the residue treated with 3 ml of HCl in ethylacetate (4N). The resulting mixture wa stirred at 5° C until the reaction was complete (TLC control). All the solvents were removed in vacuo. The residue was used without further purification for the next step.

$C_{28}H_{39}ClN_2O_4$ (503.06)

$R_f$(hexane/ethylacetate 1:1): 0.05

Example 50

N-[2-[2-[N[2]-[(1,1-Dimethylethoxy)carbonyl]-L-asparagyl]amino-1-hydroxy-3-phenyl-propyl]-cyclopentancarbonyl]-L-isoleucine-O-benzylester

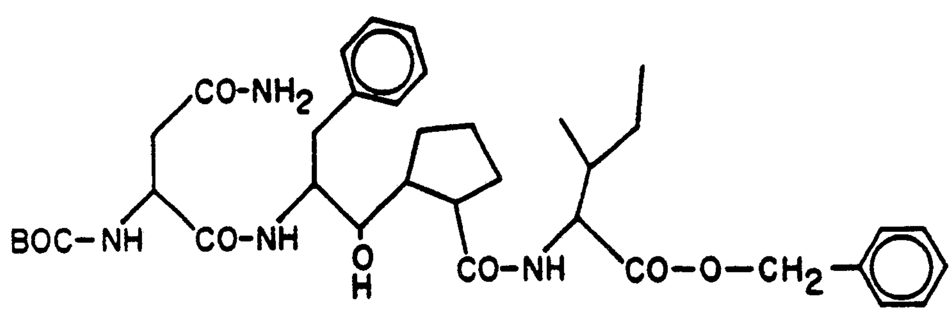

79 mg (341µmol) Boc-Asn-OH were dissolved in 300 µl N,N-dimethylformamide and added to 170 mg (338µmol) of the preceding compound followed by the addition of a solution of 70 mg (340 µmol) N,N′-dicyclohexylcarbodiimide and 39 mg (340µmol) N-ethylmorpholine and 46 mg (340 µmol) hydroxybenzotriazol in 700 µl N,N-dimethylformamide. The resulting mixture was stirred for 2 h at 0° C and then 20 h at 20° C. The solvents were stripped off and the residue was taken up in 120 ml $CH_2Cl_2$ and 30 ml ethylacetate. The organic layer was ectracted with citric acid (25 ml, 1M), saturated sodiumhydrogencarbonate (25 ml) and saturated NaCl (25ml), dried over $MgSO_4$ and evaporated to give 253 mg of a crude product, which was purified by chromatography on silica gel

(20µ) (Ethylacetate/$CH_2Cl_2$ 1:2 ⇒ $CH_2Cl_2$ ⇒ MeOH/$CH_2Cl_2$ 1:20) to yield 129 mg (56%) of the title compound.

$C_{37}H_{52}N_4O_8$ (680.82)

$R_f$(dichloromethane/methanol 10:1) : 0.57

Example 51

N-[2-[2-(-L-asparagyl)amino-1-hydroxy-3-phenyl-propyl]cyclo-pentancarbonyl]-L-isoleucine-O-benzylester hydrochloric salt

66mg (96µmol) of the preceding compound were suspended in 0.5 ml ethylacetate followed by the addition of 2.5 ml HCl in ethylacetate (4N). The resulting transparent solution was kept at 4°C for 11h. All the solvents were stripped of to yield 59 mg of a product, which was used without further purification.

$C_{32}H_{45}ClN_4O_6$ (617.16)

$R_f$ (dichloromethane/methanol 10:1): 0.13

Example 52

N-[2-[2-[$N^2$-[N-[(1,1-Dimethylethoxy)carbonyl]-L-phenylalanyl]-L-asparagyl]amino-1-hydroxy-3-phenyl-propyl]cyclo pentancarbonyl]-isoleucine-O-benzylester

54mg (87µmol) of the preceding compound were taken up in 0.8 ml N,N-dimethylformamide, followed by the addition of 30.5 mg (115 µmol) Boc-Phe-OH and 69 mg (0.6 mmol) N-ethylmorpholine. The resulting mixture was cooled to 4°C followed by the addition of 78µl (157 µmol) of a 50% solution of propanephosphonic acid anhydride in dichloromethane. After stirring at 4°C for 11 h all the solvents were removed in vacuo and the residue was taken up in 10 ml $CH_2Cl_2$ and 1ml ethylacetate, extracted with citric acid (1M, 10 ml), saturated $NaHCO_3$ (10 ml), saturated NaCl (10 ml), dried over $MgSO_4$ and evaporated to yield 77 mg of crude product. Chromatography on silica gel yields 61 mg (85%) of the title compound.

$C_{46}H_{6}\cdot N_5O_9$ (827.99)

$R_f$(dichloromethane/methanol 10:1) : 0.68

Example 53: IN VIVO ASSAY OF HIV PROTEASE ACTIVITY

H9 lymphocytes (1x $10^7$ cells/ml) were incubated in the presence or absence of HIV-I for 1 hour at 37°C. Cells were washed thoroughly to remove unadsorbed virions and resuspended at $4x10^5$ cells/ml in culture medium. Aliquots (one milliliter) were placed in wells of 24 well culture plates containing an equal volume of test compound (diluted in culture medium). After incubation for three days at 37°C, cell density of uninfected cultures was determined to assess toxicity of test compound. A p24 antigen capture assay was used to determine the level of HIV infection in HIV treated cultures. The ability of test compounds to inhibit HIV replication was measured at different concentrations of test compound relative to infected, untreated cultures.

Examples 54:

The compound of Example 25 showed 50% inhibition of p24 formation at a concentration of 200 µM in a test system according to Example 53.

Example 55:

The HIV protease purification procedure is described below. All operations were performed at room temperature, unless otherwise indicated.

A) Cell Lysis

Recombinant E. coli containing the MS2 expression plasmid were grown at 28°C in a liquid broth containing ampicillin (100 µg/ml) to a density corresponding to an absorbance of between 0.55 and 0.6 at 600nm. The growth temperature was then increased to 42°C over about 10 minutes and the culture was maintained at this temperature for 5 hours with vigorous agitation. The cells were harvested by centrifugation (8,000 RPM in a 6x400ml Sorvall GS-3 rotor), and frozen as a wet paste at -80°C until used. An

amount of paste (3.1 g) equivalent to 700ml of original culture was utilized for the purification. The cells were added to 15 ml of 50 mM tris-HCl pH8.0 containing 50mM sodium chloride, 1mM EDTA, and 0.1 mM PMSF (hereafter termed "buffer A") at room temperature. Hen egg lysozyme (E.C.3.2.1.17, Sigma (St. Louis, MO., U.S.A.) grade L-6876 was then added to 1mg/ml final. After 30 minutes, the sample was taken to -80° C, frozen then thawed to 4° C. Thirty minutes after thawing Nonidet NP-40 added (0.25%v final). Soon after detergent addition, the solution became viscous due to cell lysis and release of nucleic acids. After a further 30 minutes at room temperature, DNase I (Sigma (St. Louis, MO., U.S.A.), D-0876, 0.1 mg/ml final) and magnesium chloride (10 mM final) were added. Within 5 minutes the mixture transformed from a viscous to a free-flowing state.

B) Extraction of Inclusion Body Protein from the Whole Cell Lystate

The insoluble protein in the cell lysis mixture was collected by centrifugation (11,000 RPM 10 minutes in a Sorvall HB-4 rotor), then resuspended in 30ml of buffer A containing 0.5% (v/v) Triton X-100. The mixture was gently dounced, then swirled for 20 minutes at room temperature, then centrifuged (11,000 RPM x 10 minutes). The pellet was again resuspended in buffer A containing Triton X-100 and the above procedure repeated. The extraction into buffer A containing Triton was performed a total of three times in an identical manner. The pellet recovered from the third centrifugation of Triton treated mixture was resuspended in cartridge purified water (30 ml) by gentle dousing, swirled for 20 minutes then centrifuged (11,000 RPM x 20 minutes in a Sorvall HB-4 rotor). This procedure was performed a total of four times in an identical manner.

C) Extraction into Increasing Concentrations of Urea

The final pellet of inclusion body protein obtained above was resuspended into 30ml of 50mM tris pH8.0 containing 50mM sodium chloride and 2M urea. The protein pellet was resuspended by gentle douncing, rocked at room temperature for 90 minutes then centrifuged (11,000 RPM x 20 minutes). This procedure was performed a total of three times except on each successive occasion the final urea concentration in the buffer was increased by 2M. The protein was thus consecutively resuspended in buffer (30ml) containing 2M, 4M and 6M urea. Little or none of the enriched recombinant protein containing HIV-protease related material was solubilized at 2 to 6 M urea. About 50% of the MS2-protease was subsequently dissolved in 10ml of the above buffer containing 8M urea. The remaining non-solubilized MS2-protease fusion (recovered as a pellet by centrifugation at 11,000 RPM x 25 minutes) could be dissolved in 10ml of buffer containing 8M urea and 1% (v/v) 2-mercaptoethanol.

D) Refolding and Concomitant Cleavage of the MS2-HIV Protease Fusion to Yield a 10 kD HIV-Protease Molecule

The following room temperature, slow dialysis procedure was developed: Purified inclusion body protein (0.22 mg protein/ml as judged by the BCA assay (Pierce,Rockford, IL., U.S.A.) in 50mM tris buffer pH8.0 containing 50mM sodium chloride and 8M urea, was adjusted to 20mM Dithiothreitol (hereafter termed "DTT") then dialyzed against 50mM tris buffer pH6.8 containing 50mM sodium chloride, 20mM DTT and 6M urea (a volume ratio dialysis buffer/protein = 5 was used throughout). After no less than 2 hours, the protein was dialyzed against a fresh volume of the same buffer in which the final urea concentration had been decreased to 4M. At least 2 hours later, the protein was set dialyzing against a fresh buffer volume containing 2M urea and 25% (v/v) glycerol. After at least 2 hours the protein was transferred to dialyze against a fresh buffer volume containing 1.5M urea and 20% (v/v) glycerol. During this stage most of the HIV-protease was found to refold yielding an active protease capable of proteolytic cleavage of the MS2 fusion partner. After 2.5 hours the protein was dialyzed versus two consecutive five fold volume excesses of 50 mM sodium acetate pH 5.2, containing 50mM sodium chloride, 20mM DTT, 20% (v/v) glycerol, 0.1% (v/v) Triton X-100, and 1.5M urea. This caused the cleaved MS2 fusion partner, residual uncleaved MS2-HIV protease fusion and trace contaminating protein to precipitate from solution. These proteins could be removed by centrifugation (28,000 RPM x 25 minutes in a Beckman S.W. 41 Ti rotor) resulting in a supernatant containing soluble highly pure protease of approximate Mr = 10 kDa. Protein assay performed at this stage (Pierce (Rockford, IL., U.S.A.) BCA) indicated that a 2L cell culture (about 8.8g wet cell paste)

can yield approximately 1 milligram of purified protein. The urea could then be removed from this protease preparation by dialysis to completion versus 50 mM sodium acetate pH 5.2 containing 50 mM sodium chloride, 20 mM DTT, 20% (v/v) glycerol and 0.1% (v/v) Triton X-100. Indeed the protease was found to remain in solution if subsequently dialyzed to completion against 10mM sodium acetate pH 5.2.

Example 56 : In vitro assay for HIV protease activity

The example describes the use of N-Dansyl-SQNYPIV as a substrate for the HIV-protease. The amino acid sequence of this substrate corresponds to that which spans the cleavage site between p17 and p24 in the HIV gag poly-protein. The assay is based on measurements of the rate of enzymatic cleavage of N-dansyl SQNYPIV to yield N-dansyl-SQNY) and PIV.

A) Synthesis of peptide substrate (N-Dansyl-SQNYPIV) and authentic peptide product (N-Dansyl-SQNY

The peptides SQNYPIV and SQNY were synthesised on a model 430A peptide synthesizer (Applied Biosystems.Inc.) using standard t-boc chemistry according to Merrifield (Merrifield R.B (1963) J Amer Chem Soc 85, 2149-2154), and utilising Phenylacetamidomethyl resin. N-Dansylation of the peptides was performed prior to their being cleaved from the resin as follows; Resin-peptide (0.6g dry weight) was swelled in 5 ml of dimethylformamide/diisopropylethylamine (95:5), then mixed with an equal volume of the same solvent containing 0.56g of Dansyl chloride (Pierce chemical co). After an incubation period of 2h at room temperature, the resins were washed consecutively with an excess of each of the following solvents over a sintered glass funnel; dimethylformamide/diisopropylethylamine (95:5)-.dimethylformamide,dichloromethane,isopropanol, dimethylformamide. After drying the resins in vacuo, HF cleavage of the resin-bound N-dansyl peptides was performed according to Merrifield. Finally, the peptides were purified by successive HPLC (C18) utilising firstly a 50mM ammonium acetate/ acetonitrile linear gradient protocol, and then a 0.1% TFA/0.1%TFA-acetonitrile gradient system. The correct amino acid composition of both peptides were confirmed.

B) Incubation of HIV-protease with synthetic N-dansyl-SQNYPIV

Reactions are initiated at 37°C by addition of stock enzyme preparation (77µg/ml) to achieve the following t=0 component concentrations; 10kDa protease, (2.4-5.0µg/ml); N-Dansyl-SQNY-PIV, (100-170µM);sodium chloride, (2M); TWEEN-20, (0.017 %); Mes pH6.0 buffer, (50mM). At various time points following initiation of reactions, aliquots (5 to 10µl) are removed, acidified with 10 µl of2% (v/v) TFA then injected onto the HPLC system. Acidification was shown to effectively terminate the reactions, thus large numbers of samples can be accumulated and then set aside for subsequent automated analysis.

C) HPLC analysis of the reaction mixture in B

RP-HPLC is performed using a Hewlett Packard HP1090 complete with binary solvent delivery, heated column compartment and autoinjection capability. Fluorescence detection (post column) is performed with an in line Gilson model 121 filter fluorometer (emission at 480-520 nm) in conjunction with a Hewlett Packard Model HP3396A integrator. Aliquots (1 to 25 ul) of acidified enzymic reaction mixture containing unconverted substrate, (N-Dansyl-SQNY-PIV) and the products from enzymic proteolysis (N-Dansyl-SQNY and PIV) are then injected onto a Hypersil ODS, 5um column (4.6x100mm). Chromatography is performed at 1.2ml/minutes and 50°C. S62ubstrate and products are separated isochratically in 100mM sodium acetate pH6.5 containing 29% (v/v) acetonitrile. The following approximate retention times are observed; N-Dansyl-SQNY-PIV (1.9 minutes), N-Dansyl-SQNY (1.35 minutes). The time course of product formation can be used to calculate initial velocities if these are required for more rigorous enzymological studies. Alternatively, relative activities can be calculated from single time point measurements.

Incubation of the highly pure protease with the peptide substrate (170uM) in the pH range 4 to 8.5 resulted in a time-dependent interconversion of the substrate to dansylated product. No other metabolites are observed.The enzymatically generated fluorescent product was confirmed as N-Dansyl-SQNY by FAB-spectroscopy. The pH profile for the reaction is "bell shaped" exhibiting an optimum between pH 5.5 and

pH 6.0. The activity of the protease in this assay is strongly ionic strength dependent. The ionic strength optimum using sodium chloride as the salt in 25mM Mes buffer pH 6.0 was at 2M. Higher concentrations of salt produce a smaller stimulation of enzymatic activity. Enzymatic activity is not lost on extensive dilution of the enzyme.

Table I hereinbelow depicts the results both of the use of the above described 10 k Da protease assay as well as the cell infectivity assay in evaluating the inhibitory potency of the compounds of Examples described above.

TABLE I

| Compound of Example No. | Assay of Ex. 53 | Assay of Ex 57 |
|---|---|---|
| | % inhibition (uM) | $IC_{50}$ (uM) |
| 07 | 0 (100) | >1000* |
| 08 | 0 (100) | >1000* |
| 15 | 0 (100) | 75 |
| 15a | 0 (20) | 8 |
| 16 | 50 (100) | 21 |
| 19 | 0 (100) | >1000* |
| 20 | 0 (100) | >1000* |
| 21 | 0 (10) | 50 |
| 23 | 0 (10) | 29 |
| 24 | 0 (10) | >1000* |
| 25 | 40 (100) | 60 |
| 27 | 0 (10) | 20 |
| 28 | 0 (10) | 11 |
| 29 | 0 (100) | 20 |
| 42 | | 44 |
| 44 | | 7 |
| 45 | | 4 |
| 48 | | 140 |
| 50 | | 70 |

* < 10% inhibition observed at 100uM inhibitor concentration

It will be appreciated that the instant specification and claims are set forth by way of illustration and not limitation and the various modifications and changes may be made without departing from the spirit and scope of the present invention.

## Claims

1. A compound of the formula

$(A')_k-(Z)_n-(Y)_m-A''$

wherein

$A'$ is $R^1-\overset{O}{\overset{\|}{C}}$ , or hydrogen

$R^1$ is $OR^2$, $NR^3R^2$ or $CR^2R^3R^4$,

$R^2$, $R^3$, $R^4$ are independent of each other, hydrogen, an unsubstituted or substituted aliphatic group having 1 to 20 carbon atoms or an unsubstituted or substituted aromatic group having 6 to 18 carbon atoms, wherein k is one or zero, with the proviso that when Z is hydrogen, k is zero,

Z is hydrogen or amino acid residue Ser or Thr or

$R'-\overset{O}{\overset{\|}{C}}$ , wherein R' is as defined above, wherein n is one or zero with the proviso that when Y is hydrogen, n is zero,

Y is hydrogen or

$R^1-\overset{O}{\overset{\|}{C}}$ , wherein $R^1$ is as defined above or a group having the formula:

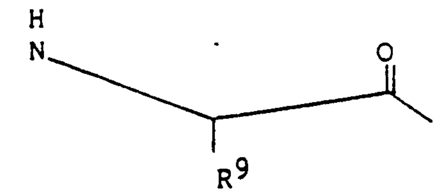

, wherein $R^9$ is hydrogen or an unsubstituted or substituted $C_1$-$C_{18}$ aliphatic group or an unsubstituted or substituted aromatic group with six to eighteen carbon atoms, wherein the substitutent is

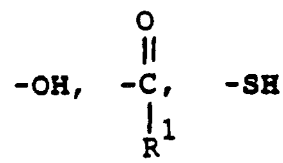

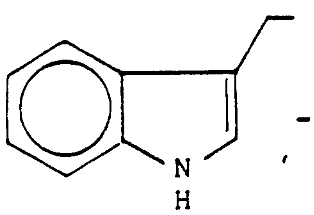 , -$NR^3R^2$ , 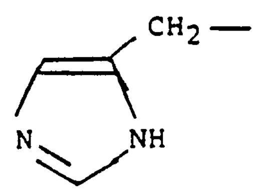 , 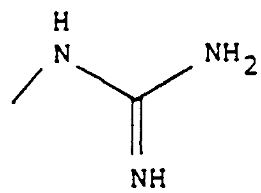

or

an aromatic group with 6 to 18 carbon atoms,

wherein R',$R^2$ and $R^3$ are defined as above, wherein m is one or zero, with the proviso that when $E^4$ is hydrogen, m is zero,

A″ is selected from the group consisting of

(a) 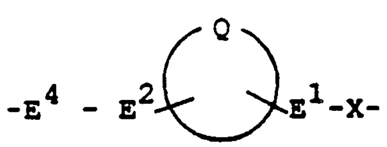 Q = S, $NR^2$, Oxygen or $CR^2R^3$,

(b) 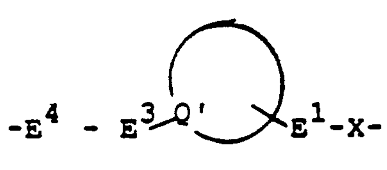 Q′ = N or $CR^2$ and

(c) 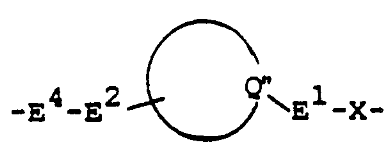 Q″ = N or $CR^2$,

wherein $E^4$ is hydrogen or amino acid residue Asn or

$R^{1}-\overset{O}{\overset{\|}{C}}$ ,
wherein $R^1$ is as defined above,
wherein the symbol

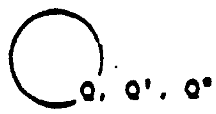

stands for a substituted or unsubstituted ring system, which is 4 to 7 membered, wherein only Q, Q′, Q″ may be different from carbon, wherein the substituent is a $C_1$-$C_{12}$ aliphatic or an aromatic group with six to twelve carbon atoms,
wherein $E^1$ is $\underset{\underset{O}{\|}}{C}$ ;
wherein $E^3$ is

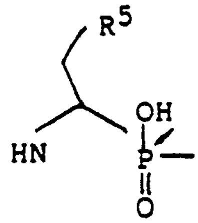 or 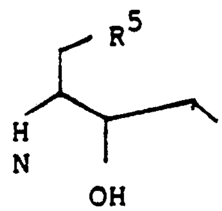 or 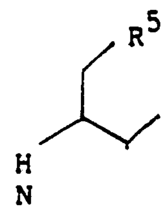

wherein $E^2$ is

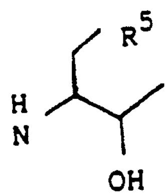 or 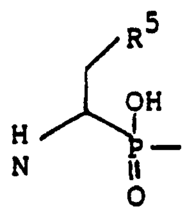 or 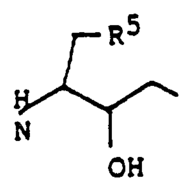 or 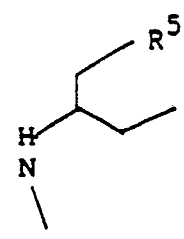

, with the proviso if $E^2$ is

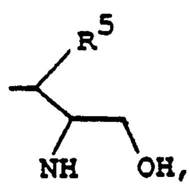

$E^4$ is not
$R^{2}-O-\overset{O}{\overset{\|}{C}}$ ,
wherein the ring is 4 to 7 membered,
wherein $R^5$ is an unsubstituted or substituted aromatic group with 6 to 18 carbon atoms, or an unsubstituted or substituted aliphatic group with 1 to 18 carbon atoms,
wherein X is hydrogen or $R^1$ or a group having the formula

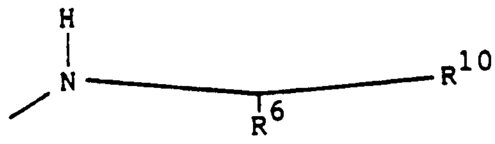

wherein $R^6$ is a substituted or unsubstituted aliphatic group with one to ten carbon atoms or a substituted or unsubstituted aromatic group with 6 to 12 carbon atoms,
wherein the substituent is

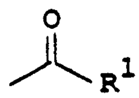

or a $C_1$-$C_{12}$ aliphatic group or an aromatic group with 6 to 12 carbon atoms, wherein $R^1$ is defined as above,
wherein $R^{10}$ is hydrogen or

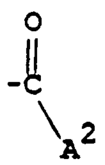 or 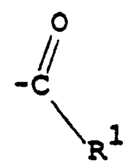 ,

wherein $A^2$ is a group having the formula

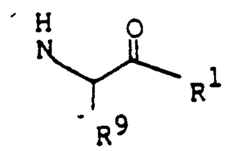

wherein $R^1$ and $R^9$ are defined as above and said therapeutic active compound further existing in different sterochemical forms and mixtures of said forms, and pharmaceutically acceptable salts or esters of said compound.

2. A therapeutic active compound according to claim 1, wherein Z is Ser or Thr.

3. A therapeutic active compound according to any of claims 1 and 2, wherein $E^1$ and $E^2$ are disposed adjacent to each other.

4. A compound according to any of claims 1 to 3, wherein $R^2$, $R^3$ and $R^4$, independently of each other are unsubstituted or substituted $C_1$-$C_{10}$-alkyl, unsubstituted or substituted $C_2$-$C_{20}$ alkenyl, unsubstituted or substituted $C_3$-$C_{10}$-alkynyl or unsubstituted or substituted $C_3$-$C_{10}$ aromatic, wherein the substituents for said alkyl, alkenyl and alkynyl are selected from the group consisting of $C_1$-$C_5$-alkyl, $C_6$-$C_{12}$ aromatics, halogen; hydroxy; and an amino of the formula $-NR^8R^7$, wherein $R^8$ and $R^7$ independently of each other are halogen, $C_1$-$C_{20}$ alkyl or oxygen; and the substituents for said aromatic are selected from the group consisting of $C_1$-$C_6$-alkyl, $C_6$-$C_{12}$ aromatics, halogen; hydroxy; and amino of the formula $-NR^8R^7$, wherein $R^8$ and $R^7$ independently of each other are halogen, $C_1$-$C_{10}$-alkyl, oxygen, $C_2$-$C_{20}$-alkenyl or $C_3$-$C_{20}$-alkynyl.

5. A therapeutic active compound according to claim 4, wherein the halogen is selected from the group consisting of chlorine, fluorine, bromine and iodine.

6. A therapeutic active compound according to any of claims 4 and 5, wherein the aromatic has 6 to 10 carbon atoms.

7. A therapeutic compound according to any of claims 1 to 6, wherein $A''$ is selected from the group consisting of

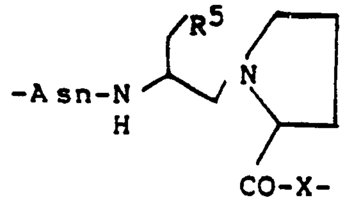

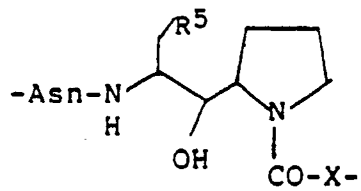

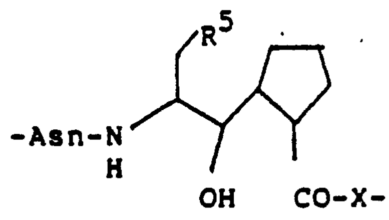

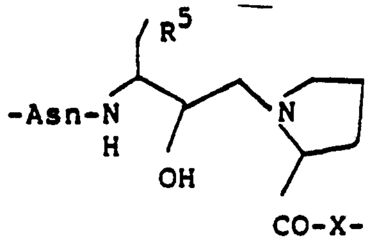

and

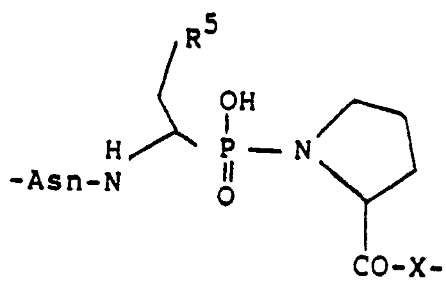

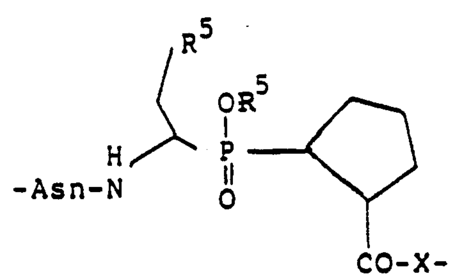

, wherein $R^2$ is as defined in claim 1,
wherein $R^5$ is phenyl, p-hydroxphenyl or cyclohexyl,
wherein X is hydrogen or $R^1$ or a group having the formula

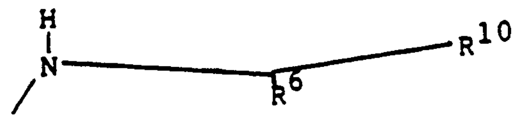

wherein $R^6$ is selected from the group consisting of methyl, ethyl, isopropyl, butyl, isobutyl, sec.-butyl, cyclohexyl, phenyl, pyridyl, all of which being unsubstituted or substituted by phenyl

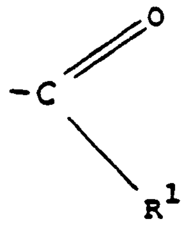

or
pyridyl, naphthal, methyl, ethyl, propyl, isopropyl, butyl, sec.-butyl or isobutyl,
wherein $R^1$ is defined as above,
wherein $R^{10}$ is hydrogen or

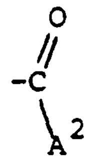

or

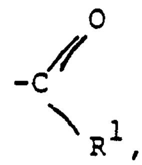

and
wherein $A^2$ and $R^1$ are defined as above.

8. A medicament for treating HIV comprising a pharmaceutically effective amount of an active compound according to any of claims 1 to 7 in a mixture with a pharmaceutically acceptable carrier.

9. A compound comprising formula III

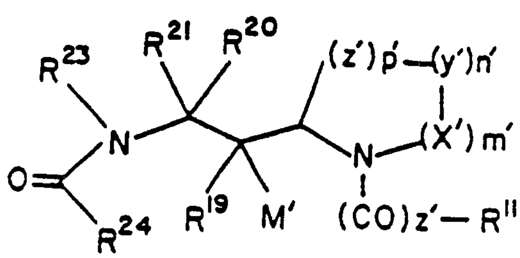

and salts thereof
wherein $R^{11}$ stands for hydrogen or $OR^{12}$ or $NR^{12}R^{13}$ or $CR^{12}R^{13}R^{14}$ or $SiR^{12}R^{13}R^{14}$ and $z'$ is zero (0) or one (1); if $z'$ is zero, $R^{11}$ is hydrogen, and R1' and $R^{17}$ do not represent a single bond; if $z'$ is one, $R^{11}$ is not hydroxyl
wherein $R^{12}$,$R^{13}$,$R^{14}$ independently of each other stand for hydroxyl
or unsubstituted or substituted alkyl
or substituted or unsubstituted cycloalkyl
or unsubstituted or substituted aryl
or unsubstituted or substituted heteroaryl
or unsubstituted or substituted aralkyl
wherein the substituents of said alkyl, cycloalkyl, aralkyl,aryl or heteroaryl are chosen from the group consisting of halogen,alkyl,cycloalkyl,aryl, heteroaryl,$NR^{15}R^{16}$,hydroxy, alkoxy, aryloxy, aralkoxy
wherein $R^{15}$,$R^{16}$ independently of each other stand for hydrogen or alkyl or cycloalkyl or aryl or aralkyl or

heteroaryl
or $R^{15}$ and $R^{16}$ together stand for an alkyl chain with two to ten carbon atoms which can be substituted by alkyl groups
or $R^{11}$ and $R^{17}$ together represent a single bond
wherein M′ stands for $OR^{17}$
wherein $R^{17}$ stands for hydrogen or $CR^{12}R^{13}R^{14}$ or $SiR^{12}R^{13}R^{14}$ or $CO-R^{18}$
wherein $R^{12}$,$R^{13}$,$R^{14}$ are defined above but can be independently chosen
wherein $R^{18}$ stands for hydrogen or $OR^{12}$ or $NR^{12}R^{13}$ or $CR^{12}R^{13}R^{14}$ wherein $R^{12}$,$R^{13}$,$R^{14}$ are defined above
or $R^{17}$ and $R^{11}$ together represent a single bond
or $R^{17}$ and $R^{23}$ together represent a group $CR^{12}R^{13}$
wherein $R^{12}$,$R^{13}$ are defined above or M′ and $R^{25}$ together represent a single bond
wherein $R^{19}$,$R^{20}$,$R^{21}$ stand for hydrogen or unsubstituted or substituted alkyl or unsubstituted or substituted cycloalkyl or unsubstituted or substituted aryl or unsubstituted or substituted heteroaryl or unsubstituted or substituted aralkyl
wherein the substituents are chosen from the group consisting of $COR^{22}$,$NR^{15}R^{16}$,$SR^{15}$,aryl, heteroaryl,alkyl,cycloalkyl, halogen, hydroxy,alkoxy,aryloxy,or a group having the formula
$R^{15}R^{16}NC(=NH)NH-$
wherein $R^{22}$ stands for hydrogen or $OR^{12}$ or $NR^{12}R^{13}$ or $CR^{12}$,$R^{13}$,$R^{14}$
wherein $R^{12}$,$R^{13}$,$R^{14}$ are defined above
wherein $R^{15}$ and $R^{16}$ are defined above
wherein $R^{23}$stands for hydrogen or $SiR^{12}R^{13}R^{14}$ or $CR^{12}R^{13}R^{14}$ or $COR^{22}$ or aryl or heteroaryl
wherein $R^{12}$,$R^{13}$,$R^{14}$,$R^{22}$ are defined above or $R^{23}$ and $R^{17}$ together represent a group $CR^{12}R^{13}$
wherein $R^{12}$ and $R^{13}$ are defined above
wherein $R^{24}$ stands for $OR^{25}$
wherein $R^{25}$ stands for unsubstituted or
substituted alkyl or cycloalkyl
or for unsubstituted or substituted aryl
or unsubstituted or substituted heteroaryl
or unsubstituted or substituted aralkyl
wherein the substituents of said groups are chosen from the group consisting of halogen,alkyl, cycloalkyl,aryl,heteroaryl, $NR^{15}R^{16}$,hydroxy,alkoxy,aryloxy, heteroaryloxy
wherein $R^{15}$,$R^{16}$ independently of each other stand for hydrogen,alkyl,cycloalkyl, aryl,aralkyl,heteroaryl
or $R^{24}$ and $R^{17}$ together stand for a single bond
wherein X′ stands for $CR^{12}R^{13}$ or $R^{12}C=CR^{13}$ or C C
wherein $R^{12}$ and $R^{13}$ are defined above
wherein m′ stands for a number between zero (0) and ten (10), and $2 \leq m' + n' + p' \leq 8$
wherein Y′ stands for O or S or $NR^{12}$ or $R^{12}R^{13}N^{+}$ or $SiR^{12}R^{13}$ or $CR^{12}R^{13}$ or $R^{12}C=CR^{13}$ or C C
wherein $R^{12}$,$R^{13}$ are defined above
wherein n′ stands for a number between zero (0) and ten (10), and $2 \leq m' + n' + p'' \leq 8$
wherein Z′ stands for $CR^{12}R^{13}$ or $R^{12}C=CR^{13}$ or C C
wherein $R^{12}$,$R^{13}$ are defined above
wherein p′ stands for a number between zero (0) and ten (10), and $2 \leq ' + n' + p' \leq 8$.

10. A compound according to claim 9,
wherein $R^{11}$ stands for hydrogen or $OR^{12}$ or $NR^{12}R^{13}$ or $CR^{12}R^{13}R^{14}$ or $SiR^{12}R^{13}R^{14}$,
z′ is zero (0) or one (1); if z′ is zero, $R^{11}$ is hydrogen and $R^{11}$ and $R^{17}$ do not represent a single bond; if z′ is one, $R^{11}$ is not hydrogen
wherein $R^{12}$,$R^{13}$;$R^{14}$ independently of each other stand for hydroxyl
or unsubstituted or substituted lower alkyl
or substituted or unsubstituted lower cycloalkyl
or unsubstituted or substituted lower aryl
or unsubstituted or substituted lower heteroaryl
or unsubstituted or substituted lower aralkyl
wherein the substituents of said alkyl,cycloalkyl, arylkyl,aryl or heteroaryl group are chosen from the group consisting of halogen, lower alkyl,lower cycloalkyl,lower aryl,lower heteroaryl,$NR^{15}R^{16}$, hydroxy,lower alkoxy,lower aryloxy,lower aralkyloxy,
wherein $R^{15}$,$R^{16}$ independently of each other stand for hydrogen or lower alkyl or lower cycloalkyl or lower aryl or lower aralkyl or lower heteroaryl
or R15 and R16 together stand for an alkyl chain with two to six carbon atoms which can be substituted by

lower alkyl
or $R^{11}$ and $R^{17}$ together represent a single bond
wherein $M'$ stands for $OR^{17}$
wherein $R^{17}$ stands for hydrogen $CR^{12}R^{13}R^{14}$ or $SiR^{12}R^{13}R^{14}$ or $CO-R^{18}$
wherein $R^{'2}$,$R^{13}$,$R^{14}$ are defined above but can be independently chosen wherein $R^{18}$ stands for hydrogen or $OR^{'2}$ or $NR^{12}R^{'}3$ or $CR^{12}R^{13}R^{14}$ wherein $R^{'2}$,$R^{13}$,$R^{14}$ are defined above
or $R^{'7}$ and $R^{11}$ together represent a single bond
or $R^{'7}$ and $R^{23}$ together represent a group $CR^{'2}R^{13}$
wherein $R^{'2}$,$R^{13}$ are defined above
or $M'$ and $R^{25}$ together represent a single bond
wherein $R^{'9}$ and $R^{20}$ stand for hydrogen or lower alkyl or lower aryl or lower heteroaryl
wherein $R^{21}$ stands for hydrogen or unsubstituted or substituted lower alkyl
or unsubstituted or substituted lower cycloalkyl
or unsubstituted or substituted lower aryl
or unsubstituted or substituted lower heteroaryl
or unsubstituted or substituted lower aralkyl
wherein the substituents of said groups are chosen from the group consisting of $COR^{22}$,$NR^{'5}R16$, $SR^{'5}$.lower aryl, lower heteroaryl.lower alkyl.lower cycloalkyl,halogen,hydroxy, lower alkoxy.lower aryloxy.lower aralkyloxy, or a group having the formula
$R^{'5}R^{'6}NC(=NH)NH$-
wherein $R^{22}$ stands for hydrogen or $OR^{'2}$ or $NR^{12}R^{13}$ or $CR^{12}R^{13}R^{14}$
wherein $R^{12}$.$R^{13}$.$R^{14}$ are defined above
wherein $R^{15}$ and $R^{'6}$ are defined above
wherein $R^{23}$ stands for hydrogen or $SiR^{12}R^{'3}R^{14}$ or $CR^{'2}R^{'3}R^{14}$ or $COR^{22}$ or lower aryl or lower heteroaryl
wherein $R^{'2}$.$R^{13}$.$R^{14}$,$R^{22}$ are defined above
or $R^{23}$ and $R^{'5}$ together represent a group $CR^{'2}R^{'3}$
wherein $R^{'2}$ and $R^{'3}$ are defined above
wherein $R^{24}$ stands for $OR^{25}$
wherein $R^{25}$ stands for unsubstituted or substituted lower alkyl or lower cycloalkyl
or unsubstituted or substituted lower aryl
or unsubstituted or substituted lower heteroaryl
or unsubstituted or substituted lower aralkyl
wherein the substituents of said groups are chosen from the group consisting of lower alkyl,lower cycloalkyl,lower aryl,lower aralkyl,or lower heteroaryl
or $R^{24}$ and $R^{'7}$ together stand for a single bond
wherein $X'$ stands for $CR^{'2}R^{'3}$ or $R^{12}C=CR^{13}$ or C C
wherein $R^{'2}$ and $R^{'3}$ are defined above
wherein $m'$ stands for a number between zero (0) and five (5), and $2 \leq m' + n' + p' \leq 8$
wherein $Y'$ stands for O or S or $NR^{12}$ or $R^{12}R^{'3}N^{+}$ or $SiR^{12}R^{13}$ or $CR^{12}R^{13}$ or $R^{12}C=CR^{13}$ or C C
wherein $R^{'2}$.$R^{13}$ are defined above
wherein $n'$ stands for a number between zero (0) and five (5), and $2 \leq m' + n' + p'' \leq 8$
wherein $Z'$ stands for $CR^{'2}R^{'3}$ or $R^{'2}C=CR^{'3}$ or C C
wherein $R^{'2}$.$R^{13}$ are defined above
wherein $p'$ stands for a number between zero (0) and five (5), and $2 \leq ' + n' + p' \leq 8$.

11. A compound according to claim 9, wherein $R^{11}$ stands for hydrogen or $OR^{'2}$ or $NR^{'2}R^{'3}$ or $CR^{'2}R^{'3}R^{'4}$ or $SiR^{'2}R^{'3}R^{'4}$
wherein $z'$ is zero (0) or one (1); if $z'$ is zero, $R^{11}$ is hydrogen and $R^{11}$ and $R^{17}$ do not represent a single bond; if $z'$ is one, $R^{11}$ is not hydroxyl wherein $R^{12}$,$R^{13}$,$R^{14}$ independently of each other stand for hydrogen or methyl.ethyl,propyl, isopropyl,butyl,isobutyl,sec-butyl,pentyl
or cyclopropyl,cyclobutyl,cyclopentyl
or phenyl.tolyl, naphtyl
or pyridyl.furyl,thienyl
or benzyl.naphtylmethyl,phenetyl,phenyl propyl
or $R^{11}$ and $R^{17}$ together represent a single bond
wherein $M'$ stands for $OR^{17}$
wherein $R^{17}$ stands for hydrogen $CR^{'2}R^{13}R^{14}$ or $SiR^{'2}R^{'3}R^{14}$ or $CO-R^{18}$
wherein $R^{12}$,$R^{13}$.$R^{14}$ are defined above but can be independently chosen

wherein $R^{18}$ stands for hydrogen or $OR^{12}$ or $NR^{12}R^{13}$ or $CR^{13}R^{13}R^{14}$
wherein $R^{12}$,$R^{13}$,$R^{14}$ are defined above
or $R^{17}$ and $R^{11}$ together represent a single bond
or $R^{17}$ and $R^{23}$ together represent a group $CR^{12}R^{13}$
wherein $R^{12}$,$R^{13}$ are defined above
or M′ and $R^{25}$ together represent a single bond
wherein $R^{19}$ stands for hydrogen or methyl, ethyl, propyl, isopropyl, butyl, isobutyl, sec-butyl, pentyl, hexyl, phenyl, naphtyl
wherein $R^{20}$ stands for hydrogen
wherein $R^{21}$ stands for hydrogen or unsubstituted or substituted methyl,ethyl,propyl,isopropyl,butyl,isobutyl, sec-butyl,pentyl,hexyl,
or unsubstituted or substituted cyclopropyl,cyclobutyl,cyclopentyl, cyclohexyl,or unsubstituted or substituted phenyl, tolyl,naphtyl or unsubstituted or substituted pyridyl,thienyl, quinolyl,furyl,thiazolyl
or unsubstituted or substituted benzyl, naphtylmethyl, phenetyl,phenylpropyl
wherein the substituents of said groups are chosen from the group consisting of $COR^{22}$,$NR^{15}R^{16}$,$SR^{15}$,phenyl,naphtyl, pyridyl,thienyl, quinolyl,furyl, thiazolyl,imidazoyl,benzimidazoyl,methyl,ethyl,propyl, isopropyl, butyl,isobutyl, sec-butyl,pentyl,cyclopropyl, cyclobutyl, cyclopentyl,cyclohexyl, halogen,hydroxy, methoxy,ethoxy,propoxy,butoxy,phenoxy, naphtoxy, benzyloxy, naphtylmethoxy
or a group having the formula
$R^{15}R^{16}NC(=NH)NH$-
wherein $R^{22}$ stands for hydrogen or $OR^{12}$ or $NR^{12}R^{13}$ or $CR^{12}R^{13}R^{14}$
wherein $R^{12}$,$R^{13}$,$R^{14}$ are defined above
wherein $R^{15}$ and $R^{16}$ are independently of each other,hydrogen,methyl,ethyl, propyl, isopropyl,butyl,isobutyl, sec-butyl,pentyl,cyclopropyl, cyclobutyl,cyclopentyl,phenyl, naphtyl,benzyl,naphtylmethyl, phenetyl,phenylpropyl, pyridyl,thienyl,quinolyl,furyl, thiazolyl
or $R^{15}$ and $R^{16}$ together stand for an alhyl chain with two to five carbon atoms
wherein $R^{23}$ stands for hydrogen or $SiR^{12}R^{13}R^{14}$ or $CR^{12}R^{13}R^{14}$ or $COR^{22}$ or phenyl or naphyl
wherein $R^{12}$,$R^{13}$,$R^{14}$,$R^{22}$ are defined above or $R^{23}$ and $R^{17}$ together represent a group $CR^{12}R^{13}$
wherein $R^{12}$ and $R^{13}$ are defined above
wherein $R^{24}$ stands for $OR^{25}$
wherein $R^{25}$ stands for methyl,ethyl,propyl, isopropyl,butyl,isobutyl,sec-butyl,cyclopropyl, cyclobutyl,cyclopentyl,cyclohexyl,phenyl, naphtyl,thienyl,furyl,pyridyl,fluorenylmethyl, benzyl,naphtylmethyl,phenetyl,phenylpropyl,
or $R^{24}$ and $R^{17}$ together stand for a single bond wherein X′ stands for $CR^{12}R^{13}$ or $R^{12}C=CR^{13}$ or C=C
wherein $R^{12}$ and $R^{13}$ are defined above
wherein m′ stands for a number between zero (0) and five (2), and $2 \leq m' + n' + p' \leq 6$
wherein Y′ stands for O or S or $NR^{12}$ or $R^{12}R^{13}N^{+}$ or $SiR^{12}R^{13}$ or $CR^{12}R^{13}$ or $R^{12}C=CR^{13}$ or C C
wherein $R^{12}$,$R^{13}$ are defined above
wherein n′ stands for a number between zero (0) and five (2),and $2 \leq m' + n' + p'' \leq 6$
wherein Z′ stands for $CR^{12}R^{13}$ or $R^{12}C=CR^{13}$ or C C
wherein$R^{12}$,$R^{13}$ are defined above
wherein p′ stands for a number between zero (0) and five (5), and $2 \leq ' + n' + p' \leq 8$.

12. A compound comprising formula IV

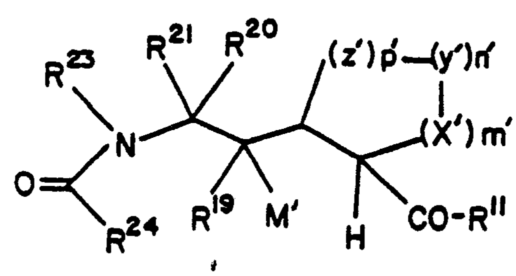

and salts thereof
wherein $R^{11}$ stands for hydrogen or $OR^{12}$ or $NR^{12}R^{13}$ or $CR^{12}R^{13}R^{14}$ or $SiR^{12}R^{13}R^{14}$
wherein $R^{12}$,$R^{13}$,$R^{14}$ independently of each other stand for hydrogen

or unsubstituted or substituted alkyl
or substituted or unsubstituted cycloalkyl
or unsubstituted or substituted aryl
or substituted or unsubstituted heteroaryl
or unsubstituted or substituted aralkyl
wherein the substituents of said alkyl, cycloalkyl, aralkyl, aryl or heteroaryl are chosen from the group consisting of halogen, alkyl, cycloalkyl, aryl, heteroaryl,$NR^{15}R^{16}$, hydroxy, alkoxy, aryloxy, alalkoxy
wherein $R^{'5}$,$R^{16}$ independently of each other stand for
hydrogen or alkyl or
cycloalkyl or aryl or aralkyl or heteroaryl
or $R^{'5}$ and $R^{16}$ together stand for an alkyl chain with two to ten carbon atoms which can be substituted by alkyl groups or $R^{11}$ and $R^{17}$ together represent a single bond
wherein M$'$ stands for $OR^{17}$
wherein $R^{'7}$ stands for hydrogen or $CR^{12}R^{13}R^{14}$ or $SiR^{12}R^{13}R^{14}$ or CO-$R^{18}$
wherein $R^{12}$,$R^{13}$,$R^{14}$ are defined above but can be independently chosen
wherein $R^{'8}$ stands for hydrogen or $OR^{12}$ or $NR^{12}R'3$ or $CR^{12}R^{13}R^{14}$
wherein $R^{'2}$,$R^{13}$,$R^{14}$ are defined above
or $R^{'7}$ and $R^{''}$ together represent a single bond
or $R^{'7}$ and $R^{23}$ together represent a group $CR^{12}R^{13}$
wherein $R^{'2}$,$R^{'3}$ are defined above or M$'$ and $R^{25}$ together represent a single bond wherein $R^{'9}$,$R^{20}$,$R^{2'}$ stand for hydrogen or unsubstituted or alkyl or unsubstituted or substituted cycloalkyl unsubstituted or substituted aryl or unsubstituted or substituted heteroaryl or unsubstituted or substituted aralkyl wherein the substituents are chosen from the group
consisting of $COR^{22}$,$NR^{15}R16$,$SR^{15}$,aryl heteroaryl,alkyl, cycloalkyl, halogen, hydroxy,alkoxy,aryloxy,or a group having the formula
$R^{'5}R^{'6}NC(=NH)NH$-
wherein $R^{22}$ stands for hydrogen or $OR^{'2}$ or $NR^{12}R^{13}$ or $CR^{12}R^{13}R^{14}$
wherein $R^{'2}$,$R^{'3}$,$R^{14}$ are defined above
wherein $R^{'5}$ and $R^{16}$ are defined above
wherein $R^{23}$ stands for hydrogen or $SiR^{12}R^{13}R^{14}$ or $CR^{12}R^{13}R^{14}$ or $COR^{22}$
wherein $R^{'2}R^{'3}$,$R^{'4}R2^{2}$ are defined above or $R^{23}$ and $R^{17}$ together represent a group $CR^{'2}R^{13}$
wherein $R^{'2}$ and $R^{13}$ are defined above
wherein $R^{24}$ stands for $OR^{25}$
wherein $R^{25}$ stands for unsubstituted or substituted alkyl or cycloalkyl
or for unsubstituted or substituted aryl or unsubstituted or substituted heteroaryl or unsubstituted or substituted aralkyl
wherein the substituents of said groups are chosen from the group consisting of halogen,alkyl, cycloalkyl,aryl,heteroaryl, $NR^{15}R^{16}$,hydroxy,alkoxy,aryloxy, heteroaryloxy
wherein $R^{'5}$,$R^{'6}$ independently of each other stand for hydrogen,alkyl,cycloalkyl, aryl,aralkyl,heteroaryl
or $R^{24}$ and $R^{17}$ together stand for a single bond
wherein X$'$ stands for $CR^{12}R^{13}$ or $R^{12}C=CR^{'3}$ or C=C
wherein $R^{'2}$ and $R^{13}$ are defined above
wherein m$'$ stands for a number between zero (0) and ten (10), and $2 \leq m' + n' + p' \leq 8$
wherein Y$'$ stands for O or S or $NR^{12}$ or $R^{12}R^{13}N+$ or $SiR^{12}R^{'3}$ or $CR^{12}R^{'3}$ or $R^{'2}C=CR^{'3}$ or C=C
wherein $R^{'2}$,$R^{'3}$ are defined above
wherein n$'$ stands for a number between zero (0) and ten (10), and $2 \leq m' + n' + p'' \leq 8$
wherein Z$'$ stands for $CR^{12}R^{13}$ or $R^{'2}C=CR^{'3}$ or C=C
wherein R12,$R^{'3}$ are defined above
wherein p$'$ stands for a number between zero (0) and ten (10), and $2 \leq m' + n' + p' \leq 8$.

13. A compound according to claim 12,
wherein $R^{''}$ stands for hydrogen or $OR^{12}$ or $NR^{12}R^{13}$ or $CR^{12}R^{13}R^{14}$ or $SiR^{12}R^{13}R^{'4}$
wherein $R^{'2}$,$R^{'3}$,$R^{'4}$ independently of each of stand for hydrogen
or unsubstituted or substituted lower alkyl
or substituted or unsubstituted lower cycloalkyl
or unsubstituted or substituted lower aryl
or unsubstituted or substituted lower heteroaryl
or unsubstituted or substituted aralkyl
wherein the substituents of said alkyl, cycloalkyl, aralkyl,aryl or heteroaryl group are chosen from the group

consisting of halogen, lower alkyl,lower cycloalkyl,lower aryl,lower heteroaryl,$NR^{15}R^{16}$, hydroxy,lower alkoxy,lower aryloxy, lower aralkyloxy,
wherein $R^{15}$,$R^{16}$ independently or each other stand for hydrogen or lower alkyl or lower cycloalkyl or lower aryl or lower aralkyl or lower heteroaryl
or $R^{15}$ and $R^{16}$ together stand for an alkyl chain with two to six carbon atoms which can be substituted by lower alkyl
or $R^{11}$ and $R^{17}$ together represent a single bond
wherein $M'$ stands for $OR^{17}$
wherein $R^{17}$ stands for hydrogen or $CR^{12}R^{13}R^{14}$ or $SiR^{12}R^{13}R^{14}$ or $CO-R^{18}$
wherein $R^{12}$,$R^{13}$,$R^{14}$ are defined above but can be independently chosen
wherein $R^{18}$ stands for hydrogen or $OR^{12}$ or $NR^{12}R^{1}3$ or $CR^{12}R^{13}R^{14}$
wherein $R^{12}$,$R^{13}R^{14}$ are defined above
or $R^{17}$ and $R^{11}$ together represent a single bond
or $R^{17}$ and $R^{23}$ together represent a group $CR^{12}R^{13}$
wherein $R^{12}$,$R^{13}$ are defined above
or $M'$ and $R^{25}$ together represent a single bond
wherein $R^{19}$ and $R^{20}$ stand for hydrogen or lower alkyl
wherein $R^{21}$ stands for hydrogen or unsubstituted or lower alkyl
or unsubstituted or substituted lower cycloalkyl
or unsubstituted or substituted lower aryl
or unsubstituted or substituted lower heteroaryl
or unsubstituted or substituted lower aralkyl
wherein the substituents of said groups are chosen from the group consisting of $COR^{22}$, $NR^{15}$,$R^{16}$,$SR^{15}$,lower aryl, lower heteroaryl, lower alkyl,lower cycloalkyl,halogen,hydroxy,lower alkoxy, lower aryloxy,lower aralkyloxy, or a group having the formula
$R^{15}R^{16}NC(=NH)NH-$
wherein $R^{22}$ stands for hydrogen or $OR^{12}$ or $NR^{12}R^{13}$ or $CR^{12}R^{13}R^{14}$
wherein $R^{12}$,$R^{13}$,$R^{14}$ are defined above
wherein $R^{15}$ and $R^{16}$ are defined above
wherein $R^{23}$ stands for hydrogen or $SiR^{12}R^{13}R^{14}$ or $CR^{12}R^{13}R^{14}$ or $COR^{22}$ or lower aryl or lower heteroxyl
wherein $R^{12}$,$R^{13}$,$R^{14}$,$R2^{2}$ are defined above
or $R^{23}$ and $R^{15}$ together represent a group $CR^{12}R^{13}$
wherein $R^{12}$ and $R^{13}$ are defined above
wherein $R^{24}$ stands for $OR^{25}$
wherein $R^{25}$ stands for unsubstituted or substituted lower alkyl or lower cycloalkyl or for unsubstituted for substituted lower aryl or unsubstituted or substituted lower heteroaryl or unsubstituted or substituted lower aralkyl
wherein the substituents of said groups are chosen from the group consisting of lower alkyl,lower cycloalkyl,lower aryl,lower aralkyl,or lower heteroaryl
or $R^{24}$ and $R^{17}$ together stand for a single bond
wherein $X'$ stands for $CR^{12}R^{13}$ or $R^{12}C=CR^{13}$ or $C=C$
wherein $R^{12}$ and $R^{13}$ are defined above
wherein $m'$ stands for a number between zero (0) and five (5), and $2 \leq m' + n' + p' \leq 8$
wherein $Y'$ stands for O or S or $NR^{12}$ or $R^{12}R^{13}N^{+}$ or $SiR^{12}R^{13}$ or $CR^{12}R^{13}$ or $R^{12}C=CR^{13}$ or C C
wherein $R^{12}$,$R^{13}$ are defined above
wherein $n'$ stands for a number between zero (0) and five (5), and $2 \leq m' + n' + p'' \leq 8$
wherein $Z'$ stands for $CR^{12}R^{13}$ or $R^{12}C=CR^{13}$ or C C
wherein R12,$R^{13}$ are defined above
wherein $p'$ stands for a number between zero (0) and five (5), and $2 \leq m' + n' + p' \leq 8$.

14. A compound according to claim 12,
wherein $R^{11}$ stands for hydrogen or $OR^{12}$ or $NR^{12}R^{13}$ or $CR^{12}R^{13}R^{14}$ or $SiR^{12}R^{13}R^{14}$
wherein $R^{12}$,$R^{13}$,$R^{14}$ independently of each other stand for hydrogen
or methyl,ethyl,propyl,isopropyl,butyl, isobutyl,sec-butyl,pentyl
or cyclopropyl,cyclobutyl,cyclopentyl
or phenyl,tolyl,napthyl
or pyridyl,furyl,thienyl
or benzyl,naphtylmethyl,phenetyl,phenyl propyl
or $R^{11}$ and $R^{17}$ together represent a single bond

wherein M′ stands for $OR^{17}$
wherein $R^{17}$ stands for hydrogen or $CR^{12}R^{13}R^{14}$ or $SiR^{12}R^{13}R^{14}$ or $CO-R^{18}$
wherein $R^{12}$,$R^{13}$,$R^{14}$ are defined above but can be independently chosen wherein $R^{18}$ stands for hydrogen or $OR^{12}$ or $NR^{12}R^{13}$ or $CR^{12}R^{13}R^{14}$ wherein $R^{12}$,$R^{13}$,$R^{14}$ are defined above
or $R^{17}$ and $R^{11}$ together represent a single bond
or $R^{17}$ and $R^{23}$ together represent a group $CR^{12}R^{13}$
wherein $R^{12}$,$R^{13}$ are defined above
or M′ and $R^{25}$ together represent a single bond wherein $R^{19}$ stands for hydrogen or methyl, ethyl, propyl, isopropyl, butyl, sec-butyl, isobutyl, pentyl, hexyl, phenyl, naphtyl,
wherein $R^{20}$ stands for hydrogen
wherein $R^{21}$ stands for hydrogen or unsubstituted or substituted methyl,ethyl,propyl,isopropyl,butyl,isobutyl sec-butyl,phenyl,hexyl,
or unsubstituted or substituted cyclopropyl,cyclobutyl, cyclopentyl,cyclohexyl,
or unsubstituted or substituted phenyl,tolyl,naphtyl
or unsubstituted or substituted pyridyl,thienyl, quinolyl,furyl,thiazolyl
or unsubstituted or substituted benzyl, naphthylmethyl, phenetyl,phenylpropyl
wherein the substituents of said groups are chosen from the group
consisting of $COR^{22}$,$NR^{15}R^{16}$,$SR^{15}$,phenyl, napthyl,pyridyl,thienyl,quinolyl,furyl, thiazolyl,imidazoyl methyl,ethyl,propyl,isopropyl,butyl,isobutyl, sec-butyl,pentyl,cyclopropyl,cyclobutyl, cyclopentyl,cyclohexyl, halogen,hydroxy,methoxy,ethoxy, propoxy,butoxy,phenoxy,naphtoxy,benzyloxy, naphtylmethoxy
or a group having the formula
$R^{15}R^{16}NC(=NH)NH-$
wherein $R^{22}$ stands for hydrogen or $OR^{12}$ or $NR^{12}R^{13}$ or $CR^{12}R^{13}R^{14}$
wherein $R^{12}$,$R^{13}$,$R^{14}$ are defined as above
wherein $R^{15}$ and $R^{16}$ are independently of each other hydrogen,methyl,ehtyl,propyl, isopropyl,butyl,isobutyl,sec-butyl, pentyl,cyclopropyl,cyclobutyl, cyclopentyl,phenyl,naphtyl,benzyl, naphtylmethyl,phenetyl,phenylpropyl, pyridyl,thienyl,quinolyl,furyl, thiazolyl
or $R^{15}$ and $R^{16}$ together stand for an alkyl chain with two to five carbon atoms
wherein $R^{23}$ stands for hydrogen or $SiR^{12}R^{13}R^{14}$ or $CR^{12}R^{13}R^{14}$ or $COR^{22}$ or phenyl or naphtyl
wherein $R^{12}$,$R^{13}$,$R^{14}$,$R^{22}$ are defined above
or $R^{23}$ and $R^{17}$ together represent a group $CR^{12}R^{13}$ wherein $R^{12}$ and $R^{13}$ are defined above
wherein $R^{24}$ stands for $OR^{25}$
wherein $R^{25}$ stands for
methyl,ethyl,propyl,isopropyl,
butyl,isobutyl,sec-butyl,cyclopropyl,cyclobutyl, cyclopentyl,cyclohexyl,phenyl,naphtyl,thienyl, furyl,pyridyl,fluorenylmethyl,benzyl, naphtylmethyl,phenetyl,phenylpropyl,
or $R^{24}$ and $R^{17}$ together stand for a single bond
wherein X′ stands for $CR^{12}R^{13}$ or $R^{12}C=CR^{13}$ or C=C
wherein $R^{12}$ and $R^{13}$ are defined above
wherein m′ stands for a number between zero (0) and five (2), and $2 \leq m' + n' + p' \leq 6$
wherein Y′ stands for O or S or $NR^{12}$ or $R^{12}R^{13}N^{+}$ or $SiR^{12}R^{13}$ or $CR^{12}R^{13}$ or $R^{12}C=CR^{13}$ or C=C
wherein $R^{12}$,$R^{13}$ are defined above
wherein n′ stands for a number between zero (0) and five (2), and $2 \leq m' + n' + p'' \leq 6$
wherein Z′ stands for $CR^{12}R^{13}$ or $R^{12}C=CR^{13}$ or C C
wherein $R^{12}$,$R^{13}$ are defined above
herein p stands for a number between zero (0) and

15. A process forfor the preparation of compounds according to claims 12, 13 and 14, characterized in that compounds of the general formula V

$[(L^{01})_a(L^{02})_b(L^{03})_c(L^{04})_d(L^{05})_e(L^{06})_f M_g]$ 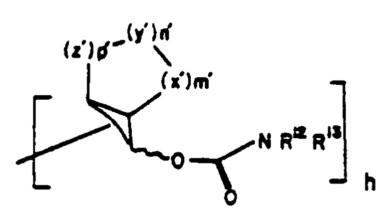

wherein X′,Y′,Z′ are defined above

wherein m′,m′,p′ are defined above
wherein $R^{12}$,$R^{13}$ are defined above
wherein $L^{01}$, $L^{02}$, $L^{03}$, $L^{04}$, $L^{05}$, $L^{06}$ independently of each other stand for hydrogen or halogen or $OR^{12}$ or $NR^{12}R^{13}$ or $CR^{12}R^{13}R^{14}$ or $PR^{12}R^{13}R^{14}$ or $NR^{12}R^{13}R^{14}$
or substituted or unsubstituted cyclopentadienyl
or unsubstituted or substituted lower aryl
or unsubstituted or substituted lower heteroaryl
wherein $R^{12}$,$R^{13}$,$R^{14}$ are defined above
or two (2) of the mentioned substituents together stand for $L^{08}$ $(L^{09})_jL^{10}$
wherein $L^{08}$,$L^{10}$ independently of each other stand for $OR^{12}$,$NR^{12}R^{13}$,$PR^{12}R^{13}$,$NR^{12}$
wherein $R^{12}$,$R^{13}$ are defined above
wherein L09 stands for $CR^{12}R^{13}$
wherein $R^{12}R^{13}$ are defined above
and if j>1 the substituents of the different carbon atoms can be chosen independently
wherein j stands for a number between one (1) and five (5)
wherein a, b, c, d, e, f, stand independently of each other for a number between zero (0) and six (6), and a+b+c+c+e+f+h = n
wherein n stands for a number between two (2) and ten (10), and n must not exceed the maximal number of ligands for a given metal-nucleus,
wherein M stands for lithium, sodium, potassium, boron, magnesium, aluminum, silicon, titanium, vanadium, chromium, manganese, germanium, zirconium,niobium, molybdenum, tin, antimony, lanthanides, hafnium, tantalum, tungsten, rhenium
wherein g stands for a number between one (1) and five (5),
and if g>1 M can represent more than one metal
where h stands for a number between one (1) and five (5)
are reacted with aldehydes or ketones of formula VI

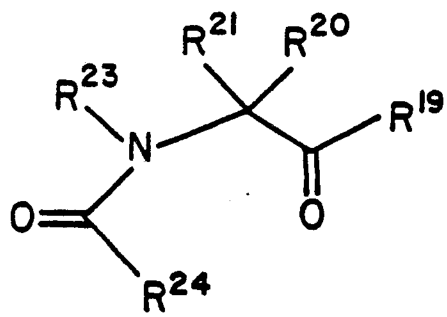

wherein $R^{19}$, $R^{20}$, $R^{21}$, $R^{23}$, $R^{24}$ are defined above
in inert solvents which is followed by oxidation under acidic conditions.

16. N2-[3-[N2-[N-[(1,1-Dimethylethoxy)carbonyl]-L-phenylalanyl]-L-histidyl]amino-2-hydroxy-4-cyclohexyl-butyl]-N-[2-(2-pyridinyl) ethyl]-L-prolineamide having the formula